(19) Europäisches Patentamt
European Patent Office
Office européen des brevets

(11) EP 2 635 304 B1

(12) **EUROPEAN PATENT SPECIFICATION**

(45) Date of publication and mention of the grant of the patent:
**08.03.2017 Bulletin 2017/10**

(21) Application number: **11785862.1**

(22) Date of filing: **04.11.2011**

(51) Int Cl.:
***A61K 39/395*** $^{(2006.01)}$ ***G01N 33/574*** $^{(2006.01)}$
***G01N 33/82*** $^{(2006.01)}$

(86) International application number:
**PCT/US2011/059411**

(87) International publication number:
**WO 2012/061759 (10.05.2012 Gazette 2012/19)**

(54) **FOLATE RECEPTOR ALPHA AS A DIAGNOSTIC AND PROGNOSTIC MARKER FOR FOLATE RECEPTOR ALPHA-EXPRESSING CANCERS**

FOLATREZEPTOR-ALPHA ALS DIAGNOSTISCHER UND PROGNOSTISCHER MARKER FÜR FOLATREZEPTOR- ALPHA-EXPRIMIERENDE KARZINOME

RÉCEPTEUR ALPHA DE FOLATE À TITRE DE MARQUEUR DIAGNOSTIQUE ET PRONOSTIQUE DES CANCERS EXPRIMANT UN RÉCEPTEUR ALPHA DE FOLATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.11.2010 US 410497 P**
**15.07.2011 US 201161508444 P**

(43) Date of publication of application:
**11.09.2013 Bulletin 2013/37**

(60) Divisional application:
**16186634.8 / 3 130 605**

(73) Proprietor: **Morphotek, Inc.**
**Exton, PA 19341 (US)**

(72) Inventors:
- **O'SHANNESSY, Daniel, J.**
  **Schwenksville**
  **PA 19473 (US)**
- **GRASSO, Luigi**
  **Bryn Mawr**
  **PA 19010 (US)**
- **WAN, Shanhong**
  **Paoli**
  **PA 19301 (US)**
- **CHAO, Qimin**
  **Havertown**
  **PA 19083 (US)**
- **SOMERS, Elizabeth, Brooke**
  **Unionville**
  **PA 19375 (US)**

(74) Representative: **Lock, Graham James**
**Fry Heath & Spence LLP**
**The Gables**
**Massetts Road**
**Horley, Surrey RH6 7DQ (GB)**

(56) References cited:
**WO-A2-03/091700**

- **EATI BASAL ET AL: "Functional Folate Receptor Alpha Is Elevated in the Blood of Ovarian Cancer Patients", PLOS ONE, vol. 4, no. 7, 20 July 2009 (2009-07-20), page E6292, XP55022613, DOI: 10.1371/journal.pone.0006292**
- **MANTOVANI L T ET AL: "Folate binding protein distribution in normal tissues and biological fluids from ovarian carcinoma patients as detected by the monoclonal antibodies MOv18 and MOv19", EUROPEAN JOURNAL OF CANCER, PERGAMON PRESS, OXFORD, GB, vol. 30, no. 3, 1 January 1994 (1994-01-01), pages 363-369, XP022643304, ISSN: 0959-8049, DOI: 10.1016/0959-8049(94)90257-7 [retrieved on 1994-01-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DOLO V ET AL: "Matrix-degrading proteinases are shed in membrane vesicles by ovarian cancer cells in vivo and in vitro", CLINICAL AND EXPERIMENTAL METASTASIS, SPRINGER NETHERLANDS, NL, vol. 17, no. 2, 1 March 1999 (1999-03-01), pages 131-140, XP002461206, ISSN: 0262-0898, DOI: 10.1023/A:1006500406240
- LINDA E. KELEMEN: "The role of folate receptor [alpha] in cancer development, progression and treatment: Cause, consequence or innocent bystander?", INTERNATIONAL JOURNAL OF CANCER, vol. 119, no. 2, 1 January 2006 (2006-01-01), pages 243-250, XP55027169, ISSN: 0020-7136, DOI: 10.1002/ijc.21712
- D K Armstrong ET AL: "Exploratory phase II efficacy study of MORAb-003, a monoclonal antibody against folate receptor alpha, in platinum-sensitive ovarian cancer in first relapse.", J Clin Oncol May 20 suppl; abstr 5500, 1 January 2008 (2008-01-01), XP55022660, Retrieved from the Internet: URL:http://www.asco.org/ascov2/Meetings/Abstracts?vmview=abst_detail_view&confID=55&abstractID=32743 [retrieved on 2012-03-22]
- PHILIP S LOW ET AL: "Discovery and Development of Folic-Acid-Based Receptor Targeting for Imaging and Therapy of Cancer and Inflammatory Diseases", ACCOUNTS OF CHEMICAL RESEARCH, ACS, WASHINGTON, DC, US, vol. 41, no. 1, 1 January 2008 (2008-01-01), pages 120-129, XP007915422, ISSN: 0001-4842, DOI: 10.1021/AR7000815 [retrieved on 2007-07-27]
- THUYET TRAN ET AL: "Enhancement of Folate Receptor A Expression in Tumor Cells Through the Glucocorticoid Receptor: A Promising Means to Improved Tumor Detection and Targeting", CANCER RES, vol. 65, no. 10, 15 May 2005 (2005-05-15), pages 4431-4441, XP55027294,
- T. Tran: "Enhancement of Folate Receptor Expression in Tumor Cells Through the Glucocorticoid Receptor: A Promising Means to Improved Tumor Detection and Targeting", Cancer research, vol. 65, no. 10, 15 May 2005 (2005-05-15), pages 4431-4441, XP055027176, ISSN: 0008-5472, DOI: 10.1158/0008-5472.CAN-04-2890

## Description

### RELATED APPLICATIONS

This application claims the benefit of the filing date of U.S. Provisional Application No. 61/410,497, filed on November 5, 2010, and U.S. Provisional Application No. 61/508,444, filed on July 15, 2011.

### BACKGROUND OF THE INVENTION

In humans, the high affinity receptor for folate comes in three isoforms: alpha, beta, and gamma. The alpha and beta forms are typically bound to the membranes of cells by a glycosyl phosphatidylinositol (GPI) anchor. They recycle between extracellular and endocytic compartments and are capable of transporting folate into the cell. Soluble forms of FRα may be derived by the action of proteases or phospholipase on membrane anchored folate receptors.

Folate receptor alpha (also referred to as FRα, FR-alpha, FOLR-1 or FOLR1) is expressed in a variety of epithelial tissues, including those of the choroid plexus, lung, thyroid, kidney, uterus, breast, Fallopian tube, epididymis, and salivary glands. Weitman, SD et al. Cancer Res 52: 3396-3401 (1992); Weitman SD et al, Cancer Res 52: 6708-6711. Overexpression of FRα has been observed in various cancers, including lung cancer (*e.g.*, bronchioalveolar carcinomas, carcinoid tumors, and non-small cell lung cancers, such as adenocarcinomas); mesothelioma; ovarian cancer; renal cancer; brain cancer *(e.g.,* anaplastic ependymoma, cerebellar juvenile pilocytic astrocytoma, and brain metastases); cervical cancer; nasopharyngeal cancer; mesodermally derived tumor; squamous cell carcinoma of the head and neck; endometrial cancer; endometrioid adenocarcinomas of the ovary, serous cystadenocarcinomas, breast cancer; bladder cancer; pancreatic cancer; bone cancer *(e.g.,* high-grade osteosarcoma); pituitary cancer *(e.g.,* pituitary adenomas); colorectal cancer and medullary thyroid cancer. See *e.g.,* U.S. Patent No. 7,754,698; U.S. Patent Application No. 2005/0232919; WO 2009/132081; Bueno R et al. J of Thoracic and Cardiovascular Surgery, 121(2) : 225-233 (2001); Elkanat H & Ratnam M. Frontiers in Bioscience, 11, 506-519 (2006); Fisher R.E. J Nucl Med, 49: 899-906 (2008); Franklin, WA et al. Int J Cancer, Suppl 8: 89-95 (1994); Hartman L.C. et al. Int J Cancer 121: 938-942 (2007); Iwakiri S et al. Annals of Surgical Oncology, 15(3): 889-899; Parker N. et al. Analytical Biochemistry, 338: 284-293 (2005); Weitman, SD et al. Cancer Res 52: 3396-3401 (1992); Saba N.F. et al. Head Neck, 31(4): 475-481 (2009); Yang R et al. Clin Cancer Res 13: 2557-2567 (2007). In some types of cancers (*e.g.*, squamous cell carcinoma of the head and neck), a higher level of FRα expression is associated with a worse prognosis, whereas in other types of cancers (*e.g.*, non-small-cell lung cancers), a higher level of FRα expression is associated with a better prognosis. See, *e.g.,* Iwakiri S et al. Annals of Surgical Oncology, 15(3): 889-899; Saba N.F. et al. Head Neck, 31(4): 475-481 (2009).

Mantovani et al. 1994 assesses the distribution of folate receptor alpha in biological fluids, ultimately concluding that there is no statistically significant difference in the level of folate receptor alpha in the urine of ovarian cancer subjects as compared to that of healthy subjects.

Earlier detection of cancer improves survival rates and quality of life. To improve the likelihood of early detection and treatment, a pressing need exists for non-invasive methods for diagnosing cancer, for determining the level of risk of developing cancer, and for predicting the progression of cancer. The present invention satisfies these needs for FRα-expressing cancers.

### SUMMARY OF THE INVENTION

The present invention is defined by the appended claims. Any subject-matter identified in the application as "invention", "embodiment", "aspect", etc., which exceeds the scope of the invention as represented by the claims does not form part of the claimed invention but only serves as background information to better understand the invention.

The present invention provides methods of assessing whether a subject is afflicted with FRα-expressing cancers such as lung or ovarian cancer, methods of assessing the progression of FRα-expressing cancers such as lung or ovarian cancer in a subject afflicted with the FRα-expressing cancers, methods of stratifying an FRα-expressing cancer subject into one of at least four cancer therapy groups, methods of assessing the efficacy of MORAb-003 treatment of ovarian cancer or lung cancer and kits for assessing whether a subject is afflicted with FRα-expressing cancers such as lung or ovarian cancer or for assessing the progression of FRα-expressing cancers such as lung or ovarian cancer in a subject.

#### *Methods of Assessing Whether a Subject is Afflicted with an Frα Expressing Cancer*

In a first aspect, the present invention provides a method of assessing whether a subject is afflicted with an FRα-expressing cancer, by determining the level of folate receptor alpha (FRα) which is not bound to a cell, in a sample derived from the subject; and comparing the level of folate receptor alpha (FRα) which is not bound to a cell with the level of FRα in a control sample, wherein a difference between the level of FRα in the sample derived from the subject and the level of FRα in the control sample is an indication that the subject is afflicted with an FRα-expressing cancer; wherein the level of FRα in the sample derived from the subject is assessed by contacting the sample with an antibody that binds FRα. In a particular embodiment, the sample is either urine, serum, plasma or ascites.

In another aspect, the present invention is directed to a method of assessing whether a subject is afflicted with an FRα-expressing cancer, by determining the level of folate receptor alpha (FRα) which is not bound to a cell in a urine sample derived from the subject; and comparing the level of folate receptor alpha (FRα) which is not bound to a cell in the urine sample derived from the subject with the level of FRα in a control sample, wherein a difference between the level of FRα in the urine sample derived from the subject and the level of FRα in the control sample is an indication that the subject is afflicted with an FRα-expressing cancer. In a further aspect, the present invention provides a method of assessing whether a subject is afflicted with a cancer that expresses FRα, by determining the level of folate receptor alpha (FRα) which is not bound to a cell in a serum sample derived from the subject; and comparing the level of folate receptor alpha (FRα) which is not bound to a cell in the serum sample derived from the subject with the level of FRα in a control sample, wherein a difference between the level of FRα in the serum sample derived from the subject and the level of FRα in the control sample is an indication that the subject is afflicted with an FRα-expressing cancer.

In various embodiments of the foregoing aspects of the invention, the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer. In a particular embodiment, the FRα-expressing cancer is ovarian cancer. In another embodiment, the FRα-expressing cancer is non-small cell lung cancer, such as an adenocarcinoma.

In another aspect, the present invention is directed to methods of assessing whether a subject is afflicted with ovarian cancer, by determining the level of folate receptor alpha (FRα) which is not bound to a cell in a urine sample derived from the subject, wherein the presence of FRα which is not bound to a cell in the urine sample at a concentration of greater than about 9100 pg/ml is an indication that the subject is afflicted with ovarian cancer.

In various aspects of the foregoing aspects of the invention, the presence of FRα in the urine sample at a concentration of greater than about 9500 pg/mL, about 10,000 pg/mL, about 11,000 pg/mL, about 12,000 pg/mL, about 13,000 pg/mL, about 14,000 pg/mL, about 15,000 pg/mL, about 16,000 pg/mL, about 17,000 pg/mL, about 18,000 pg/mL, about 19,000 pg/mL, or about 20,000 pg/mL is an indication that the subject is afflicted with ovarian cancer.

In various aspects, the level of FRα is determined by contacting the sample with an antibody that binds FRα. For example, the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) the MOV18 antibody;
(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1 , SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;

(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In a particular embodiment, the antibody binds the same epitope as the MORAb-003 antibody. In another embodiment, the antibody includes SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In another embodiment, the antibody is the MOV18 antibody. In yet another embodiment, the antibody binds the same epitope as the MOV18 antibody. In a further embodiment, the antibody comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16). Alternatively or in combination, the antibody includes a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). In certain embodiments, the antibody includes (i) the heavy chain variable region LK26KOLHuVH (SEQ ID NO: 21) and the light chain variable region LK26HuVKPW,Y (SEQ ID NO: 16); the heavy chain variable region LK26HuVH SLF (SEQ ID NO: 19) and the light chain variable region LK26HuVKPW,Y (SEQ ID NO: 16); or the heavy chain variable region LK26HuVH FAIS,N (SEQ ID NO: 18) and the light chain variable region LK26HuVKPW,Y (SEQ ID NO: 16).

In a particular embodiment, the level of FRα in the sample derived from said subject is assessed by contacting the sample with a pair of antibodies selected from the group consisting of (a) MOV18 antibody immobilized to a solid support and labeled MORAB-003 antibody; (b) 9F3 antibody immobilized to a solid support and labeled 24F12 antibody; (c) 26B3 antibody immobilized to a solid support and labeled 19D4 antibody; and (d) 9F3 antibody immobilized to a solid support and labeled 26B3 antibody.

In certain embodiments, the antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody. Alternatively, or in combination, the antibody is labeled, for example, with a label selected from the group consisting of a radio-label, a biotin-label, a chromophore-label, a fluorophore-label, or an enzyme-label.

In certain embodiments, the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

In various embodiments of the foregoing aspects of the invention, the control sample is a standardized control level of FRα in a healthy subject.

In certain embodiments, the sample is treated with guanidine prior to determining the level of FRα in the sample. Alternatively or in combination, the sample is diluted prior to determining the level of FRα in the sample. Alternatively, or in combination, the sample is centrifuged, vortexed, or both, prior to determining the level of FRα in the sample.

In yet another aspect, the present invention is directed to a method of assessing whether a subject is afflicted with ovarian cancer, by determining the level of folate receptor alpha (FRα) which is not bound to a cell in a sample derived from the subject; and comparing the level of folate receptor alpha (FRα) which is not bound to a cell in the sample with the level of FRα in a control sample, wherein a difference between the levels of FRα in the sample derived from the subject and in the control sample is an indication that the subject is afflicted with ovarian cancer; wherein the level of FRα in the sample derived from the subject is assessed by contacting the sample with (a) MOV18 antibody immobilized to a solid support and labeled MORAB-003 antibody, (b) 9F3 antibody immobilized to a solid support and labeled 24F12 antibody, (c) 26B3 antibody immobilized to a solid support and labeled 19D4 antibody, and (d) 9F3 antibody immobilized to a solid support and labeled 26B3 antibody. For example, the sample may be urine, serum, plasma or ascites.

*Methods of Assessing the Progression of an FRα Expressing Cancer in a Subject*

In a further aspect, the present invention is directed to a method of assessing the progression of an FRα-expressing cancer in a subject afflicted with an FRα-expressing cancer, by determining the level of folate receptor alpha (FRα) which is not bound to a cell, in a sample derived from the subject; and comparing the level of folate receptor alpha (FRα) which is not bound to a cell with the level of FRα in a control sample, wherein an increase in the level of FRα in the sample derived from the subject as compared with the level of FRα in the control sample is an indication that the cancer will progress rapidly; and wherein a decrease in the level of FRα in the sample derived from the subject as compared with the level of FRα in the control sample is an indication that the cancer will progress slowly or will regress, thereby assessing the progression of the FRα-expressing cancer in the subject; wherein the level of FRα which is not bound to a cell in the sample derived from the subject is assessed by contacting the sample with an antibody that binds FRα. In a particular embodiment, the sample is urine, serum, plasma or ascites.

In another aspect, the present invention provides a method of assessing the progression of an FRα-expressing cancer in a subject afflicted with an FRα-expressing cancer, by determining the level of folate receptor alpha (FRα) which is not bound to a cell in a urine sample derived from the subject; and comparing the level of folate receptor alpha (FRα) which is not bound to a cell in the urine sample derived from the subject with the level of FRα in a control sample, wherein an increase in the level of FRα in the urine sample derived from the subject as compared with the level of FRα in the control sample is an indication that the cancer will progress rapidly; and wherein a decrease in the level of FRα in the urine sample derived from the subject as compared with the level of FRα in the control sample is an indication that the cancer will progress slowly or will regress, thereby assessing the progression of the FRα-expressing cancer in the subject.

In a further aspect, the present invention provides methods of assessing the progression of an FRα-expressing cancer in a subject afflicted with an FRα-expressing cancer, by determining the level of folate receptor alpha (FRα) which is not bound to a cell in a serum sample derived from the subject; and comparing the level of folate receptor alpha (FRα) which is not bound to a cell in the serum sample derived from the subject with the level of FRα in control sample, wherein an increase in the level of FRα in the serum sample derived from the subject as compared with the level of FRα in the control sample is an indication that the cancer will progress rapidly; and wherein a decrease in the level of FRα in the serum sample derived from the subject as compared with the level of FRα in the control sample is an indication that the cancer will progress slowly or will regress, thereby assessing the progression of the FRα-expressing cancer in the subject.

In various embodiments of the foregoing aspects of the invention, the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer. In a particular embodiment, the FRα-expressing cancer is ovarian cancer. In another embodiment, the FRα-expressing cancer is non-small cell lung cancer, such as an adenocarcinoma.

In another aspect, the present invention is directed to methods of assessing whether a subject is afflicted with ovarian cancer, by determining the level of folate receptor alpha (FRα) which is not bound to a cell in a urine sample derived from the subject, wherein the presence of FRα which is not bound to a cell in the urine sample at a concentration of greater than about 9100 pg/ml is an indication that the subject is afflicted with ovarian cancer.

In various aspects of the foregoing aspects of the invention, the presence of FRα in the urine sample at a concentration of greater than about 9500 pg/mL, about 10,000 pg/mL, about 11,000 pg/mL, about 12,000 pg/mL, about 13,000 pg/mL, about 14,000 pg/mL, about 15,000 pg/mL, about 16,000 pg/mL, about 17,000 pg/mL, about 18,000 pg/mL, about 19,000 pg/mL, or about 20,000 pg/mL is an indication that the subject is afflicted with ovarian cancer.

In various aspects, the level of FRα is determined by contacting the sample with an antibody that binds FRα. For example, the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) the MOV18 antibody;
(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;

(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1 , SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In a particular embodiment, the antibody binds the same epitope as the MORAb-003 antibody. In another embodiment, the antibody includes SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In another embodiment, the antibody is the MOV18 antibody. In yet another embodiment, the antibody binds the same epitope as the MOV18 antibody. In a further embodiment, the antibody comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16). Alternatively or in combination, the antibody includes a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). In certain embodiments, the antibody includes (i) the heavy chain variable region LK26KOLHuVH (SEQ ID NO: 21) and the light chain variable region LK26HuVKPW,Y (SEQ ID NO: 16); the heavy chain variable region LK26HuVH SLF (SEQ ID NO: 19) and the light chain variable region LK26HuVKPW,Y (SEQ ID NO: 16); or the heavy chain variable region LK26HuVH FAIS,N (SEQ ID NO: 18) and the light chain variable region LK26HuVKPW,Y (SEQ ID NO: 16).

In a particular embodiment, the level of FRα in the sample derived from said subject is assessed by contacting the sample with a pair of antibodies selected from the group consisting of (a) MOV18 antibody immobilized to a solid support and labeled MORAB-003 antibody; (b) 9F3 antibody immobilized to a solid support and labeled 24F12 antibody; (c) 26B3 antibody immobilized to a solid support and labeled 19D4 antibody; and (d) 9F3 antibody immobilized to a solid support and labeled 26B3 antibody.

In certain embodiments, the antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody. Alternatively, or in combination, the antibody is labeled, for example, with a label selected from the group consisting of a radio-label, a biotin-label, a chromophore-label, a fluorophore-label, or an enzyme-label.

In certain embodiments, the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

In various embodiments of the foregoing aspects of the invention, the control sample is a standardized control level of FRα in a healthy subject. In another embodiment, the control sample is a sample previously obtained from the subject.

In certain embodiments, the sample is treated with guanidine prior to determining the level of FRα in the sample. Alternatively or in combination, the sample is diluted prior to determining the level of FRα in the sample. Alternatively, or in combination, the sample is centrifuged, vortexed, or both, prior to determining the level of FRα in the sample.

In a further aspect, the present invention provides methods of assessing the progression of ovarian cancer in a subject afflicted with ovarian cancer, by determining the level of folate receptor alpha (FRα) which is not bound to a cell in a sample derived from the subject; and comparing the level of folate receptor alpha (FRα) which is not bound to a cell in the sample with the level of FRα in a control sample, wherein an increase in the level of FRα in the sample derived from the subject as compared with the level of FRα in the control sample is an indication that the ovarian cancer will progress rapidly; and wherein a decrease in the level of FRα in the sample derived from the subject as compared with the level of FRα in the control sample is an indication that the ovarian cancer will progress slowly or will regress, thereby assessing the progression of ovarian cancer in the subject; wherein the level of FRα in the sample derived from the subject is assessed by contacting the sample with (a) MOV18 antibody immobilized to a solid support and labeled MORAB-003 antibody, (b) 9F3 antibody immobilized to a solid support and labeled 24F12 antibody, (c) 26B3 antibody immobilized to a solid support and labeled 19D4 antibody, and (d) 9F3 antibody immobilized to a solid support and labeled 26B3 antibody. For example, the sample may be urine, serum, plasma or ascites.

*<u>Methods of Stratifying an FRα Expressing Cancer into Cancer Therapy Groups</u>*

In a further aspect, the present invention provides a method of stratifying a subject afflicted with an FRα-expressing cancer into one of at least four cancer therapy groups by determining the level of folate receptor alpha (FRα) which is not bound to a cell, in a sample derived from the subject; and stratifying the subject into one of at least four cancer therapy groups based on the level of folate receptor alpha (FRα) which is not bound to a cell; wherein the level of FRα which is not bound to a cell in the sample derived from the subject is assessed by contacting the sample with an antibody that binds FRα. For example, the sample is selected from the group consisting of urine, serum, plasma or ascites.

In yet another aspect, the present invention provides a method of stratifying a subject afflicted with an FRα-expressing cancer into one of at least four cancer therapy groups by determining the level of folate receptor alpha (FRα) which is not bound to a cell in a urine sample derived from the subject; and stratifying the subject into one of at least four cancer therapy groups based on the level of folate receptor alpha (FRα) which is not bound to a cell in the sample. In a further aspect, the present invention is directed to methods of stratifying a subject afflicted with an FRα-expressing cancer into one of at least four cancer therapy groups by determining the level of folate receptor alpha (FRα) which is not bound to a cell in a serum sample derived from the subject; and stratifying the subject into one of at least four cancer therapy groups based on the level of folate receptor alpha (FRα) which is not bound to a cell in the serum sample.

In various embodiments of the foregoing aspects of the invention, the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer. In a particular embodiment, the FRα-expressing cancer is ovarian cancer. In another embodiment, the FRα-expressing cancer is non-small cell lung cancer, such as an adenocarcinoma.

In another aspect, the present invention is directed to methods of assessing whether a subject is afflicted with ovarian cancer, by determining the level of folate receptor alpha (FRα) which is not bound to a cell in a urine sample derived from the subject, wherein the presence of FRα which is not bound to a cell in the urine sample at a concentration of greater than about 9100 pg/ml is an indication that the subject is afflicted with ovarian cancer.

In various aspects of the foregoing aspects of the invention, the presence of FRα in the urine sample at a concentration of greater than about 9500 pg/mL, about 10,000 pg/mL, about 11,000 pg/mL, about 12,000 pg/mL, about 13,000 pg/mL, about 14,000 pg/mL, about 15,000 pg/mL, about 16,000 pg/mL, about 17,000 pg/mL, about 18,000 pg/mL, about 19,000 pg/mL, or about 20,000 pg/mL is an indication that the subject is afflicted with ovarian cancer.

In various aspects, the level of FRα is determined by contacting the sample with an antibody that binds FRα. For example, the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) the MOV18 antibody;

(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1, SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In a particular embodiment, the antibody binds the same epitope as the MORAb-003 antibody. In another embodiment, the antibody includes SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In another embodiment, the antibody is the MOV18 antibody. In yet another embodiment, the antibody binds the same epitope as the MOV18 antibody. In a further embodiment, the antibody comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16). Alternatively or in combination, the antibody includes a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). In certain embodiments, the antibody includes (i) the heavy chain variable region LK26KOLHuVH (SEQ ID NO: 21) and the light chain variable region LK26HuVKPW,Y (SEQ ID NO: 16); the heavy chain variable region LK26HuVH SLF (SEQ ID NO: 19) and the light chain variable region LK26HuVKPW,Y (SEQ ID NO: 16); or the heavy chain variable region LK26HuVH FAIS,N (SEQ ID NO: 18) and the light chain variable region LK26HuVKPW,Y (SEQ ID NO: 16).

In a particular embodiment, the level of FR$\alpha$ in the sample derived from said subject is assessed by contacting the sample with a pair of antibodies selected from the group consisting of (a) MOV18 antibody immobilized to a solid support and labeled MORAB-003 antibody; (b) 9F3 antibody immobilized to a solid support and labeled 24F12 antibody; (c) 26B3 antibody immobilized to a solid support and labeled 19D4 antibody; and (d) 9F3 antibody immobilized to a solid support and labeled 26B3 antibody.

In certain embodiments, the antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody. Alternatively, or in combination, the antibody is labeled, for example, with a label selected from the group consisting of a radio-label, a biotin-label, a chromophore-label, a fluorophore-label, or an enzyme-label.

In certain embodiments, the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

In various embodiments of the foregoing aspects of the invention, the control sample is a standardized control level of FRα in a healthy subject.

In certain embodiments, the sample is treated with guanidine prior to determining the level of FRα in the sample. Alternatively or in combination, the sample is diluted prior to determining the level of FRα in the sample. Alternatively, or in combination, the sample is centrifuged, vortexed, or both, prior to determining the level of FRα in the sample.

In a particular embodiment, the subject is stratified in Stage I, Stage II, Stage III or Stage IV ovarian cancer.

In a further aspect, the present invention provides a method of stratifying an ovarian cancer subject into one of at least four cancer therapy groups by determining the level of folate receptor alpha (FRα) which is not bound to a cell in a sample derived from the subject; and stratifying the subject into one of at least four cancer therapy groups based on the level of folate receptor alpha (FRα) which is not bound to a cell in the sample; wherein the level of FRα in the sample derived from the subject is assessed by contacting the sample with (a) MOV18 antibody immobilized to a solid support and labeled MORAB-003 antibody, (b) 9F3 antibody immobilized to a solid support and labeled 24F12 antibody, (c) 26B3 antibody immobilized to a solid support and labeled 19D4 antibody, and (d) 9F3 antibody immobilized to a solid support and labeled 26B3 antibody. For example, the sample may be urine, serum, plasma or ascites.

*Methods of Monitoring the Efficacy of MORAb-003 Treatment of Ovarian Cancer or Lung Cancer*

In one aspect, the present invention provides a method of monitoring the efficacy of MORAb-003 treatment of ovarian cancer or lung cancer in a subject suffering from ovarian cancer or lung cancer, by determining the level of folate receptor alpha (FRα) which is not bound to a cell, in a sample derived from the subject, wherein the subject has been previously administered MORAb-003; and comparing the level of folate receptor alpha (FRα) which is not bound to a cell with the level of FRα in a control sample, wherein an increase in the level of FRα in the sample derived from the subject as compared with the level of FRα in the control sample is an indication that the MORAb-003 treatment is not efficacious; and wherein a decrease in the level of FRα in the sample derived from the subject as compared with the level of FRα in the control sample is an indication that the MORAb-003 treatment is efficacious. In particular embodiments, the level of FRα which is not bound to a cell in the sample derived from the subject is assessed by contacting the sample with an antibody that binds FRα. For example, the sample may be urine, serum, plasma or ascites.

In a further aspect, the present invention provides a method of monitoring the efficacy of MORAb-003 treatment of ovarian cancer or lung cancer in a subject suffering from ovarian cancer or lung cancer, by determining the level of folate receptor alpha (FRα) which is not bound to a cell in a urine sample derived from the subject, wherein the subject has been previously administered MORAb-003; and comparing the level of folate receptor alpha (FRα) which is not bound to a cell in the urine sample derived from the subject with the level of FRα in a control sample, wherein an increase in the level of FRα in the urine sample derived from the subject as compared with the level of FRα in the control sample is an indication that the MORAb-003 treatment is not efficacious; and wherein a decrease in the level of FRα in the urine sample derived from the subject as compared with the level of FRα in the control sample is an indication that the MORAb-003 treatment is efficacious.

In yet another aspect, the present invention is directed to a method of monitoring the efficacy of MORAb-003 treatment of ovarian cancer or lung cancer in a subject suffering from ovarian cancer or lung cancer, by determining the level of folate receptor alpha (FRα) which is not bound to a cell in a serum sample derived from the subject, wherein the subject has been previously administered MORAb-003; and comparing the level of folate receptor alpha (FRα) which is not bound to a cell in the serum sample derived from the subject with the level of FRα in a control sample, wherein an increase in the level of FRα in the serum sample derived from the subject as compared with the level of FRα in the control sample is an indication that the MORAb-003 treatment is not efficacious; and wherein a decrease in the level of FRα in the serum sample derived from the subject as compared with the level of FRα in the control sample is an indication that the MORAb-003 treatment is efficacious.

In various aspects, the level of FRα is determined by contacting the sample with an antibody that binds FRα. For example, the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;

(c) the MOV18 antibody;
(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1 , SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In a particular embodiment, the antibody binds the same epitope as the MORAb-003 antibody. In another embodiment, the antibody includes SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In another embodiment, the antibody is the MOV18 antibody. In yet another embodiment, the antibody binds the same epitope as the MOV18 antibody. In a further embodiment, the antibody comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16). Alternatively or in combination, the antibody includes a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). In certain embodiments, the antibody includes (i) the heavy chain variable region LK26KOLHuVH (SEQ ID NO: 21) and the light chain variable region LK26HuVKPW,Y (SEQ ID NO: 16); the heavy chain variable region LK26HuVH SLF (SEQ ID NO: 19) and the light chain variable region LK26HuVKPW,Y (SEQ ID NO: 16); or the heavy chain variable region LK26HuVH FAIS,N (SEQ ID NO: 18) and the light chain variable region LK26HuVKPW,Y (SEQ ID NO: 16).

In a particular embodiment, the level of FRα in the sample derived from said subject is assessed by contacting the sample with a pair of antibodies selected from the group consisting of (a) MOV18 antibody immobilized to a solid support and labeled MORAB-003 antibody; (b) 9F3 antibody immobilized to a solid support and labeled 24F12 antibody; (c) 26B3 antibody immobilized to a solid support and labeled 19D4 antibody; and (d) 9F3 antibody immobilized to a solid support and labeled 26B3 antibody.

In certain embodiments, the antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody. Alternatively, or in combination, the antibody is labeled, for example, with a label selected from the group consisting of a radio-label, a biotin-label, a chromophore-label, a fluorophore-label, or an enzyme-label.

In certain embodiments, the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

In various embodiments of the foregoing aspects of the invention, the control sample is a standardized control level of FRα in a healthy subject. In another embodiment, the control sample is a sample previously obtained from the subject.

In certain embodiments, the sample is treated with guanidine prior to determining the level of FRα in the sample. Alternatively or in combination, the sample is diluted prior to determining the level of FRα in the sample. Alternatively, or in combination, the sample is centrifuged, vortexed, or both, prior to determining the level of FRα in the sample.

*Methods of Predicting Whether a Subject Will Respond to MORAb-003 treatment*

In one aspect, the present invention provides a method for predicting whether a subject suffering from an FRα expressing cancer, such as ovarian cancer or lung cancer, will respond to treatment with MORAb-003, by determining the level of folate receptor alpha (FRα) which is not bound to a cell in a sample derived from the subject; and comparing the level of folate receptor alpha (FRα) which is not bound to a cell in the sample derived from the subject with the level of FRα in a control sample, wherein a difference between the level of FRα in the sample derived from the subject and the level of FRα in the control sample is an indication that the subject will respond to treatment with MORAb-003.

In one aspect, the present invention provides a method for predicting whether a subject suffering from an FRα expressing cancer, such as ovarian cancer or lung cancer, will respond to treatment with MORAb-003, by determining the level of folate receptor alpha (FRα) which is not bound to a cell in a urine sample derived from the subject; and comparing the level of folate receptor alpha (FRα) which is not bound to a cell in the urine sample derived from the subject with the level of FRα in a control sample, wherein a difference between the level of FRα in the urine sample derived from the subject and the level of FRα in the control sample is an indication that the subject will respond to treatment with MORAb-003.

In a further aspect, the present invention provides a method for predicting whether a subject suffering from an FRα expressing cancer, such as ovarian cancer or lung cancer, will respond to treatment with MORAb-003, by determining the level of folate receptor alpha (FRα) which is not bound to a cell in a serum sample derived from the subject; and comparing the level of folate receptor alpha (FRα) which is not bound to a cell in the serum sample derived from the subject with the level of FRα in a control sample, wherein a difference between the level of FRα in the serum sample derived from the subject and the level of FRα in the control sample is an indication that the subject will respond to treatment with MORAb-003.

In further embodiments, the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer. In a particular embodiment, the FRα-expressing cancer is ovarian cancer. In another embodiment, the FRα-expressing lung cancer is non-small cell lung cancer, such as adenocarcinoma.

In a further aspect, the present invention provides methods for predicting whether a subject suffering from ovarian cancer will respond to treatment with MORAb-003, by determining the level of folate receptor alpha (FRα) which is not bound to a cell in a urine sample derived from the subject, wherein the presence of FRα which is not bound to a cell in the urine sample at a concentration of greater than about 9100 pg/ml is an indication that the subject will respond to treatment with MORAb-003.

In various embodiments of the foregoing aspects of the invention, the presence of FRα in the urine sample at a concentration of greater than about 9500 pg/mL, about 10,000 pg/mL, about 11,000 pg/mL, about 12,000 pg/mL, about 13,000 pg/mL, about 14,000 pg/mL, about 15,000 pg/mL, about 16,000 pg/mL, about 17,000 pg/mL, about 18,000 pg/mL, about 19,000 pg/mL, or about 20,000 pg/mL is an indication that the subject is afflicted with ovarian cancer.

In various embodiments of the foregoing aspects of the invention, the level of FRα is determined by contacting the sample with an antibody that binds FRα. For example, the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;

(c) the MOV18 antibody;
(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1 , SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In a particular embodiment, the antibody binds the same epitope as the MORAb-003 antibody. In another embodiment, the antibody includes SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In another embodiment, the antibody is the MOV18 antibody. In yet another embodiment, the antibody binds the same epitope as the MOV18 antibody. In a further embodiment, the antibody comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16). Alternatively or in combination, the antibody includes a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). In certain embodiments, the antibody includes (i) the heavy chain variable region LK26KOLHuVH (SEQ ID NO: 21) and the light chain variable region LK26HuVKPW,Y (SEQ ID NO: 16); the heavy chain variable region LK26HuVH SLF (SEQ ID NO: 19) and the light chain variable region LK26HuVKPW,Y (SEQ ID NO: 16); or the heavy chain variable region LK26HuVH FAIS,N (SEQ ID NO: 18) and the light chain variable region LK26HuVKPW,Y (SEQ ID NO: 16).

In a particular embodiment, the level of FR$\alpha$ in the sample derived from said subject is assessed by contacting the sample with a pair of antibodies selected from the group consisting of (a) MOV18 antibody immobilized to a solid support and labeled MORAB-003 antibody; (b) 9F3 antibody immobilized to a solid support and labeled 24F12 antibody; (c) 26B3 antibody immobilized to a solid support and labeled 19D4 antibody; and (d) 9F3 antibody immobilized to a solid support and labeled 26B3 antibody.

In certain embodiments, the antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody. Alternatively, or in combination, the antibody is labeled, for example, with a label selected from the group consisting of a radio-label, a biotin-label, a chromophore-label, a fluorophore-label, or an enzyme-label.

In certain embodiments, the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

In various embodiments of the foregoing aspects of the invention, the control sample is a standardized control level of FRα in a healthy subject.

In certain embodiments, the sample is treated with guanidine prior to determining the level of FRα in the sample. Alternatively or in combination, the sample is diluted prior to determining the level of FRα in the sample. Alternatively, or in combination, the sample is centrifuged, vortexed, or both, prior to determining the level of FRα in the sample.

In a further aspect, the present invention provides a method for predicting whether a subject suffering from an FRα expressing cancer, such as ovarian cancer or lung cancer, will respond to treatment with MORAb-003, by determining the level of folate receptor alpha (FRα) which is not bound to a cell in a sample derived from the subject; and comparing the level of folate receptor alpha (FRα) which is not bound to a cell in the sample with the level of FRα in a control sample, wherein a difference between the levels of FRα in the sample derived from the subject and in the control sample is an indication that the subject will respond to treatment with MORAb-003; wherein the level of FRα in the sample derived from the subject is assessed by contacting the sample with (a) MOV18 antibody immobilized to a solid support and labeled MORAB-003 antibody, (b) 9F3 antibody immobilized to a solid support and labeled 24F12 antibody, (c) 26B3 antibody immobilized to a solid support and labeled 19D4 antibody, and (d) 9F3 antibody immobilized to a solid support and labeled 26B3 antibody. For example, the sample may be urine, serum, plasma or ascites.

In various embodiments of the foregoing aspects of the invention, the MORAb-003 for treatment is (a) an antibody that comprises the heavy chain amino acid sequence as set forth in SEQ ID NO:7 and the light chain amino acid sequence as set forth in SEQ ID NO:8; (b) an antibody that binds the same epitope as the MORAb-003 antibody; or (c) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3.

*Methods of Treating a Subject Having Ovarian Cancer or Lung Cancer*

In another aspect, the present invention provides methods of treating a subject having ovarian cancer or lung cancer by determining the level of folate receptor alpha (FRα) which is not bound to a cell, in a sample derived from said subject (for example, urine, serum, plasma or ascites); and comparing the level of folate receptor alpha (FRα) which is not bound to a cell with the level of FRα in a control sample, wherein a difference between the level of FRα in the sample derived from said subject and the level of FRα in the control sample is an indication that the subject is afflicted with ovarian cancer or lung cancer; and administering a therapeutically effective amount of MORAb-003 to said subject.

In another aspect, the present invention provides methods of treating a subject having ovarian cancer or lung cancer by determining the level of folate receptor alpha (FRα) which is not bound to a cell, in a urine sample derived from said subject; and comparing the level of folate receptor alpha (FRα) which is not bound to a cell with the level of FRα in a control sample, wherein a difference between the level of FRα in the urine sample derived from said subject and the level of FRα in the control sample is an indication that the subject is afflicted with ovarian cancer or lung cancer; and administering a therapeutically effective amount of MORAb-003 to said subject.

In another aspect, the present invention provides methods of treating a subject having ovarian cancer or lung cancer by determining the level of folate receptor alpha (FRα) which is not bound to a cell, in a serum sample derived from said subject; and comparing the level of folate receptor alpha (FRα) which is not bound to a cell with the level of FRα in a control sample, wherein a difference between the level of FRα in the serum sample derived from said subject and the level of FRα in the control sample is an indication that the subject is afflicted with ovarian cancer or lung cancer; and administering a therapeutically effective amount of MORAb-003 to said subject.

In a further aspect, the present invention provides methods for treating a subject suffering from ovarian cancer by determining the level of folate receptor alpha (FRα) which is not bound to a cell in a urine sample derived from the subject, wherein the presence of FRα which is not bound to a cell in the urine sample at a concentration of greater than about 9100 pg/ml is an indication that the subject will respond to treatment with MORAb-003; and administering a therapeutically effective amount of MORAb-003 to said subject.

In particular embodiments, the level of FRα which is not bound to a cell in the sample derived from said subject is assessed by contacting the sample with an antibody that binds FRα.

In various embodiments of the foregoing aspects of the invention, the presence of FRα in the urine sample at a concentration of greater than about 9500 pg/mL, about 10,000 pg/mL, about 11,000 pg/mL, about 12,000 pg/mL, about 13,000 pg/mL, about 14,000 pg/mL, about 15,000 pg/mL, about 16,000 pg/mL, about 17,000 pg/mL, about 18,000 pg/mL, about 19,000 pg/mL, or about 20,000 pg/mL is an indication that the subject is afflicted with ovarian cancer.

In various embodiments of the foregoing aspects of the invention, the level of FRα is determined by contacting the sample with an antibody that binds FRα. For example, the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) the MOV18 antibody;
(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1, SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In a particular embodiment, the antibody binds the same epitope as the MORAb-003 antibody. In another embodiment, the antibody includes SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In another embodiment, the antibody is the MOV18 antibody. In yet another embodiment, the antibody binds the same epitope as the MOV18 antibody. In a further embodiment, the antibody comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16). Alternatively or in combination, the antibody includes a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). In certain embodiments, the antibody includes (i) the heavy chain variable region LK26KOLHuVH (SEQ ID NO: 21) and the light chain variable region LK26HuVKPW,Y (SEQ ID NO: 16); the heavy chain variable region LK26HuVH SLF (SEQ ID NO: 19) and the light chain variable region LK26HuVKPW,Y (SEQ ID NO: 16); or the heavy chain variable region LK26HuVH FAIS,N (SEQ ID NO: 18) and the light chain variable region LK26HuVKPW,Y (SEQ ID NO: 16).

In a particular embodiment, the level of FRα in the sample derived from said subject is assessed by contacting the sample with a pair of antibodies selected from the group consisting of (a) MOV18 antibody immobilized to a solid support and labeled MORAB-003 antibody; (b) 9F3 antibody immobilized to a solid support and labeled 24F12 antibody; (c) 26B3 antibody immobilized to a solid support and labeled 19D4 antibody; and (d) 9F3 antibody immobilized to a solid support and labeled 26B3 antibody.

In certain embodiments, the antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody. Alternatively, or in combination, the antibody is labeled, for example, with a label selected from the group consisting of a radio-label, a biotin-label, a chromophore-label, a fluorophore-label, or an enzyme-label.

In certain embodiments, the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

In various embodiments of the foregoing aspects of the invention, the control sample is a standardized control level of FRα in a healthy subject.

In certain embodiments, the sample is treated with guanidine prior to determining the level of FRα in the sample. Alternatively or in combination, the sample is diluted prior to determining the level of FRα in the sample. Alternatively, or in combination, the sample is centrifuged, vortexed, or both, prior to determining the level of FRα in the sample.

In a further aspect, the present invention provides a method for treating a subject suffering from ovarian cancer or lung cancer, by determining the level of folate receptor alpha (FRα) which is not bound to a cell in a sample derived from the subject; and comparing the level of folate receptor alpha (FRα) which is not bound to a cell in the sample with the level of FRα in a control sample, wherein a difference between the levels of FRα in the sample derived from the subject and in the control sample is an indication that the subject will respond to treatment with MORAb-003; wherein the level of FRα in the sample derived from the subject is assessed by contacting the sample with (a) MOV18 antibody immobilized to a solid support and labeled MORAB-003 antibody, (b) 9F3 antibody immobilized to a solid support and labeled 24F12 antibody, (c) 26B3 antibody immobilized to a solid support and labeled 19D4 antibody, and (d) 9F3 antibody immobilized to a solid support and labeled 26B3 antibody. For example, the sample may be urine, serum, plasma or ascites.

In various embodiments of the foregoing aspects of the invention, the MORAb-003 for treatment is (a) an antibody that comprises the heavy chain amino acid sequence as set forth in SEQ ID NO:7 and the light chain amino acid sequence as set forth in SEQ ID NO:8; (b) an antibody that binds the same epitope as the MORAb-003 antibody; or (c) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3.

*Kits of the Invention*

In one aspect, the present invention provides a kit for assessing whether a subject is afflicted with an FRα-expressing cancer or for assessing the progression of an FRα-expressing cancer in a subject, the kit including means for determining the level of folate receptor alpha (FRα) which is not bound to a cell in a sample derived from the subject; and instructions for use of the kit to assess whether the subject is afflicted with an FRα-expressing cancer or to assess the progression of an FRα-expressing cancer. For example, the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer. In a particular embodiment, the FRα-expressing cancer is ovarian cancer. In yet another embodiment, the FRα-expressing cancer is non-small cell lung cancer, for example, adenocarcinoma. In a further embodiment, the sample is either urine, serum, plasma or ascites.

In a further embodiment, the means includes a folate receptor alpha (FRα) binding agent, for example, an antibody. In a further embodiment, the antibody is selected from the group consisting of

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) the MOV18 antibody;

(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1 , SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

In certain embodiments, the antibody is labeled including, but not limited to, a radio-label, a biotin-label, a chromophore-label, a fluorophore-label, or an enzyme-label.
In yet another embodiment, the kit includes a means for obtaining a sample from the subject.
The present invention is further illustrated by the following detailed description and drawings.

## BRIEF DESCRIPTION OF THE DRAWINGS

Figure 1 is a schematic depiction of an electrochemiluminescence immunoassay (ECLIA) method for assessing the FR$\alpha$ in urine as described in the Examples. MOV18 antibody attached to solid supports bound FR$\alpha$ in urine. The FR$\alpha$ was subsequently detected by binding to Ru-labeled MORAb-003 antibody.
Figure 2 shows the distribution of FR$\alpha$ levels in the urine of ovarian cancer subjects and normal control subjects as measured by ECLIA (see Example 1).
Figure 3 depicts the detection of FR$\alpha$ in urine of ovarian cancer patients (pale band on lane 1, clear band on lane 2) using immunoblotting (see Example 5).
Figure 4 shows the distribution of FR$\alpha$ levels in the urine of ovarian cancer subjects and normal control subjects as measured by ECLIA after the urine was treated with guanidine, as described in Example 7.
Figure 5 depicts an ROC curve showing the sensitivity and specificity of the ECLIA measurement of FR$\alpha$ levels in urine after the urine was treated with guanidine, as described in Example 7. The area under the curve (AUC) measures the accuracy of the test in separating ovarian cancer from control subjects. A cutoff value (above which the test results were considered abnormal) of 9100 pg/mL was used.

Figure 6 shows the distribution of FRα levels in ovarian cancer (OC) and normal control subjects after correction for creatinine levels. There is a statistically significant difference between ovarian cancer patients and controls in creatinine-corrected levels of FRα (p=0.007) (see Example 8).

Figure 7 depicts an ROC analysis of creatinine-corrected FRα levels determined using electrochemiluminescence assay (ECLIA) of guanidine-treated urine samples (see Example 8).

Figure 8 is a schematic depiction of the enzyme immunoassay (EIA) method used for assessing the level of FRα (*i.e.,* FRα) in samples, as described in Example 9. MOV-18 served as the capture antibody, which bound FRα from biological fluids. The FRα was detected by binding to biotinylated MORAb-003, which was detected using avidin conjugated to horseradish peroxidase (avidin-HRP).

Figure 9 depicts results obtained for the measurement of FRα in serum using one- and two-step incubation procedures, as described in Example 9.

Figure 10 is a schematic depiction of the three different combinations of capture and detector antibodies that were used with the enzyme immunoassay (EIA) method for assessing the level of FRα in human plasma, as described in Example 11.

Figure 11 shows the plasma concentrations of FRα (pg/mL) for individual subjects determined using EIA with three combinations of capture and detector antibodies, as described in Example 11.

Figure 12 depicts the relationship between OD values and FRα concentrations (see Example 11).

Figure 13 shows the distribution of plasma FRα concentrations in subjects with ovarian cancer and normal control subjects as determined using EIA (see Example 12).

Figure 14 depicts the correlation between FRα plasma concentrations determined using EIA and ECLIA (see Example 12).

Figure 15 shows correlations between ECLIA measures of FRα levels in serum and urine. The correlation for lung cancer patients was r=0.24 (upper panel) and the correlation for ovarian cancer patients was r=-0.76 (lower panel) (see Example 13).

Figure 16 shows the correlation of serum versus plasma FRα levels for assays conducted using pair 1 (see Example 16).

Figure 17 shows the correlation of serum versus plasma FRα levels for assays conducted using pair 2 (see Example 16).

Figure 18 shows the correlation of serum FRα levels for assays conducted using pair 1 versus pair 2 (see Example 16).

Figure 19 shows the correlation of plasma FRα levels for assays conducted using pair 1 versus pair 2 (see Example 16).

Figure 20 shows the intraday correlation of serum FRα levels for assays conducted using pair 2 (Example 16).

## DETAILED DESCRIPTION OF THE INVENTION

The present invention is based, at least in part, on the unexpected discovery that folate receptor alpha (FRα), not bound to a cell, is found at elevated levels in the body fluids, for example, urine or serum, of a subject having an FRα-expressing cancer such as lung or ovarian cancer as compared to a control sample. Moreover, the present invention is based, at least in part, on the identification of an immunological assay that exhibits the necessary sensitivity for assessing FRα levels in samples, where prior attempts to do so repeatedly failed. As a result, the present invention provides methods for diagnosing an FRα-expressing cancer by assessing levels of an FRα not bound to a cell in samples derived from the subject. Indeed, the present invention overcomes the challenges observed during prior attempts to develop an FRα based diagnostic assay for FRα-expressing cancers such as ovarian cancer by providing an immunological assay capable of accurately assessing levels of FRα not bound to a cell in samples.

Accordingly, methods and kits for assessing whether a subject has or is at risk for developing an FRα-expressing cancer and, further, for assessing the progression of FRα-expressing cancer are provided. In various embodiments, the methods involve the comparison of levels of FRα not bound to a cell in samples, for example, urine and serum, as compared to control levels, in assessing the presence, degree or risk of development of ovarian cancer in the subject. In particular embodiments, the methods involve the use of the MORAb-003 antibody, antibodies that bind the same epitope as the MORAb-003 antibody or antibodies having SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3, in assessing the levels of FRα not bound to a cell in a sample, *e.g.*, urine or serum. Alternatively or in addition, the MOV18 antibody or an antibody that binds the same epitope of the MOV18 antibody, the 548908 antibody, an antibody that binds the same epitope of the 548908 antibody, the 6D398 antibody or an antibody that binds the same epitope of the 548908 antibody may be used in accordance with the methods of the present invention.

Various aspects of the invention are described in further detail in the following subsections:

## I. Definitions

As used herein, each of the following terms has the meaning associated with it in this section.

The articles "a" and "an" are used herein to refer to one or to more than one (*i.e.* to at least one) of the grammatical object of the article. By way of example, "an element" means one element or more than one element.

As used herein, the term "subject" refer to human and non-human animals, including veterinary subjects. The term "non-human animal" includes all vertebrates, *e.g.*, mammals and non-mammals, such as non-human primates, mice, rabbits, sheep, dog, cat, horse, cow, chickens, amphibians, and reptiles. In a preferred embodiment, the subject is a human.

The terms "cancer" or "tumor" are well known in the art and refer to the presence, *e.g.,* in a subject, of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within a subject, or may be non-tumorigenic cancer cells, such as leukemia cells. As used herein, the term "cancer" includes pre-malignant as well as malignant cancers.

As used herein, an "FRα-expressing cancer" includes any type of cancer characterized in that the cancer cells express FRα. In particular embodiments, the FRα expressing cancer includes cancerous conditions characterized in that the cancer cells are capable of secreting, shedding, exporting or releasing FRα in such a manner that elevated levels of FRα are detectable in a biological sample from the subject. FRα-expressing cancers include, but are not limited to, lung cancer (*e.g.*, bronchioalveolar carcinomas, carcinoid tumors, and non-small cell lung cancers, such as adenocarcinomas); mesothelioma; ovarian cancer; renal cancer; brain cancer *(e.g.,* anaplastic ependymoma and cerebellar juvenile pilocytic astrocytoma); cervical cancer; nasopharyngeal cancer; mesodermally derived tumor; squamous cell carcinoma of the head and neck; endometrial cancer; endometrioid adenocarcinomas of the ovary, serous cystadenocarcinomas, breast cancer; bladder cancer; pancreatic cancer; bone cancer *(e.g.,* high-grade osteosarcoma); pituitary cancer *(e.g.,* pituitary adenoma). See *e.g.,* U.S. Patent No. 7,754,698; U.S. Patent Application No. 2005/0232919; WO 2009/132081; Bueno R et al. J of Thoracic and Cardiovascular Surgery, 121(2) : 225-233 (2001); Elkanat H & Ratnam M. Frontiers in Bioscience, 11, 506-519 (2006); Franklin, WA et al. Int J Cancer, Suppl 8: 89-95 (1994); Hartman L.C. et al. Int J Cancer 121: 938-942 (2007); Iwakiri S et al. Annals of Surgical Oncology, 15(3): 889-899; Weitman, SD et al. Cancer Res 52: 3396-3401 (1992); Saba N.F. et al. Head Neck, 31(4): 475-481 (2009); Yang R et al. Clin Cancer Res 13: 2557-2567 (2007). In a particular embodiment, the FRα-expressing cancer is ovarian cancer. In another embodiment, the FRα-expressing cancer is lung cancer such as non-small cell lung cancer. In other embodiments, the FRα-expressing cancer is colorectal cancer and medullary thyroid cancer.

As used herein, a subject who is "afflicted with an FRα-expressing cancer" is one who is clinically diagnosed with such a cancer by a qualified clinician (for example, by the methods of the present invention), or one who exhibits one or more signs or symptoms (for example, elevated levels of FRα in biological fluids) of such a cancer and is subsequently clinically diagnosed with such a cancer by a qualified clinician (for example, by the methods of the present invention). A non-human subject that serves as an animal model of FRα-expressing cancer may also fall within the scope of the term a subject "afflicted with an FRα-expressing cancer."

The term "ovarian cancer" refers to the art recognized disease and includes each of epithelial ovarian cancer (EOC; >90% of ovarian cancer in Western countries), germ cell tumors (*circa* 2-3% of ovarian cancer), and stromal ovarian cancer. Ovarian cancer is stratified into different groups based on the differentiation of the tumor tissue. In grade I, the tumor tissue is well differentiated. In grade II, tumor tissue is moderately well differentiated. In grade III, the tumor tissue is poorly differentiated. This grade correlates with a less favorable prognosis than grades I and II.

Ovarian cancer is stratified into different stages based on the spread of the cancer. Stage I is generally confined within the capsule surrounding one (stage IA) or both (stage IB) ovaries, although in some stage I (*i.e.* stage IC) cancers, malignant cells may be detected in ascites, in peritoneal rinse fluid, or on the surface of the ovaries. Stage II involves extension or metastasis of the tumor from one or both ovaries to other pelvic structures. In stage IIA, the tumor extends or has metastasized to the uterus, the fallopian tubes, or both. Stage IIB involves extension of the tumor to the pelvis. Stage IIC is stage IIA or IIB in which malignant cells may be detected in ascites, in peritoneal rinse fluid, or on the surface of the ovaries. In stage III, the tumor comprises at least one malignant extension to the small bowel or the omentum, has formed extrapelvic peritoneal implants of microscopic (stage IIIA) or macroscopic (< 2 centimeter diameter, stage IIIB; > 2 centimeter diameter, stage IIIC) size, or has metastasized to a retroperitoneal or inguinal lymph node (an alternate indicator of stage IIIC). In stage IV, distant (*i.e.* non-peritoneal) metastases of the tumor can be detected.

The durations of the various stages of ovarian cancer are not presently known, but are believed to be at least about a year each (Richart et al., 1969, Am. J. Obstet. Gynecol. 105:386). Prognosis declines with increasing stage designation. For example, 5-year survival rates for human subjects diagnosed with stage I, II, III, and IV ovarian cancer are 80%, 57%, 25%, and 8%, respectively.

Each of the foregoing types, groups and stages of ovarian cancer are encompassed by the term "ovarian cancer" as used herein.

As used herein, the term "lung cancer" refers to a disease in tissues of the lung involving uncontrolled cell growth, which, in some cases, leads to metastasis. Lung cancer is the most common cause of cancer-related death in men and women. The majority of primary lung cancers are carcinomas of the lung, derived from epithelial cells. The main types of lung cancer are small cell lung carcinoma (SCLC) and non-small cell lung carcinoma (NSCLC). In a particular embodiment, the FRα-expressing cancer is a non-small cell lung cancer.

Small cell lung cancer or small cell lung carcinoma (SCLC) is a malignant cancer of the lung, wherein the cancer cells have a flat shape and scanty cytoplasm; therefore, SCLC is sometimes called "oat cell carcinoma." SCLC is generally more metastatic than NSCLC and is sometimes seen in combination with squamous cell carcinomas.

As used herein, the term "non-small cell lung cancer," also known as non-small cell lung carcinoma (NSCLC), refers to epithelial lung cancer other than small cell lung carcinoma (SCLC). There are three main sub-types: adenocarcinoma, squamous cell lung carcinoma, and large cell lung carcinoma. Other less common types of non-small cell lung cancer include pleomorphic, carcinoid tumor, salivary gland carcinoma, and unclassified carcinoma. Adenocarcinomas account for approximately 40% of lung cancers, and are the most common type of lung cancer in people who have never smoked. Squamous cell carcinomas account for about 25% of lung cancers. Squamous cell carcinoma of the lung is more common in men than in women and is even more highly correlated with a history of tobacco smoking than are other types of lung carcinoma. There are at least four variants (papillary, small cell, clear cell, and basaloid) of squamous cell carcinoma of the lung. Large cell lung carcinomas are a heterogeneous group of malignant neoplasms originating from transformed epithelial cells in the lung. Large cell lung carcinomas are carcinomas that lack light microscopic characteristics of small cell carcinoma, squamous cell carcinoma, or adenocarcinoma.

Different staging systems are used for SCLC and NSCLC. SCLC is categorized as limited disease confined to the ipsilateral hemithorax or as extensive disease with metastasis beyond the ipsilateral hemithorax.

NSCLC may be categorized using the tumor-nodes-metastasis (TNM) staging system. See Spira J & Ettinger, D.S. Multidisciplinary management of lung cancer, N Engl J Med, 350:382- (2004) (hereinafter Spira); Greene FL, Page DL, Fleming ID, Fritz AG, Balch CM, Haller DG, et al (eds). AJCC Cancer Staging Manual. 6th edition. New York: Springer-Verlag, 2002:167-77 (hereinafter Greene); Sobin LH, Wittekind CH (eds). International Union Against Cancer. TNM classification of malignant tumours. 6th edition. New York: Wiley-Liss (2002) (hereinafter Sobin). In addition, NSCLC is typically treated according to the stage of cancer determined by the following classification scheme (see http://www.cancer.gov/cancertopics/pdq/treatment/non-small-cell-lung/Patient/page2#Keypoint10).

In the *occult (hidden) stage*, cancer cells are found in sputum (mucus coughed up from the lungs), but no tumor can be found in the lung by imaging or bronchoscopy, or the tumor is too small to be checked.

In *stage 0 (carcinoma in situ)*, abnormal cells are found in the lining of the airways. These abnormal cells may become cancer and spread into nearby normal tissue. Stage 0 is also called carcinoma in situ.

Stage I, in which cancer has formed, is divided into stages IA and IB.

In *Stage IA*, the tumor is in the lung only and is 3 centimeters or smaller.

In *Stage IB,* the cancer has not spread to the lymph nodes and one or more of the following is true: (i) The tumor is larger than 3 centimeters but not larger than 5 centimeters; (ii) cancer has spread to the main bronchus and is at least 2 centimeters below where the trachea joins the bronchus; (iii) cancer has spread to the innermost layer of the membrane that covers the lung; (iv) part of the lung has collapsed or developed pneumonitis (inflammation of the lung) in the area where the trachea joins the bronchus.

In *Stage IIA*, cancer has spread to certain lymph nodes on the same side of the chest as the primary tumor; the cancer is (a) 5 cm or smaller, (b) has spread to the main bronchus, and/or (c) has spread to the innermost layer of the lung lining. OR, cancer has not spread to lymph nodes; the cancer is (d) larger than 5 cm but not larger than 7 cm, (e) has spread to the main bronchus, and/or (f) has spread to the innermost layer of the lung lining. Part of the lung may have collapsed or become inflamed. Stage IIA is divided into two sections depending on the size of the tumor, where the tumor is found, and whether there is cancer in the lymph nodes. In the first section, cancer has spread to lymph nodes on the same side of the chest as the tumor. The lymph nodes with cancer are within the lung or near the bronchus. Also, one or more of the following is true: (i) the tumor is not larger than 5 centimeters, (ii) cancer has spread to the main bronchus and is at least 2 centimeters below where the trachea joins the bronchus, (iii) cancer has spread to the innermost layer of the membrane that covers the lung, (iv) part of the lung has collapsed or developed pneumonitis (inflammation of the lung) in the area where the trachea joins the bronchus. In the second section, cancer has not spread to lymph nodes and one or more of the following is true: (i) the tumor is larger than 5 centimeters but not larger than 7 centimeters, (ii) cancer has spread to the main bronchus and is at least 2 centimeters below where the trachea joins the bronchus, (iii) cancer has spread to the innermost layer of the membrane that covers the lung, (iv) part of the lung has collapsed or developed pneumonitis (inflammation of the lung) in the area where the trachea joins the bronchus.

In *Stage IIB*, cancer has spread to certain lymph nodes on the same side of the chest as the primary tumor; the cancer is (a) larger than 5 cm but not larger than 7 cm, (b) has spread to the main bronchus, and/or (c) has spread to the innermost layer of the lung lining. Part of the lung may have collapsed or become inflamed. Alternatively, (d) the cancer is larger than 7 cm; (e) has spread to the main bronchus, (f) the diaphragm, (g) the chest wall or the lining of the chest wall; and/or (h) has spread to the membrane around the heart. There may be one or more separate tumors in the same lobe of the lung; cancer may have spread to the nerve that controls the diaphragm; the whole lung may have collapsed or become inflamed. Stage IIB is divided into two sections depending on the size of the tumor, where the tumor is found, and whether there is cancer in the lymph nodes. In the first section, cancer has spread to nearby lymph nodes on the same side of the chest as the tumor. The lymph nodes with cancer are within the lung or near the bronchus. Also, one or more of the following is true: (i) the tumor is larger than 5 centimeters but not larger than 7 centimeters, (ii) cancer has spread to the main bronchus and is at least 2 centimeters below where the trachea joins the bronchus, (iii) cancer has spread to the innermost layer of the membrane that covers the lung, (iv) part of the lung has collapsed or developed pneumonitis (inflammation of the lung) in the area where the trachea joins the bronchus. In the second section, cancer has not spread to lymph nodes and one or more of the following is true: (i) the tumor is larger than 7 centimeters, (ii) cancer has spread to the main bronchus (and is less than 2 centimeters below where the trachea joins the bronchus), the chest wall, the diaphragm, or the nerve that controls the diaphragm, (iii) cancer has spread to the membrane around the heart or lining the chest wall, (iv) the whole lung has collapsed or developed pneumonitis (inflammation of the lung), (v) there are one or more separate tumors in the same lobe of the lung.

*Stage IIIA* is divided into three sections depending on the size of the tumor, where the tumor is found, and which lymph nodes have cancer (if any). In the first section of Stage IIIA, cancer has spread to lymph nodes on the same side of the chest as the tumor. The lymph nodes with cancer are near the sternum (chest bone) or where the bronchus enters the lung. Also, the tumor may be any size; part of the lung (where the trachea joins the bronchus) or the whole lung may have collapsed or developed pneumonitis (inflammation of the lung); there may be one or more separate tumors in the same lobe of the lung; and cancer may have spread to any of the following: (i) main bronchus, but not the area where the trachea joins the bronchus, (ii) chest wall, (iii) diaphragm and the nerve that controls it, (iv) membrane around the lung or lining the chest wall, (iv) membrane around the heart. In the second section of Stage IIIA, cancer has spread to lymph nodes on the same side of the chest as the tumor. The lymph nodes with cancer are within the lung or near the bronchus. Also, the tumor may be any size; the whole lung may have collapsed or developed pneumonitis (inflammation of the lung); there may be one or more separate tumors in any of the lobes of the lung with cancer; and cancer may have spread to any of the following: (i) main bronchus, but not the area where the trachea joins the bronchus, (ii) chest wall, (iii) diaphragm and the nerve that controls it, (iv) membrane around the lung or lining the chest wall, (v) heart or the membrane around it, (vi) major blood vessels that lead to or from the heart, (vi) trachea, (vii) esophagus, (viii) nerve that controls the larynx (voice box), (ix) sternum (chest bone) or backbone, (x) carina (where the trachea joins the bronchi). In the third section of Stage IIIA, cancer has not spread to the lymph nodes and the tumor may be any size, and cancer has spread to any of the following: (i) heart, (ii) major blood vessels that lead to or from the heart, (iii) trachea, (iv) esophagus, (v) nerve that controls the larynx (voice box), (vi) sternum (chest bone) or backbone, (vi) carina (where the trachea joins the bronchi).

*Stage IIIB* is divided into two sections depending on the size of the tumor, where the tumor is found, and which lymph nodes have cancer. In the first section, cancer has spread to lymph nodes above the collarbone or to lymph nodes on the opposite side of the chest as the tumor; the tumor may be any size; part of the lung (where the trachea joins the bronchus) or the whole lung may have collapsed or developed pneumonitis (inflammation of the lung); there may be one or more separate tumors in any of the lobes of the lung with cancer; and cancer may have spread to any of the following: (i) main bronchus, (ii) chest wall, (iii) diaphragm and the nerve that controls it, (iv) membrane around the lung or lining the chest wall, (iv) heart or the membrane around it, (v) major blood vessels that lead to or from the heart, (vi) trachea, (vii) esophagus, (viii) nerve that controls the larynx (voice box), (ix) sternum (chest bone) or backbone, (x) carina (where the trachea joins the bronchi). In the second section of Stage IIIB, cancer has spread to lymph nodes on the same side of the chest as the tumor; the lymph nodes with cancer are near the sternum (chest bone) or where the bronchus enters the lung; the tumor may be any size; there may be separate tumors in different lobes of the same lung; and cancer has spread to any of the following: (i) heart, (ii) major blood vessels that lead to or from the heart, (iii) trachea, (iv) esophagus, (v) nerve that controls the larynx (voice box), (vi) sternum (chest bone) or backbone, (vii) carina (where the trachea joins the bronchi).

In *Stage IV,* the tumor may be any size and cancer may have spread to lymph nodes. One or more of the following is true: there are one or more tumors in both lungs; cancer is found in fluid around the lungs or the heart; cancer has spread to other parts of the body, such as the brain, liver, adrenal glands, kidneys, or bone.

Accordingly, in various embodiments of the foregoing invention, the lung cancer may be stratified into any of the preceding stages *(e.g.,* occult, stage 0, stage IA, stage IB, stage IIA, stage IIB, stage IIIA, stage IIIB or stage IV) based on assessing of the levels of FRα not bound to a cell, such as a normal or cancerous cell, in a sample (for example, urine or serum) of a subject.

As used herein, the term "folate receptor alpha" (also referred to as FRα, FR-alpha, FOLR-1 or FOLR1) refers to the alpha isoform of the high affinity receptor for folate. Membrane bound FRα is attached to the cell surface by a glycosyl phosphatidylinositol (GPI) anchor), recycles between extracellular and endocytic compartments and is capable of transporting folate into the cell. FRα is expressed in a variety of epithelial tissues including those of the female reproductive tract, placenta, breast, kidney proximal tubules, choroid plexus, lung and salivary glands. Soluble forms of FRα may be derived by the action of proteases or phospholipase on membrane anchored folate receptors.

The consensus nucleotide and amino acid sequences for human FRα are set forth herein as SEQ ID NOs: 24 and 25, respectively. Variants, for example, naturally occurring allelic variants or sequences containing at least one amino acid substitution, are encompassed by the terms as used herein.

As used herein, the term "not bound to a cell" refers to FRα that is not attached to the cellular membrane of a cell, such as a cancerous cell. In a particular embodiment, the FRα not bound to a cell is unbound to any cell and is freely floating or solubilized in biological fluids, *e.g.,* urine or serum. For example, the FRα may be shed, secreted or exported from normal or cancerous cells, for example, from the surface of cancerous cells, into biological fluids.

The "level" of folate receptor alpha not bound to a cell, as used herein, refers to the level of folate receptor alpha protein as determined using any method known in the art for the measurement of protein levels. Such methods include, for example, electrophoresis, capillary electrophoresis, high performance liquid chromatography (HPLC), thin layer chromatography (TLC), hyperdiffusion chromatography, fluid or gel precipitin reactions, absorption spectroscopy, a colorimetric assays, spectrophotometric assays, flow cytometry, immunodiffusion (single or double), solution phase assay, immunoelectrophoresis, Western blotting, radioimmunoassay (RIA), enzyme-linked immunosorbent assays (ELISAs), immunofluorescent assays, and electrochemiluminescence immunoassay (exemplified below), and the like. In a preferred embodiment, the level is determined using antibody-based techniques, as described in more detail herein.

It is generally preferable to immobilize either an antibody or binding protein specific for FRα not bound to a cell on a solid support for Western blots and immunofluorescence techniques. Suitable solid phase supports or carriers include any support capable of binding an antigen or an antibody. Well-known supports or carriers include glass, polystyrene, polypropylene, polyethylene, dextran, nylon, amylases, natural and modified celluloses, polyacrylamides, gabbros, and magnetite.

One skilled in the art will know many other suitable carriers for binding antibody or antigen, and will be able to adapt such support for use with the present invention. For example, protein isolated from a subject sample (*e.g.*, urine or serum) can be run on a polyacrylamide gel electrophoresis and immobilized onto a solid phase support such as nitrocellulose. The support can then be washed with suitable buffers followed by treatment with the labeled antibody. The solid phase support can then be washed with the buffer a second time to remove unbound antibody. The amount of bound label on the solid support can then be detected by conventional means. Means of detecting proteins using electrophoretic techniques are well known to those of skill in the art (see generally, R. Scopes (1982) Protein Purification, Springer-Verlag, N.Y.; Deutscher, (1990) Methods in Enzymology Vol. 182: Guide to Protein Purification, Academic Press, Inc., N.Y.).

Other standard methods include immunoassay techniques which are well known to one of ordinary skill in the art and may be found in Principles And Practice Of Immunoassay, 2nd Edition, Price and Newman, eds., MacMillan (1997) and Antibodies, A Laboratory Manual, Harlow and Lane, eds., Cold Spring Harbor Laboratory, Ch. 9 (1988).

Antibodies used in immunoassays to determine the level of expression of folate receptor alpha may be labeled with a detectable label. The term "labeled", with regard to the binding agent or antibody, is intended to encompass direct labeling of the binding agent or antibody by coupling (*i.e.*, physically linking) a detectable substance to the binding agent or antibody, as well as indirect labeling of the binding agent or antibody by reactivity with another reagent that is directly labeled. An example of indirect labeling includes detection of a primary antibody using a fluorescently labeled secondary antibody. In one embodiment, the antibody is labeled, *e.g.*, radio-labeled, chromophore-labeled, fluorophore-labeled, or enzyme-labeled. In another embodiment, the antibody is an antibody derivative (*e.g.*, an antibody conjugated with a substrate or with the protein or ligand of a protein-ligand pair (*e.g.*, biotin-streptavidin), or an antibody fragment (*e.g.*, a single-chain antibody, an isolated antibody hypervariable domain) which binds specifically with FRα not bound to a cell.

In one embodiment of the invention, proteomic methods, *e.g.*, mass spectrometry, are used. Mass spectrometry is an analytical technique that consists of ionizing chemical compounds to generate charged molecules (or fragments thereof) and measuring their mass-to-charge ratios. In a typical mass spectrometry procedure, a sample is obtained from a subject, loaded onto the mass spectrometry, and its components (*e.g.*, FRα) are ionized by different methods (*e.g.*, by impacting them with an electron beam), resulting in the formation of charged particles (ions). The mass-to-charge ratio of the particles is then calculated from the motion of the ions as they transit through electromagnetic fields.

For example, matrix-associated laser desorption/ionization time-of-flight mass spectrometry (MALDI-TOF MS) or surface-enhanced laser desorption/ionization time-of-flight mass spectrometry (SELDI-TOF MS) which involves the application of a sample, such as urine or serum, to a protein-binding chip (Wright, G.L., Jr., et al. (2002) Expert Rev Mol Diagn 2:549; Li, J., et al. (2002) Clin Chem 48:1296; Laronga, C., et al. (2003) Dis Markers 19:229; Petricoin, E.F., *et al.* (2002) 359:572; Adam, B.L., et al. (2002) Cancer Res 62:3609; Tolson, J., et al. (2004) Lab Invest 84:845; Xiao, Z., et al. (2001) Cancer Res 61:6029) can be used to determine the level of FRα.

Furthermore, *in vivo* techniques for determination of the level of FRα not bound to a cell include introducing into a subject a labeled antibody directed against FRα, which binds to and transforms FRα into a detectable molecule. The presence, level, or location of the detectable FRα not bound to a cell in a subject may be determined using standard imaging techniques.

The term "sample" as used herein refers to a collection of similar fluids, cells, or tissues isolated from a subject, as well as fluids, cells, or tissues present within a subject. In preferred embodiments the sample is a biological fluid containing FRα not bound to a cancerous cell. Biological fluids are typically liquids at physiological temperatures and may include naturally occurring fluids present in, withdrawn from, expressed or otherwise extracted from a subject or biological source. Certain biological fluids derive from particular tissues, organs or localized regions and certain other biological fluids may be more globally or systemically situated in a subject or biological source. Examples of biological fluids include blood, serum and serosal fluids, plasma, lymph, urine, cerebrospinal fluid, saliva, ocular fluids, cystic fluid, tear drops, feces, sputum, mucosal secretions of the secretory tissues and organs, vaginal secretions, gynecological fluids, ascites fluids such as those associated with non-solid tumors, fluids of the pleural, pericardial, peritoneal, abdominal and other body cavities, fluids collected by bronchial lavage and the like. In a particular embodiment, the sample is urine or serum. In another embodiment, the sample does not include ascites or is not an ascite sample. In another embodiment, the sample does not include peritoneal fluid or is not peritoneal fluid.

In one embodiment, the sample is removed from the subject. In another embodiment, the sample is present within the subject. Biological fluids may also include liquid solutions contacted with a subject or biological source, for example, cell and organ culture medium including cell or organ conditioned medium, lavage fluids and the like.

In some embodiments, only a portion of the sample is subjected to an assay for determining the level of FRα not bound to a cell, or various portions of the sample are subjected to various assays for determining the level of FRα not bound to a cell. Also, in many embodiments, the sample may be pre-treated by physical or chemical means prior to the assay. For example, in embodiments discussed in more detail in the Examples section, samples, for example, urine samples, were subjected to centrifugation, dilution and/or treatment with a solubilizing substance (*e.g.*, guanidine treatment) prior to assaying the samples for FRα not bound to a cell. Such techniques serve to enhance the accuracy, reliability and reproducibility of the assays of the present invention.

The term "control sample," as used herein, refers to any clinically relevant control sample, including, for example, a sample from a healthy subject not afflicted with ovarian cancer, a sample from a subject having a less severe or slower progressing ovarian cancer than the subject to be assessed, a sample from a subject having some other type of cancer or disease, and the like. A control sample may include a sample derived from one or more subjects. A control sample may also be a sample made at an earlier timepoint from the subject to be assessed. For example, the control sample could be a sample taken from the subject to be assessed before the onset of the FRα expressing cancer such as lung or ovarian cancer, at an earlier stage of disease, or before the administration of treatment or of a portion of treatment. The control sample may also be a sample from an animal model, or from a tissue or cell lines derived from the animal model, of the FRα expressing cancer such as lung or ovarian cancer. The level of FRα not bound to a cell in a control sample that consists of a group of measurements may be determined based on any appropriate statistical measure, such as, for example, measures of central tendency including average, median, or modal values.

The term "control level" refers to an accepted or pre-determined level of FRα which is used to compare with the level of FRα in a sample derived from a subject. In one embodiment, the control level of FRα is based on the level of FRα not bound to a cell in sample(s) from a subject(s) having slow disease progression. In another embodiment, the control level of FRα not bound to a cell is based on the level in a sample from a subject(s) having rapid disease progression. In another embodiment, the control level of FRα is based on the level of FRα not bound to a cell in a sample(s) from an unaffected, *i.e.*, non-diseased, subject(s), *i.e.*, a subject who does not have an FRα expressing cancer such as lung or ovarian cancer. In yet another embodiment, the control level of FRα is based on the level of FRα not bound to a cell in a sample from a subject(s) prior to the administration of a therapy for ovarian cancer. In another embodiment, the control level of FRα is based on the level of FRα not bound to a cell in a sample(s) from a subject(s) having an FRα expressing cancer such as lung or ovarian cancer that is not contacted with a test compound. In another embodiment, the control level of FRα is based on the level of FRα not bound to a cell in a sample(s) from a subject(s) not having an FRα expressing cancer such as lung or ovarian cancer that is contacted with a test compound. In one embodiment, the control level of FRα is based on the level of FRα not bound to a cell in a sample(s) from an animal model of an FRα expressing cancer such as lung or ovarian cancer, a cell, or a cell line derived from the animal model of an FRα expressing cancer such as lung or ovarian cancer.

In one embodiment, the control is a standardized control, such as, for example, a control which is predetermined using an average of the levels of FRα not bound to a cell from a population of subjects having no FRα expressing cancer such as lung or ovarian cancer. In still other embodiments of the invention, a control level of FRα is based on the level of FRα not bound to a cell in a non-cancerous sample(s) derived from the subject having an FRα expressing cancer such as lung or ovarian cancer. For example, when a laparotomy or other medical procedure reveals the presence of ovarian cancer in one portion of the ovaries, the control level of FRα may be determined using the non-affected portion of the ovaries, and this control level may be compared with the level of FRα in an affected portion of the ovaries. Similarly, when a biopsy or other medical procedure reveals the presence of a lung cancer in one portion of the lungs, the control level of FRα may be determined using the non-affected portion of the lungs, and this control level may be compared with the level of FRα in an affected portion of the lungs.

As used herein, "a difference" between the level of folate receptor alpha not bound to a cell in a sample from a subject (*i.e.*, a test sample) and the level of folate receptor alpha not bound to a cell in a control sample refers broadly to any clinically relevant and/or statistically significant difference in the level of folate receptor alpha in the two samples. In an exemplary embodiment, the difference is selected based on a cutoff value determined using a receiver operating characteristic (ROC) analysis, an example of which is presented in Example 6.

In other embodiments, the difference must be greater than the limits of detection of the method for determining the level of FRα not bound to a cell. It is preferred that the difference be at least greater than the standard error of the assessment method, and preferably a difference of at least about 2-, about 3-, about 4-, about 5-, about 6-, about 7-, about 8-, about 9-, about 10-, about 15-, about 20-, about 25-, about 100-, about 500-, about 1000-fold or greater than the standard error of the assessment method. The difference may be assessed by any appropriate comparison, including any appropriate parametric or nonparametric descriptive statistic or comparison. For example, "an increase" in the level of FRα not bound to a cell may refer to a level in a test sample that is about two, and more preferably about three, about four, about five, about six, about seven, about eight, about nine, about ten or more times more than the level of FRα in the control sample. An increase may also refer to a level in a test sample that is preferably at least about 1.5, and more preferably about two, about three, about four, about five or more standard deviations above the average level of FRα in the control sample. Likewise, "a decrease" in the level of FRα not bound to a cell may refer to a level in a test sample that is preferably at least about two, and more preferably about three, about four, about five, about six, about seven, about eight, about nine, about ten or more times less than the level of FRα in the control sample. A decrease may also refer to a level in a test sample that is preferably at least about 1.5, and more preferably about two, about three, about four, about five or more standard deviations below the average level of FRα in the control sample.

As used herein, the term "contacting the sample" with an FRα binding agent, *e.g.,* an anti-FRα antibody, includes exposing the sample, or any portion thereof with the agent or antibody, such that at least a portion of the sample comes into contact with the agent or antibody. The sample or portion thereof may be altered in some way, such as by subjecting it to physical or chemical treatments (*e.g.*, dilution or guanidine treatment), prior to the act of contacting it with the agent or antibody.

The term "antibody" as used herein, comprises four polypeptide chains, two heavy (H) chains and two light (L) chains, interconnected by disulfide bonds, as well as any functional (*i.e.*, antigen-binding) fragment, mutant, variant, or derivation thereof, which retains the essential epitope binding features of an Ig molecule. Such mutant, variant, or derivative antibody formats are known in the art, and include molecules such as Fab fragments, Fab' fragments, $F(ab')_2$ fragments, Fd fragments, Fabc fragments, Sc antibodies (single chain antibodies), diabodies, individual antibody light chains, individual antibody heavy chains, chimeric fusions between antibody chains and the like. Immunoglobulin molecules can be of any type (*e.g.*, IgG, IgE, IgM, IgD, IgA and IgY), class (*e.g.*, IgG1, IgG2, IgG3, IgG4, IgA1 and IgA2) or subclass.

Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Light chains are classified as either kappa or lambda. Heavy chains are classified as gamma, mu, alpha, delta, or epsilon, and define the antibody's isotype as IgG, IgM, IgA, IgD and IgE, respectively.

The variable regions of the heavy and light chains contain a binding domain that interacts with an antigen. The constant regions of the antibodies may mediate the binding of the immunoglobulin to host tissues or factors, including various cells of the immune system (*e.g*., effector cells) and the first component (Clq) of the classical complement system.

The term "antigen-binding portion" of an antibody, as used herein, refers to one or more fragments of an antibody that retain the ability to specifically bind to an antigen *(e.g.,* FRα not bound to a cell). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the $V_L$, $V_H$, CL and CH1 domains; (ii) a $F(ab')_2$ fragment, a bivalent fragment comprising two Fab fragments linked by a disulfide bridge at the hinge region; (iii) a Fd fragment consisting of the $V_H$ and CH1 domains; (iv) a Fv fragment consisting of the $V_L$ and $V_H$ domains of a single arm of an antibody, (v) a dAb including VH and VL domains; (vi) a dAb fragment (Ward et al. (1989) Nature 341, 544-546), which consists of a $V_H$ domain; (vii) a dAb which consists of a VH or a VL domain; and (viii) an isolated complementarity determining region (CDR) or (ix) a combination of two or more isolated CDRs which may optionally be joined by a synthetic linker. Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules (known as single chain Fv (scFv); see *e.g.,* Bird et al. (1988) Science 242, 423-426; and Huston et al. (1988) Proc. Natl. Acad. Sci. USA 85, 5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

The term "antibody", as used herein, includes polyclonal antibodies, monoclonal antibodies, murine antibodies, chimeric antibodies, humanized antibodies, and human antibodies, and those that occur naturally or are recombinantly produced according to methods well known in the art.

In one embodiment, an antibody for use in the methods of the invention is a bispecific antibody. A "bispecific antibody" is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, (1990) Clin. Exp. Immunol. 79, 315-321; Kostelny et al. (1992) J. Immunol. 148, 1547-1553.

In another embodiment, an antibody for use in the methods of the invention is a camelid antibody as described in, for example, PCT Publication WO 94/04678.

A region of the camelid antibody that is the small, single variable domain identified as $V_{HH}$ can be obtained by genetic engineering to yield a small protein having high affinity for a target, resulting in a low molecular weight, antibody-derived protein known as a "camelid nanobody". See U.S. Pat. No. 5,759,808; see also Stijlemans et al., 2004 J. Biol. Chem. 279: 1256-1261; Dumoulin et al., 2003 Nature 424: 783-788; Pleschberger et al., 2003 Bioconjugate Chem. 14: 440-448; Cortez-Retamozo et al., 2002 Int. J. Cancer 89: 456-62; and Lauwereys et al., 1998 EMBO J. 17: 3512-3520. Engineered libraries of camelid antibodies and antibody fragments are commercially available, for example, from Ablynx, Ghent, Belgium. Accordingly, a feature of the present invention is a camelid nanobody having high affinity for FRα.

In other embodiments of the invention, an antibody for use in the methods of the invention is a diabody, a single chain diabody, or a di-diabody.

Diabodies are bivalent, bispecific molecules in which $V_H$ and $V_L$ domains are expressed on a single polypeptide chain, connected by a linker that is too short to allow for pairing between the two domains on the same chain. The $V_H$ and $V_L$ domains pair with complementary domains of another chain, thereby creating two antigen binding sites (see *e.g.,* Holliger et al., 1993 Proc. Natl. Acad. Sci. USA 90:6444-6448; Poljak et al., 1994 Structure 2:1121-1123). Diabodies can be produced by expressing two polypeptide chains with either the structure $V_{HA}$-$V_{LB}$ and $VH_{B}$-$V_{LA}$ ($V_H$-$V_L$ configuration), or $V_{LA}$-$V_{HB}$ and $V_{LB}$-$V_{HA}$ ($V_L$-$V_H$ configuration) within the same cell. Most of them can be expressed in soluble form in bacteria.

Single chain diabodies (scDb) are produced by connecting the two diabody-forming polypeptide chains with linker of approximately 15 amino acid residues (see Holliger and Winter, 1997 Cancer Immunol. Immunother., 45(3-4):128-30; Wu et al., 1996 Immunotechnology, 2(1):21-36). scDb can be expressed in bacteria in soluble, active monomeric form (see Holliger and Winter, 1997 Cancer Immunol. Immunother., 45(34): 128-30; Wu et al., 1996 Immunotechnology, 2(1):21-36; Pluckthun and Pack, 1997 Immunotechnology, 3(2): 83-105; Ridgway et al., 1996 Protein Eng., 9(7):617-21).

A diabody can be fused to Fc to generate a "di-diabody" (see Lu et al., 2004 J. Biol. Chem., 279(4):2856-65).

FRα binding molecules that exhibit functional properties of antibodies but derive their framework and antigen binding portions from other polypeptides (*e.g.*, polypeptides other than those encoded by antibody genes or generated by the recombination of antibody genes *in vivo*) may also be used in the methods of the present invention. The antigen binding domains (*e.g.*, FRα binding domains) of these binding molecules are generated through a directed evolution process. See U.S. Pat. No. 7,115,396. Molecules that have an overall fold similar to that of a variable domain of an antibody (an "immunoglobulin-like" fold) are appropriate scaffold proteins. Scaffold proteins suitable for deriving antigen binding molecules include fibronectin or a fibronectin dimer, tenascin, N-cadherin, E-cadherin, ICAM, titin, GCSF-receptor, cytokine receptor, glycosidase inhibitor, antibiotic chromoprotein, myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, NCAM, twitchin, neuroglian, growth hormone receptor, erythropoietin receptor, prolactin receptor, interferon-gamma receptor, β-galactosidase/glucuronidase, β-glucuronidase, transglutaminase, T-cell antigen receptor, superoxide dismutase, tissue factor domain, cytochrome F, green fluorescent protein, GroEL, and thaumatin.

"Specific binding" when used in the context of antibodies, or antibody fragments, represents binding via domains encoded by immunoglobulin genes or fragments of immunoglobulin genes to one or more epitopes of a protein of interest, but which do not substantially recognize and bind other molecules in a sample containing a mixed population of antigenic molecules. Typically, an antibody binds to a cognate antigen with a Kd of less than about $1x10^{-8}$ M, as measured by a surface plasmon resonance assay or a cell binding assay.

As used herein, a folate receptor alpha "binding agent" includes an antibody that binds FRα not bound to a cell as well as non-antibody binding agents. To generate non-antibody binding agents or binding molecules, a library of clones can be created in which sequences in regions of the scaffold protein that form antigen binding surfaces (*e.g.*, regions analogous in position and structure to CDRs of an antibody variable domain immunoglobulin fold) are randomized. Library clones are tested for specific binding to the antigen of interest (*e.g.,* FRα) and for other functions (*e.g.*, inhibition of biological activity of FRα). Selected clones can be used as the basis for further randomization and selection to produce derivatives of higher affinity for the antigen.

High affinity binding molecules are generated, for example, using the tenth module of fibronectin III ($^{10}$Fn3) as the scaffold, described in U.S. Pat. Nos. 6,818,418 and 7,115,396; Roberts and Szostak, 1997 Proc. Natl. Acad. Sci USA 94:12297; U.S. Pat. No. 6,261,804; U.S. Pat. No. 6,258,558; and Szostak et al. (WO98/31700).

Non-antibody binding molecules can be produced as dimers or multimers to increase avidity for the target antigen. For example, the antigen binding domain is expressed as a fusion with a constant region (Fc) of an antibody that forms Fc-Fc dimers. See, *e.g.,* U.S. Pat. No. 7,115,396.

An "antigen" is a molecule recognized by the immune system; the term originally came from "antibody generator" and includes a molecule that binds specifically to an antibody. At the molecular level, an antigen is characterized by its ability to be bound at the antigen-binding site of an antibody. In the present invention, the antigen is FRα, such as FRα which is not bound to a cell FRα or a portion thereof.

As used herein, the term "epitope" refers to the molecular surface features of an antigen, *e.g.,* FRα, capable of being bound by an antibody. Antigenic molecules, normally being "large" biological polymers, usually present several surface features that can act as points of interaction for specific antibodies. Any such distinct molecular feature constitutes an epitope. Most antigens therefore have the potential to be bound by several distinct antibodies, each of which is typically specific to a particular epitope. In one embodiment of the present invention, a binding agent, *e.g.,* antibody, binds to an epitope on FRα which is available in the form of the receptor which is not bound to a cell but not in the membrane bound form of the receptor. For example, the antibody may bind to the same epitope on FRα to which MORAB-003 binds.

As used herein, the phrase "progression of an FRα-expressing cancer in a subject afflicted with an FRα-expressing cancer" includes the progression of such a cancer from a less severe to a more severe state. This could include an increase in the number or severity of tumors, the degree of metastasis, the speed with which the cancer is growing and spreading, and the like. For example, "the progression of ovarian cancer" includes the progression of such a cancer from a less severe to a more severe state, such as the progression from Stage I to Stage II, from Stage II to Stage III, etc. Alternatively, the phrase "progression of an FRα-expressing cancer in a subject afflicted with an FRα-expressing cancer" may refer to the regression of an FRα-expressing cancer from a more severe state to a less severe state. For example, in one embodiment, "the progression of ovarian cancer" refers to the regression from Stage IV to Stage III, from Stage III to Stage II, etc. In other embodiments, the "progression of an FRα-expressing cancer in a subject afflicted with an FRα-expressing cancer" may refer to the survival rate determined from the beginning of symptoms of the FRα-expressing cancer, or to the survival rate from the time of diagnosis of the FRα-expressing cancer.

As used herein, the term "stratifying" refers to characterizing an FRα expressing cancer, for example, ovarian or lung cancer, into an appropriate stage based, for example, on the degree of the spread of the cancer, as well accepted stratifications in the art. For example, stratifying includes characterizing the FRα expressing cancer into Stage I, Stage II, Stage III or Stage IV. In certain embodiments, Stage I refers to cancers that are localized to one part of the body. In certain embodiments, Stages II and III refer to cancers that are locally advanced, wherein a distinction between the stages are often specific to the particular cancer. Finally, Stage IV refers to cancers that have often metastasized, or spread to other organs or throughout the body.

As used herein, the term "survival" refers to the continuation of life of a subject which has been treated for cancer. In one embodiment, survival refers to the failure of a tumor to recur. As used herein, the term "recur" refers to the re-growth of tumor or cancerous cells in a subject in whom primary treatment for the tumor has been administered. The tumor may recur in the original site or in another part of the body. In one embodiment, a tumor that recurs is of the same type as the original tumor for which the subject was treated. For example, if a subject had an ovarian cancer tumor, was treated and subsequently developed another ovarian cancer tumor, the tumor has recurred. In addition, a cancer can recur in a different organ or tissue than the one where it originally occurred.

## II. Methods and Kits of the Invention

The present invention is based, at least in part, on the unexpected discovery that folate receptor alpha (FRα), not bound to a cell, is found at elevated levels in the body fluids, for example, urine or serum, of a subject having an FRα-expressing cancer as compared to a control sample. Moreover, the present invention is based, at least in part, on the identification of an immunological assay that exhibits the necessary sensitivity for assessing levels of FRα not bound to a cells in samples, where prior attempts to do so had repeatedly failed. Indeed, the present invention overcomes the challenges observed during prior attempts to develop an FRα-based diagnostic assay for FRα-expressing cancer such as lung or ovarian cancer by providing an immunological assay capable of accurately assessing levels of FRα not bound to a cell in a sample, *e.g.,* urine or serum.

Accordingly, methods and kits for assessing whether a subject has or is at risk for developing an FRα-expressing cancer and, further, for assessing the progression of an FRα-expressing cancer are provided. In various embodiments, the methods involve the comparison of levels of FRα not bound to a cell in samples, for example, urine and serum, as compared to control levels, in assessing the presence, degree or risk of development of ovarian cancer in the subject.

**A. Diagnostic Methods, Prognostic Methods, Risk Assessment Methods, and Stratification Methods**

Specifically, the present invention provides diagnostic methods for assessing whether a subject is afflicted with an FRα-expressing cancer, such as lung or ovarian cancer, prognostic methods for predicting the progression of an FRα-expressing cancer such as lung or ovarian cancer, and risk assessment methods for assessing the level of risk that a subject will develop the FRα-expressing cancer. Furthermore, the invention provides stratification methods for stratifying an FRα-expressing cancer such as lung or ovarian cancer subject into cancer therapy groups. The various aspects and embodiments of the invention discussed here are intended to be non-limiting and to encompass all possible combinations of the specific embodiments mentioned, which may apply to any of the methods and kits discussed herein or claimed below.

The methods of the present invention can be practiced in conjunction with any other method used by the skilled practitioner to diagnose an FRα-expressing cancer, predict the progression of an FRα-expressing cancer, or to assess the level of risk that a subject will develop an FRα-expressing cancer.

In one aspect, the invention provides a method of assessing whether a subject is afflicted with an FRα-expressing cancer, by determining the level of folate receptor alpha (FRα) which is not bound to a cell, in a sample (such as urine or serum) derived from the subject; and comparing the level of folate receptor alpha (FRα) which is not bound to a cell with the level of FRα in a control sample, wherein a difference between the level of FRα in the sample derived from the subject and the level of FRα in the control sample is an indication that the subject is afflicted with an FRα-expressing cancer. In a particular embodiment, the level of FRα in the sample derived from the subject is assessed by contacting the sample with an antibody that binds FRα not bound to a cell and is selected from the group consisting of (a) an antibody that binds the same epitope as the MORAb-003 antibody; and (b) an antibody comprising SEQ ID NO: 1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. In one embodiment, the sample is selected from the group consisting of urine and serum.

In another aspect, the present invention provides a method of assessing whether a subject is afflicted with an FRα-expressing cancer such as lung cancer or ovarian cancer, the method comprising determining the level of folate receptor alpha (FRα) not bound to a cell in a urine sample derived from the subject; and comparing the level of folate receptor alpha (FRα) in the urine sample derived from the subject with the level of FRα in a control sample, wherein a difference between the level of FRα in the urine sample derived from the subject and the level of FRα in the control sample is an indication that the subject is afflicted with an FRα-expressing cancer.

In another aspect, the invention provides a method of assessing whether a subject is afflicted with an FRα-expressing cancer, by determining the level of folate receptor alpha (FRα) not bound to a cell in a serum sample derived from the subject; and comparing the level of folate receptor alpha (FRα) in the serum sample derived from the subject with the level of FRα in a control sample, wherein a difference between the level of FRα in the serum sample derived from the subject and the level of FRα in the control sample is an indication that the subject is afflicted with an FRα-expressing cancer. In particular embodiments, the subject has not been treated with an agent, such as a steroid, that enhances the levels of FRα in serum. In a specific embodiment, the FRα-expressing cancer is ovarian cancer and the subject has not been treated with an agent, such as a steroid, that enhances the levels of FRα in serum.

In the methods and kits of the present invention, FRα-expressing cancers include cancers characterized in that the cancer cells express FRα. In particular embodiments, the FRα is released from the cancer cells, for example, from the surface of the cancer cell, and into the biological fluids of the subject. FRα-expressing cancers include lung cancer (*e.g.,* bronchioalveolar carcinomas, carcinoid tumors, and non-small cell lung cancers, such as adenocarcinomas); mesothelioma; ovarian cancer; renal cancer; brain cancer (*e.g.,* anaplastic ependymoma and cerebellar juvenile pilocytic astrocytoma); cervical cancer; nasopharyngeal cancer; mesodermally derived tumor; squamous cell carcinoma of the head and neck; endometrial cancer; endometrioid adenocarcinomas of the ovary, serous cystadenocarcinomas, breast cancer; bladder cancer; pancreatic cancer; bone cancer (*e.g.,* high-grade osteosarcoma); and pituitary cancer (*e.g.,* pituitary adenoma). In a particular embodiment, the FRα-expressing cancer is ovarian cancer.

In certain embodiments of the methods and kits of the present invention, the FRα-expressing cancer is lung cancer. In more specific embodiments, the lung cancer is non-small cell lung carcinoma (NSCLC). In one such embodiment, the NSCLC is selected from the group consisting of adenocarcinoma, squamous cell lung carcinoma, large cell lung carcinoma, pleomorphic NSCLC, carcinoid tumor, salivary gland carcinoma, and unclassified carcinoma. In a preferred embodiment, the NSCLC is adenocarcinoma. In alternative embodiments, the lung cancer is small cell lung carcinoma (SCLC). In another embodiment, the lung cancer is bronchioalveolar carcinoma. In yet another embodiment, the lung cancer is a lung carcinoid tumor.

The present invention also provides methods to assess whether a subject is afflicted with ovarian cancer by determining the level of folate receptor alpha (FRα) not bound to a cell in a urine sample derived from the subject, wherein the presence of FRα in the urine sample at a concentration of greater than about 3000 a.u./ml is an indication that the subject is afflicted with ovarian cancer. In particular embodiments, the presence of FRα in the urine sample at a concentration of greater than about 4000 a.u./ml, about 5000 a.u./ml, about 6000 a.u./ml, about 7000 a.u./ml, about 8000 a.u./ml, about 9000 a.u./ml, about 10,000 a.u./ml, about 11,000 a.u./ml, about 12,000 a.u./ml, about 13,000 a.u./ml, about 14,000 a.u./ml, about 15,000 a.u./ml, about 16,000 a.u./ml, about 17,000 a.u./ml, about 18,000 a.u./ml, about 19,000 a.u./ml, about 20,000 a.u./ml, about 21,000 a.u./ml, about 22,000 a.u./ml, about 23,000 a.u./ml, about 24,000 a.u./ml, about 25,000 a.u./ml, about 26,000 a.u./ml, about 27,000 a.u./ml, about 28,000 a.u./ml, about 29,000 a.u./ml or about 30,000 a.u./ml is an indication that the subject is afflicted with ovarian cancer.

In yet another aspect, the present invention provides a method of assessing whether a subject is afflicted with ovarian cancer, by determining the level of folate receptor alpha (FRα) in a urine sample derived from the subject, wherein the presence of FRα in the urine sample at a concentration of greater than about 9100 pg/ml is an indication that the subject is afflicted with ovarian cancer or wherein a concentration of less than about 9100 pg/ml is an indication that the subject is not afflicted with ovarian cancer. For example, the presence of FRα in the urine sample at a concentration of greater than about 9500 pg/mL, about 10,000 pg/mL, about 11,000 pg/mL, about 12,000 pg/mL, about 13,000 pg/mL, about 14,000 pg/mL, about 15,000 pg/mL, about 16,000 pg/mL, about 17,000 pg/mL, about 18,000 pg/mL, about 19,000 pg/mL, about 20,000 pg/mL, about 21,000 pg/mL, about 22,000 pg/mL, about 23,000 pg/mL, about 24,000 pg/mL, about 25,000 pg/mL, about 26,000 pg/mL, about 27,000 pg/mL, about 28,000 pg/mL, about 29,000 pg/mL, about 30,000 pg/mL, about 40,000 pg/mL, about 50,000 pg/mL, about 60,000 pg/mL, about 70,000 pg/mL, about 80,000 pg/ml, about 90,000 pg/ml, about 100,000 pg/ml or about 150,000 pg/ml is an indication that the subject is afflicted with ovarian cancer.

In certain embodiments of the foregoing aspects of the invention, the levels of FRα not bound to a cell in a sample (*e.g.,* a sample such as a urine sample or serum sample) derived from a subject are compared with the levels of FRα in a control sample, wherein a difference between the levels is an indication that the subject is afflicted with an FRα-expressing cancer such as lung or ovarian cancer. In a particular embodiment, the difference constitutes an increase in the level of FRα not bound to a cell in the sample derived from the subject as compared with the level of FRα in the control sample, wherein this increase is indicative of the presence or growth of FRα-expressing cancer. Alternatively, the difference constitutes a decrease in the level of FRα, wherein the decrease is indicative of the absence or regression of FRα-expressing cancer. As used herein, "a difference" between the level of folate receptor alpha not bound to a cell in a sample from a subject (*i.e.*, a test sample) and the level of folate receptor alpha in a control sample refers broadly to any clinically relevant change (including an increase or a decrease) and/or statistically significant difference in the level of folate receptor alpha in the two samples. In an exemplary embodiment, the difference is selected based on a cutoff value determined using a receiver operating characteristic (ROC) analysis, an example of which is presented in Example 6. The optimal cutoff value may vary depending on the assay methods and conditions employed. In other embodiments, the difference must be greater than the limits of detection of the method for determining the level of FRα not bound to a cell. It is preferred that the difference be at least greater than the standard error of the assessment method, and preferably a difference of at least about 2-, about 3-, about 4-, about 5-, about 6-, about 7-, about 8-, about 9-, about 10-, about 15-, about 20-, about 25-, about 100-, about 500-, about 1000-fold or greater than the standard error of the assessment method. The difference may be assessed by any appropriate comparison, including any appropriate parametric or nonparametric descriptive statistic or comparison. For example, "an increase" in the level of FRα may refer to a level that exceeds a cutoff value determined using an ROC analysis. It may also refer to a level in a test sample that is two, and more preferably about 5%, about 10%, about 15%, about 20%, about 25%, about 30%, about 40%, about 50%, about 60%, about 70%, about 80%, about 90%, about 100%, about 150%, about 200%, about 300%, about 400%, about 500%, about 600%, about 700%, about 800%, about 900% or about 1000% more than the level of FRα in the control sample. An increase may also refer to a level in a test sample that is preferably at least about 1.5, and more preferably about two, about three, about four, about five or more standard deviations above the average level of FRα in the control sample. Likewise, "a decrease" in the level of FRα not bound to a cell may refer to a level in a test sample that does not exceed a cutoff value determined using an ROC analysis. It may also refer to a level in a test sample that is about 5%, about 10%, about 15%, about 20%, about 25%, about 30%, about 40%, about 50%, about 60%, about 70%, about 80%, or about 90% less than the level of FRα in the control sample. A decrease may also refer to a level in a test sample that is preferably at least about 1.5, and more preferably about two, about three, about four, about five or more standard deviations below the average level of FRα in the control sample.

Samples useful in the methods and kits of the invention include any tissue, cell, biopsy, or bodily fluid that may contain detectable levels of FRα not bound to a cell. In one embodiment, a sample may be a tissue, a cell, whole blood, plasma, buccal scrape, saliva, cerebrospinal fluid, stool, or bronchoalveolar lavage. In some embodiments, the sample is FRα-expressing tumor sample or a sample of tissues or cells where FRα-expressing cancer may be found. In preferred embodiments, the sample is a urine or serum sample.

Body samples may be obtained from a subject by a variety of techniques known in the art including, for example, by the use of a biopsy or by scraping or swabbing an area or by using a needle to aspirate bodily fluids. Methods for collecting various body samples are well known in the art.

Samples suitable for detecting and quantitating the FRα protein level may be fresh, frozen, or fixed according to methods known to one of skill in the art. Suitable tissue samples are preferably sectioned and placed on a microscope slide for further analyses. Solid samples, *i.e.*, tissue samples, may be solubilized and/or homogenized and subsequently analyzed as soluble extracts. Liquid samples may also be subjected to physical or chemical treatments. In some embodiments, urine samples are treated by centrifugation, vortexing, dilution and/or treatment with a solubilizing substance (such as, for example, guanidine treatment).

In one embodiment, a freshly obtained biopsy sample is frozen using, for example, liquid nitrogen or difluorodichloromethane. The frozen sample is mounted for sectioning using, for example, OCT, and serially sectioned in a cryostat. The serial sections are collected on a glass microscope slide. For immunohistochemical staining the slides may be coated with, for example, chrome-alum, gelatine or poly-L-lysine to ensure that the sections stick to the slides. In another embodiment, samples are fixed and embedded prior to sectioning. For example, a tissue sample may be fixed in, for example, formalin, serially dehydrated and embedded in, for example, paraffin.

Once the sample is obtained, any method known in the art to be suitable for detecting and quantitating FRα not bound to a cell may be used (either at the nucleic acid or, preferably, at the protein level), as described in section (B) below. Exemplary methods are well known in the art and include but are not limited to western blots, northern blots, southern blots, immunohistochemistry, solution phase assay, ELISA, *e.g.,* amplified ELISA, immunoprecipitation, immunofluorescence, flow cytometry, immunocytochemistry, mass spectrometrometric analyses, *e.g.,* MALDI-TOF and SELDI-TOF, nucleic acid hybridization techniques, nucleic acid reverse transcription methods, and nucleic acid amplification methods.

In many embodiments, the level of FRα not bound to a cell in the sample (such as, for example, urine or serum) is assessed by contacting the sample with an antibody that binds FRα. Antibodies that bind FRα are known in the art and include (i) the murine monoclonal LK26 antibody (the heavy and light chains thereof are presented herein as SEQ ID NOs: 22 and 23), as described in European Patent Application No. 86104170.5 (Rettig) ; (ii) the MORAB-003 antibody, as described in International Publication No. WO2004/113388 and U.S. Patent No. 5,646,253.

The monoclonal antibodies MOV18 and MOv19 also bind different epitopes on the FRα molecule (previously known as gp38/FBP). Miotti, S. et al. Int J Cancer, 38: 297-303 (1987). For example, the MOV18 antibody binds the epitope set forth herein as SEQ ID NO:26 (TELLNVXMNAK*XKEKPXPX*KLXXQX) (note that at position 12, a tryptophan or histidine residue is possible, and at position 21, an aspartic acid or glutamic acid residue is possible), as taught in Coney et al. Cancer Res, 51: 6125-6132 (1991).

As used herein, the term "MORAb-003" refers to an antibody that specifically binds FRα and which comprises the mature heavy chain amino acid sequence as set forth in SEQ ID NO:7 and the mature light chain sequence of SEQ ID NO:8. The corresponding pre-protein amino acid sequences for MORAb-003 are set forth in SEQ ID NOs: 9 (heavy chain) and 10 (light chain). The MORAb-003 antibody comprises the following CDRs: SEQ ID NO:1 as CDRH1, SEQ ID NO:2 as CDRH2, SEQ ID NO:3 as CDRH3, SEQ ID NO:4 as CDRL1, SEQ ID NO:5 as CDRL2, and SEQ ID NO:6 as CDRL3. MORAb-003 antibody producing cells have been deposited with the American Type Culture Collection (10801 University Blvd., Manassas, Virginia 20110-2209) on April 24, 2006 and have been assigned Accession No. PTA-7552.

Other antibodies that bind FRα and for use in the methods of the present invention include 9F3.H9.H3.H3.B5.G2 (also referred to as 9F3),19D4.B7 (also referred to as 19D4), 24F12.B1 (also referred to as 24F12), and 26B3.F2 (also referred to as 26B3). The amino acid sequences of these antibodies, their CDRs, and their heavy and light chain variable domains, as well as polynucleotide sequences that may encode them, are provided in Table 33. In some embodiments, these antibodies are murine IgG, or derivatives thereof. In other embodiments, the antibodies are human, humanized, or chimeric.

<u>9F3</u>

In some embodiments, the antibody that binds FRα is an antibody or antigen-binding fragment that includes a light chain CDR1 amino acid sequence substantially the same as, or identical to, SEQ ID NO:27. In some embodiments, the antibody that binds FRα includes a light chain CDR2 amino acid sequence substantially the same as, or identical to, SEQ ID NO:28. In some embodiments, the antibody that binds FRα includes a light chain CDR3 amino acid sequence substantially the same as, or identical to, SEQ ID NO 29. In some embodiments, the antibody that binds FRα includes a heavy chain CDR1 amino acid sequence substantially the same as, or identical to, SEQ ID NO 31. In some embodiments, the antibody that binds FRα includes a heavy chain CDR2 amino acid sequence substantially the same as, or identical to, SEQ ID NO:32. In some embodiments, the antibody that binds FRα includes a heavy chain CDR3 amino acid sequence substantially the same as, or identical to, SEQ ID NO:33. The antibody that binds FRα may include a light chain having a CDR1 amino acid sequence substantially the same as, or identical to, SEQ ID NO:27; a CDR2 amino acid sequence substantially the same as, or identical to, SEQ ID NO:28; and a CDR3 amino acid sequence substantially the same as, or identical to, SEQ ID NO:29. The antibody that binds FRα may include a heavy chain having a CDR1 amino acid sequence substantially the same as, or identical to, SEQ ID NO:31; a CDR2 amino acid sequence substantially the same as, or identical to, SEQ ID NO: 32; and a CDR3 amino acid sequence substantially the same as, or identical to, SEQ ID NO:33. The antibody that binds FRα may include a light chain having a CDR1 amino acid sequence substantially the same as, or identical to, SEQ ID NO:27; a CDR2 amino acid sequence substantially the same as, or identical to, SEQ ID NO:28; and a CDR3 amino acid sequence substantially the same as, or identical to, SEQ ID NO:29, and also have a heavy chain having a CDR1 amino acid sequence substantially the same as, or identical to, SEQ ID NO:31; a CDR2 amino acid sequence substantially the same as, or identical to, SEQ ID NO:32; and a CDR3 amino acid sequence substantially the same as, or identical to, SEQ ID NO:33.

The antibody that binds FRα may include a light chain variable domain that includes an amino acid sequence substantially the same as, or identical to, SEQ ID NO:30. The antibody that binds FRα may include a heavy chain variable domain that includes an amino acid sequence substantially the same as, or identical to, SEQ ID NO:34. The antibody that binds FRα may include a light and a heavy chain variable domains, wherein the light chain variable domain includes an amino acid sequence substantially the same as, or identical to, SEQ ID NO:30, and the heavy chain variable domain includes an amino acid sequence substantially the same as, or identical to, SEQ ID NO:34. In some embodiments the antibody that binds FRα is the 9F3.H9.H3.H3.B5.G2 (9F3) antibody or an antigen-binding fragment thereof, capable of binding either a native or nonreduced form of FRα. In some embodiments, the antibody has a murine IgG2a constant region.

In some embodiments, the antibody that binds FRα is an antibody that is produced by antibody-producing cells deposited with the American Type Culture Collection (10801 University Blvd., Manassas, Virginia 20110-2209) on May 19, 2011 and have been assigned Accession No. PTA-11887. In some embodiments, the antibody that binds FRα comprises one or more of the light and heavy chain CDRs of the antibodies produced by the deposited antibody-producing cells. In some embodiments, antibody that binds FRα comprises the light and heavy chain variable regions of the antibodies produced by the deposited antibody-producing cells.

<u>19D4</u>

In some embodiments, the antibody that binds FRα is an antibody or antigen-binding fragment that includes a light chain CDR1 amino acid sequence substantially the same as, or identical to, SEQ ID NO:35. In some embodiments, the antibody that binds FRα includes a light chain CDR2 amino acid sequence substantially the same as, or identical to, SEQ ID NO:36. In some embodiments, the antibody that binds FRα includes a light chain CDR3 amino acid sequence substantially the same as, or identical to, SEQ ID NO:37. In some embodiments, the antibody that binds FRα includes a heavy chain CDR1 amino acid sequence substantially the same as, or identical to, SEQ ID NO:39. In some embodiments, the antibody that binds FRα includes a heavy chain CDR2 amino acid sequence substantially the same as, or identical to, SEQ ID NO:40. In some embodiments, the antibody that binds FRα includes a heavy chain CDR3 amino acid sequence substantially the same as, or identical to, SEQ ID NO:41. The antibody that binds FRα may include a light chain having a CDR1 amino acid sequence substantially the same as, or identical to, SEQ ID NO:35; a CDR2 amino acid sequence substantially the same as, or identical to, SEQ ID NO:36; and a CDR3 amino acid sequence substantially the same as, or identical to, SEQ ID NO:37. The antibody that binds FRα may include a heavy chain having a CDR1 amino acid sequence substantially the same as, or identical to, SEQ ID NO:39; a CDR2 amino acid sequence substantially the same as, or identical to, SEQ ID NO: 40; and a CDR3 amino acid sequence substantially the same as, or identical to, SEQ ID NO:41. The antibody that binds FRα may include a light chain having a CDR1 amino acid sequence substantially the same as, or identical to, SEQ ID NO:35; a CDR2 amino acid sequence substantially the same as, or identical to, SEQ ID NO:36; and a CDR3 amino acid sequence substantially the same as, or identical to, SEQ ID NO:37, and also have a heavy chain having a CDR1 amino acid sequence substantially the same as, or identical to, SEQ ID NO:39; a CDR2 amino acid sequence substantially the same as, or identical to, SEQ ID NO:40; and a CDR3 amino acid sequence substantially the same as, or identical to, SEQ ID NO:41.

The antibody that binds FRα may include a light chain variable domain that includes an amino acid sequence substantially the same as, or identical to, SEQ ID NO:38. The antibody that binds FRα may include a heavy chain variable domain that includes an amino acid sequence substantially the same as, or identical to, SEQ ID NO:42. The antibody that binds FRα may include a light and a heavy chain variable domains, wherein the light chain variable domain includes an amino acid sequence substantially the same as, or identical to, SEQ ID NO:38, and the heavy chain variable domain includes an amino acid sequence substantially the same as, or identical to, SEQ ID NO:42. In some embodiments, the antibody that binds FRα is the 19D4.B7 (19D4) antibody or an antigen-binding fragment thereof, capable of binding either a native or nonreduced form of FRα. In some embodiments, the antibody has a murine IgG2a constant region.

In some embodiments, the antibody that binds FRα is an antibody that is produced by antibody-producing cells deposited with the American Type Culture Collection (10801 University Blvd., Manassas, Virginia 20110-2209) on May 19, 2011 and have been assigned Accession No. PTA-11884. In some embodiments, the antibody that binds FRα comprises one or more of the light and heavy chain CDRs of the antibodies produced by the deposited antibody-producing cells. In some embodiments, antibody that binds FRα comprises the light and heavy chain variable regions of the antibodies produced by the deposited antibody-producing cells.

<u>24F12</u>

In some embodiments, the antibody that binds FRα is an antibody or antigen-binding fragment that includes a light chain CDR1 amino acid sequence substantially the same as, or identical to, SEQ ID NO:43. In some embodiments, the antibody that binds FRα includes a light chain CDR2 amino acid sequence substantially the same as, or identical to, SEQ ID NO:44. In some embodiments, the antibody that binds FRα includes a light chain CDR3 amino acid sequence substantially the same as, or identical to, SEQ ID NO:45. In some embodiments, the antibody that binds FRα includes a heavy chain CDR1 amino acid sequence substantially the same as, or identical to, SEQ ID NO:47. In some embodiments, the antibody that binds FRα includes a heavy chain CDR2 amino acid sequence substantially the same as, or identical to, SEQ ID NO:48. In some embodiments, the antibody that binds FRα includes a heavy chain CDR3 amino acid sequence substantially the same as, or identical to, SEQ ID NO:49. The antibody that binds FRα may include a light chain having a CDR1 amino acid sequence substantially the same as, or identical to, SEQ ID NO:43; a CDR2 amino acid sequence substantially the same as, or identical to, SEQ ID NO:44; and a CDR3 amino acid sequence substantially the same as, or identical to, SEQ ID NO:45. The antibody that binds FRα may include a heavy chain having a CDR1 amino acid sequence substantially the same as, or identical to, SEQ ID NO:47; a CDR2 amino acid sequence substantially the same as, or identical to, SEQ ID NO: 48; and a CDR3 amino acid sequence substantially the same as, or identical to, SEQ ID NO:49. The antibody that binds FRα may include a light chain having a CDR1 amino acid sequence substantially the same as, or identical to, SEQ ID NO:43; a CDR2 amino acid sequence substantially the same as, or identical to, SEQ ID NO:44; and a CDR3 amino acid sequence substantially the same as, or identical to, SEQ ID NO:45, and also have a heavy chain having a CDR1 amino acid sequence substantially the same as, or identical to, SEQ ID NO:47; a CDR2 amino acid sequence substantially the same as, or identical to, SEQ ID NO:48; and a CDR3 amino acid sequence substantially the same as, or identical to, SEQ ID NO:49.

The antibody that binds FRα may include a light chain variable domain that includes an amino acid sequence substantially the same as, or identical to, SEQ ID NO:46. The antibody that binds FRα may include a heavy chain variable domain that includes an amino acid sequence substantially the same as, or identical to, SEQ ID NO:50. The antibody that binds FRα may include a light and a heavy chain variable domains, wherein the light chain variable domain includes an amino acid sequence substantially the same as, or identical to, SEQ ID NO:46, and the heavy chain variable domain includes an amino acid sequence substantially the same as, or identical to, SEQ ID NO:50. In some embodiments the antibody that binds FRα is the 24F12.B1 (24F12) antibody or an antigen-binding fragment thereof, capable of binding either a native or nonreduced form of FRα. In some embodiments, the antibody has a murine IgG1 constant region.

In some embodiments, the antibody that binds FRα is an antibody that is produced by antibody-producing cells deposited with the American Type Culture Collection (10801 University Blvd., Manassas, Virginia 20110-2209) on May 19, 2011 and have been assigned Accession No. PTA-11886. In some embodiments, the antibody that binds FRα comprises one or more of the light and heavy chain CDRs of the antibodies produced by the deposited antibody-producing cells. In some embodiments, antibody that binds FRα comprises the light and heavy chain variable regions of the antibodies produced by the deposited antibody-producing cells.

<u>26B3</u>

In some embodiments, the antibody that binds FRα is an antibody or antigen-binding fragment that includes a light chain CDR1 amino acid sequence substantially the same as, or identical to, SEQ ID NO:51. In some embodiments, the antibody that binds FRα includes a light chain CDR2 amino acid sequence substantially the same as, or identical to, SEQ ID NO:52. In some embodiments, the antibody that binds FRα includes a light chain CDR3 amino acid sequence substantially the same as, or identical to, SEQ ID NO:53. In some embodiments, the antibody that binds FRα includes a heavy chain CDR1 amino acid sequence substantially the same as, or identical to, SEQ ID NO:55. In some embodiments, the antibody that binds FRα includes a heavy chain CDR2 amino acid sequence substantially the same as, or identical to, SEQ ID NO:56. In some embodiments, the antibody that binds FRα includes a heavy chain CDR3 amino acid sequence substantially the same as, or identical to, SEQ ID NO:57. The antibody that binds FRα may include a light chain having a CDR1 amino acid sequence substantially the same as, or identical to, SEQ ID NO:51; a CDR2 amino acid sequence substantially the same as, or identical to, SEQ ID NO:52; and a CDR3 amino acid sequence substantially the same as, or identical to, SEQ ID NO:53. The antibody that binds FRα may include a heavy chain having a CDR1 amino acid sequence substantially the same as, or identical to, SEQ ID NO:55; a CDR2 amino acid sequence substantially the same as, or identical to, SEQ ID NO:56; and a CDR3 amino acid sequence substantially the same as, or identical to, SEQ ID NO:57. The antibody that binds FRα may include a light chain having a CDR1 amino acid sequence substantially the same as, or identical to, SEQ ID NO:51; a CDR2 amino acid sequence substantially the same as, or identical to, SEQ ID NO:52; and a CDR3 amino acid sequence substantially the same as, or identical to, SEQ ID NO:53, and also have a heavy chain having a CDR1 amino acid sequence substantially the same as, or identical to, SEQ ID NO:55; a CDR2 amino acid sequence substantially the same as, or identical to, SEQ ID NO:56; and a CDR3 amino acid sequence substantially the same as, or identical to, SEQ ID NO:57.

The antibody that binds FRα may include a light chain variable domain that includes an amino acid sequence substantially the same as, or identical to, SEQ ID NO:54. The antibody that binds FRα may include a heavy chain variable domain that includes an amino acid sequence substantially the same as, or identical to, SEQ ID NO:58. The antibody that binds FRα may include a light and a heavy chain variable domains, wherein the light chain variable domain includes an amino acid sequence substantially the same as, or identical to, SEQ ID NO:54, and the heavy chain variable domain includes an amino acid sequence substantially the same as, or identical to, SEQ ID NO:58. In some embodiments the antibody that binds FRα is the 26B3.F2 (26B3) antibody or an antigen-binding fragment thereof, capable of binding either a native or nonreduced form of FRα. In some embodiments, the antibody has a murine IgG1 constant region.

In some embodiments, the antibody that binds FRα is an antibody that is produced by antibody-producing cells deposited with the American Type Culture Collection (10801 University Blvd., Manassas, Virginia 20110-2209) on May 19, 2011 and have been assigned Accession No. PTA-11885. In some embodiments, the antibody that binds FRα comprises one or more of the light and heavy chain CDRs of the antibodies produced by the deposited antibody-producing cells. In some embodiments, antibody that binds FRα comprises the light and heavy chain variable regions of the antibodies produced by the deposited antibody-producing cells.

Antigen binding arrangements of CDRs may be engineered using antibody-like proteins as CDR scaffolding. Engineered antigen-binding proteins are included within the scope of antibodies that bind FRα.

Other reagent antibodies that bind FRα are known in the art, and presently, multiple such reagent antibodies are commercially available (based on search of anti-FRα antibodies at http://www.biocompare.com), as listed in the table below.

| Product | Company | Quantity | Applications | Reactivity |
|---|---|---|---|---|
| Mouse Anti-Human FRα Purified - MaxPab Polyclonal Antibody, Unconjugated | Abnova Corporation | 50 μg | Detection Antibody, Western Blot (Transfected lysate) | Human |
| Mouse Anti-Human FRα Purified MaxPab Polyclonal Antibody, Unconjugated | Abnova Corporation | 50 μg | Detection Antibody, Western Blot (Transfected lysate) | Human |
| Rabbit Anti-Human FRα Purified MaxPab Polyclonal Antibody, Unconjugated | Abnova Corporation | 100 μg | Detection Antibody, Western Blot (Transfected lysate) | Human |
| Rabbit Anti-FRα Polyclonal Antibody, Unconjugated | Aviva Systems Biology | 50 μg | Western Blot | Human, Mouse, Rat |
| Rabbit Anti-Human FRα Polyclonal Antibody, Unconjugated | GeneTex | 100 μL | Western blot. The usefulness of this product in other applications has not been determined. | Human |
| Goat Anti-Bovine Folate Receptor Alpha (FRα) Polyclonal, Biotin Conjugated | LifeSpan BioSciences | 10 mg | ELISA (1:4000 - 1:20000), Immunofluorescence, Immunohistochemistry, Western Blot | Bovine |
| Goat Anti-Bovine Folate Receptor Alpha (FRα) Polyclonal, Biotin Conjugated | LifeSpan BioSciences | Not provided | ELISA (1:5000 - 1:25000), Western Blot | Bovine |
| Goat Anti-Bovine Folate Receptor Alpha (FRα) Polyclonal, Hrp Conjugated | LifeSpan BioSciences | 20 mg | ELISA (1:2000 - 1:10000), Immunohistochemistry, Western Blot | Bovine |

(continued)

| Product | Company | Quantity | Applications | Reactivity |
|---|---|---|---|---|
| Goat Anti-Bovine Folate Receptor Alpha (FRα) Polyclonal, Hrp Conjugated | LifeSpan BioSciences | 1000 μg | ELISA (1:2000 - 1:12000), Gel Shift, Immunohistochemistry (1:100 - 1:200), Immunohistochemistry | Bovine |
| Goat Anti-Bovine Folate Receptor Alpha (FRα) Polyclonal, Hrp Conjugated | LifeSpan BioSciences | 2000 μg (200 μl) | ELISA, Western Blot | Bovine |
| Goat Anti-Bovine Folate Receptor Alpha (FRα) Polyclonal, Hrp Conjugated | LifeSpan BioSciences | Not provided | ELISA, Immunohistochemistry (Frozen sections), Immunohistochemistry (Parrafin), Western Blot | Bovine |
| Goat Anti-Bovine Folate Receptor Alpha (FRα) Polyclonal, Unconjugated | LifeSpan BioSciences | 50 mg | ELISA (1:10000 - 1:40000), Immunoprecipitation, Western Blot | Bovine |
| Goat Anti-Bovine Folate Receptor Alpha (FRα) Polyclonal, Unconjugated | LifeSpan BioSciences | 10000 μg | ELISA (1:10000 - 1:40000), Immunoprecipitation, Western Blot | Bovine |
| Goat Anti-Bovine Folate Receptor Alpha (FRα) Polyclonal, Unconjugated | LifeSpan BioSciences | 1 ml | ELISA (1:3000 - 1:9000), Immunoprecipitation, Western Blot | Bovine |
| Goat Anti-Bovine Folate Receptor Alpha (FRα) Polyclonal, Unconjugated Folate Receptor Alpha (FRα) | LifeSpan BioSciences | Not provided | ELISA (1:3000 - 1:9000), Immunoprecipitation, Western Blot | Bovine |
| Mouse Anti-Bovine Folate Receptor Alpha (FRα) Monoclonal, Unconjugated | LifeSpan BioSciences | 200 μg | ELISA | Bovine |
| Mouse Anti-Bovine Folate Receptor Alpha (FRα) Monoclonal, Unconjugated | LifeSpan BioSciences Folate Receptor Alpha (FRα) | 200 μg | ELISA | Human |
| Mouse Anti-Bovine Folate Receptor Alpha (FRα) Monoclonal, Unconjugated | LifeSpan BioSciences | 200 μg | ELISA | Human |
| Mouse Anti-Bovine Folate Receptor Alpha (FRα) Monoclonal, Unconjugated | LifeSpan BioSciences | 200 μg | ELISA | Not provided |
| Mouse Anti-Human Folate Receptor Alpha (FRα) Monoclonal, Unconjugated, Clone 6d398 | LifeSpan BioSciences | 100 μl | ELISA (1 - 10 μg/ml), Flow Cytometry, Immunocytochemistry, Immunohistochemistry (Frozen sections) | Monkey |
| Rabbit Anti-Bovine Folate Receptor Alpha (FRα) Polyclonal, Unconjugated | LifeSpan BioSciences | 1 ml | ELISA | Bovine |
| Rabbit Anti-Bovine Folate Receptor Alpha (FRα) Polyclonal, Unconjugated | LifeSpan BioSciences | Not provided | Not provided | Bovine |

(continued)

| Product | Company | Quantity | Applications | Reactivity |
|---|---|---|---|---|
| Mouse Anti-Human FRα Polyclonal antibody, Unconjugated, Clone folate receptor 1 (adult) | Novus Biologicals | 0.05 ml | Western Blot, ELISA | |
| Mouse Anti-Human FRα Polyclonal, Unconjugated | Novus Biologicals | 0.05 mg | ELISA, Western Blot | Human |
| Goat Anti-Human FRα Affinity purified Polyclonal antibody, Biotin Conjugated | R&D Systems | 50 μg | Western Blot | Human |
| Goat Anti-Human FRα Affinity purified Polyclonal antibody, Unconjugated | R&D Systems | 100 μg | Flow Cytometry, Western Blot | Human |
| Mouse Anti-Human FRα Monoclonal Antibody, Allophycocyanin Conjugated, Clone 548908 | R&D Systems | 100 Tests | Flow Cytometry | Human |
| Mouse Anti-Human FRα Monoclonal Antibody, Phycoerythrin Conjugated, Clone 548908 | R&D Systems | 100 Tests | Flow Cytometry | Human |
| Mouse Anti-Human FRα Monoclonal antibody, Unconjugated, Clone 548908 | R&D Systems | 100 μg | Flow Cytometry, Immunocytochemistry, Western Blot | Human |
| Mouse Anti- Human FRα Monoclonal Antibody, Unconjugated | United States Biological | 100 μg | ELISA, Flow Cytometry, Immunocytochemistry, Western Blot | Human |

In a preferred embodiment, the antibody that binds FRα comprises at least one of the following CDRs, as derived from the murine LK26 heavy and light chains: SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3. See US Patent No. 5,646,253.

Further mutations may be made in the framework regions as taught in US Patent No. 5,646,253.

In another preferred embodiment, the antibody includes a variable region light chain selected from the group consisting of LK26HuVK (SEQ ID NO: 13) LK26HuVKY (SEQ ID NO: 14), LK26HuVKPW (SEQ ID NO: 15), and LK26HuVKPW,Y (SEQ ID NO: 16); and a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21). See US Patent No. 5,646,253 and US Patent No. 6,124,106. In another embodiment, the antibody comprises the heavy chain variable region LK26KOLHuVH (SEQ ID NO: 21) and the light chain variable region LK26HuVKPW,Y (SEQ ID NO: 16). In another embodiment, the antibody comprises the heavy chain variable region LK26HuVH SLF (SEQ ID NO: 19) and the light chain variable region LK26HuVKPW,Y (SEQ ID NO: 16). In a further embodiment, the antibody comprises the heavy chain variable region LK26HuVH FAIS,N (SEQ ID NO: 18) and the light chain variable region LK26HuVKPW,Y (SEQ ID NO: 16).

In some embodiments, samples may need to be modified in order to make FRα accessible to antibody binding. In a particular aspect of the immunocytochemistry or immunohistochemistry methods, slides may be transferred to a pretreatment buffer and optionally heated to increase antigen accessibility. Heating of the sample in the pretreatment buffer rapidly disrupts the lipid bi-layer of the cells and makes the antigens (may be the case in fresh specimens, but not typically what occurs in fixed specimens) (*i.e.,* the FRα protein) more accessible for antibody binding. The term "pretreatment buffer" are used interchangeably herein to refer to a buffer that is used to prepare cytology or histology samples for immunostaining, particularly by increasing FRα protein accessibility for antibody binding. The pretreatment buffer may comprise a pH-specific salt solution, a polymer, a detergent, or a nonionic or anionic surfactant such as, for example, an ethyloxylated anionic or nonionic surfactant, an alkanoate or an alkoxylate or even blends of these surfactants or even the use of a bile salt. The pretreatment buffer may, for example, be a solution of 0.1% to 1% of deoxycholic acid, sodium salt, or a solution of sodium laureth-13-carboxylate (*e.g.,* Sandopan LS) or and ethoxylated anionic complex. In some embodiments, the pretreatment buffer may also be used as a slide storage buffer. In a particular embodiment, the sample, for example, the urine sample, is centrifuged, vortexed, diluted and/or subjected to guanidine treatment.

Any method for making FRα protein more accessible for antibody binding may be used in the practice of the invention, including the antigen retrieval methods known in the art. See, for example, Bibbo, et al. (2002) Acta. Cytol. 46:25-29; Saqi, et al. (2003) Diagn. Cytopathol. 27:365-370; Bibbo, et al. (2003) Anal. Quant. Cytol. Histol. 25:8-11.

Following pretreatment to increase FRα protein accessibility, samples may be blocked using an appropriate blocking agent, *e.g.,* a peroxidase blocking reagent such as hydrogen peroxide. In some embodiments, the samples may be blocked using a protein blocking reagent to prevent non-specific binding of the antibody. The protein blocking reagent may comprise, for example, purified casein. An antibody, particularly a monoclonal or polyclonal antibody, that specifically binds to FRα is then incubated with the sample.

Techniques for detecting antibody binding are well known in the art. Antibody binding to FRα may be detected through the use of chemical reagents that generate a detectable signal that corresponds to the level of antibody binding and, accordingly, to the level of FRα protein expression. In one of the immunohistochemistry or immunocytochemistry methods of the invention, antibody binding is detected through the use of a secondary antibody that is conjugated to a labeled polymer. Examples of labeled polymers include but are not limited to polymer-enzyme conjugates. The enzymes in these complexes are typically used to catalyze the deposition of a chromagen at the antigen-antibody binding site, thereby resulting in cell staining that corresponds to expression level of the biomarker of interest. Enzymes include, but are not limited to, horseradish peroxidase (HRP) and alkaline phosphatase (AP).

In one immunohistochemistry or immunocytochemistry method of the invention, antibody binding to the FRα protein is detected through the use of an HRP-labeled polymer that is conjugated to a secondary antibody. Antibody binding can also be detected through the use of a species-specific probe reagent, which binds to monoclonal or polyclonal antibodies, and a polymer conjugated to HRP, which binds to the species specific probe reagent. Slides are stained for antibody binding using any chromagen, *e.g.,* the chromagen 3,3-diaminobenzidine (DAB), and then counterstained with hematoxylin and, optionally, a bluing agent such as ammonium hydroxide or TBS/Tween-20. Other suitable chromagens include, for example, 3-amino-9-ethylcarbazole (AEC). In some aspects of the invention, slides are reviewed microscopically by a cytotechnologist and/or a pathologist to assess cell staining, *e.g.,* fluorescent staining (*i.e.*, FRα expression). Alternatively, samples may be reviewed *via* automated microscopy or by personnel with the assistance of computer software that facilitates the identification of positive staining cells.

In a preferred embodiment of the invention, the antibody is labeled. For example, detection of antibody binding can be facilitated by coupling the anti-FRα antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include $^{125}I$, $^{131}I$, $^{35}S$, $^{14}C$, or $^{3}H$. In a particular embodiment, the antibody is labeled with a radiolabel, chromophore-label, fluorophore-label, or enzyme-label.

In one embodiment of the invention frozen samples are prepared as described above and subsequently stained with antibodies against FRα diluted to an appropriate concentration using, for example, Tris-buffered saline (TBS). Primary antibodies can be detected by incubating the slides in biotinylated anti-immunoglobulin. This signal can optionally be amplified and visualized using diaminobenzidine precipitation of the antigen. Furthermore, slides can be optionally counterstained with, for example, hematoxylin, to visualize the cells.

In another embodiment, fixed and embedded samples are stained with antibodies against FRα and counterstained as described above for frozen sections. In addition, samples may be optionally treated with agents to amplify the signal in order to visualize antibody staining. For example, a peroxidase-catalyzed deposition of biotinyl-tyramide, which in turn is reacted with peroxidase-conjugated streptavidin (Catalyzed Signal Amplification (CSA) System, DAKO, Carpinteria, CA) may be used.

One of skill in the art will recognize that the concentration of a particular antibody used to practice the methods of the invention will vary depending on such factors as time for binding, level of specificity of the antibody for FRα, and method of sample preparation. Moreover, when multiple antibodies are used, the required concentration may be affected by the order in which the antibodies are applied to the sample, *e.g.,* simultaneously as a cocktail or sequentially as individual antibody reagents. Furthermore, the detection chemistry used to visualize antibody binding to FRα must be optimized to produce the desired signal to noise ratio.

In one embodiment of the invention, proteomic methods, *e.g.,* mass spectrometry, are used for detecting and quantitating the FRα protein. For example, matrix-associated laser desorption/ionization time-of-flight mass spectrometry (MALDI-TOF MS) or surface-enhanced laser desorption/ionization time-of-flight mass spectrometry (SELDI-TOF MS) which involves the application of a sample, such as serum, to a protein-binding chip (Wright, G.L., Jr., et al. (2002) Expert Rev Mol Diagn 2:549; Li, J., et al. (2002) Clin Chem 48:1296; Laronga, C., et al. (2003) Dis Markers 19:229; Petricoin, E.F., *et al.* (2002) 359:572; Adam, B.L., et al. (2002) Cancer Res 62:3609; Tolson, J., et al. (2004) Lab Invest 84:845; Xiao, Z., et al. (2001) Cancer Res 61:6029) can be used to detect and quantitate the FRα protein. Mass spectrometric methods are described in, for example, U.S. Patent Nos. 5,622,824, 5,605,798 and 5,547,835.

The present invention is further predicated, at least in part, on the identification of FRα as a prognostic biomarker, *i.e.*, as a biomarker of the progression and/ or severity, of an FRα-expressing cancer such as ovarian cancer or non-small cell lung cancer. Accordingly, the present invention provides methods of assessing the progression of an FRα-expressing cancer in a subject afflicted with ovarian cancer by comparing the level of FRα in a sample derived from a subject with the level of FRα in a control sample, wherein a difference in the level of FRα in the sample (such as a urine or serum sample) derived from the subject compared with the control sample is an indication that the cancer will progress rapidly. Similarly, methods of assessing the level of risk that a subject will develop an FRα-expressing cancer involve comparing the level of FRα in a sample derived from a subject with the level of FRα in a control sample, wherein a difference in the level of FRα in the sample (such as urine or serum sample) derived from the subject compared with the control sample is an indication that the subject has a higher level of risk of developing an FRα-expressing cancer as compared to normal risk in a healthy individual.

In one embodiment, the difference is an increase. In another embodiment, the difference is a decrease. In some types of cancers (*e.g.,* squamous cell carcinoma of the head and neck, ovarian cancer), a higher level of FRα expression is associated with a worse prognosis, whereas in other types of cancers (*e.g.,* non-small-cell lung cancers), a higher level of FRα expression is associated with a better prognosis. Thus, in one specific embodiment, the FRα-expressing cancer is ovarian cancer or squamous cell carcinoma of the head and neck and the difference is an increase. In another specific embodiment, the FRα-expressing cancer is a non small-cell lung cancer, and the difference is a decrease.

In certain aspects, the invention provides methods of assessing the progression of an FRα-expressing cancer in a subject afflicted with an FRα-expressing cancer by comparing the level of FRα in a sample derived from a subject with the level of FRα in a control sample, wherein an increase in the level of FRα in the sample (such as a urine or serum sample) derived from the subject compared with the control sample is an indication that the cancer will progress rapidly, or a decrease in the level of FRα in the sample derived from the subject as compared with the level of FRα in the control sample is an indication that the cancer will progress slowly or will regress. Similarly, methods of assessing the level of risk that a subject will develop an FRα-expressing cancer involve comparing the level of FRα in a sample derived from a subject with the level of FRα in a control sample, wherein an increase in the level of FRα in the sample (such as urine or serum sample) derived from the subject compared with the control sample is an indication that the subject has a higher level of risk of developing an FRα-expressing cancer as compared to normal risk in a healthy individual, or a decrease in the level of FRα in the sample derived from the subject as compared with the level of FRα in the control sample is an indication that the subject has a lower level of risk of developing an FRα-expressing cancer as compared to a normal risk in a healthy individual.

Any clinically relevant or statistically significant increase or decrease, using any analytical method known in the art, may be utilized in the prognostic, risk assessment and other methods of the invention. In one embodiment, an increase in the level of FRα in the level of FRα refers to a level that exceeds a cutoff value determined using an ROC analysis as exemplified in Example 6. In another embodiment, a decrease in the level of FRα refers to a level in a test sample that does not exceed a cutoff value determined using an ROC analysis.

In other embodiments, the increase or decrease must be greater than the limits of detection of the method for determining the level of FRα. In further embodiments, the increase or decrease be at least greater than the standard error of the assessment method, and preferably a difference of at least about 2-, about 3-, about 4-, about 5-, about 6-, about 7-, about 8-, about 9-, about 10-, about 15-, about 20-, about 25-, about 100-, about 500-, about 1000-fold or greater than the standard error of the assessment method. In some embodiments, the increase or decrease is assessed using parametric or nonparametric descriptive statistics, comparisons, regression analyses, and the like.

In other embodiments, the increase or decrease is a level in the sample derived from the subject that is about 5%, about 10%, about 15%, about 20%, about 25%, about 30%, about 40%, about 50%, about 60%, about 70%, about 80%, about 90%, about 100%, about 150%, about 200%, about 300%, about 400%, about 500%, about 600%, about 700%, about 800%, about 900% or about 1000% more or less than the level of FRα in the control sample. In alternative embodiments, the increase or decrease is a level in the sample derived from the subject that is at least about 1.5, and more preferably about two, about three, about four, about five or more standard deviations above or below the average level of FRα in the control sample. As used herein, the phrase "progression of an FRα-expressing cancer in a subject afflicted with an FRα-expressing cancer" may refer to the progression of an FRα-expressing cancer from a less severe to a more severe cancer state. This could include an increase in the number or severity of tumors, the degree of metastasis, the speed with which the cancer is growing and spreading, and the like. In certain embodiments, the progression is a progression from a less severe stage to a more severe stage, wherein the stage is assessed according to a staging scheme known in the art. In one embodiment, wherein the FRα-expressing cancer is ovarian cancer, the progression refers to a progression from Stage I to Stage II, from Stage II to Stage III, etc. In another embodiment, wherein the FRα-expressing cancer is non-small cell lung cancer (NSCLC), the progression refers to a progression from Stage 0 to Stage IA, Stage IA to Stage IB, Stage IB to Stage IIA, Stage IIA to Stage IIB, Stage IIB to Stage IIC, etc. In another embodiment, wherein the FRα-expressing cancer is non-small cell lung cancer (NSCLC), the progression refers to a progression from a less severe to a more severe stage as determined under the TNM classification system. See Spira; Greene; Sobin.

Alternatively, the phrase "progression of an FRα-expressing cancer in a subject afflicted with an FRα-expressing cancer" may refer to a regression of an FRα-expressing cancer from a more severe state to a less severe state, such as a decrease in the number or severity of tumors, the degree of metastasis, the speed with which the cancer is growing and spreading, and the like. In certain embodiments, the progression is a progression from a more severe stage to a less severe stage, wherein the stage is assessed according to a staging scheme known in the art. In one embodiment, wherein the FRα-expressing cancer is ovarian cancer, the progression refers to a regression from Stage IV to Stage III, from Stage III to Stage II, etc. In another embodiment, wherein the FRα-expressing cancer is non-small cell lung cancer (NSCLC), the progression refers to a progression from Stage IV to Stage IIIB, Stage IIIB to Stage IIIA, Stage IIIA to Stage IIB, etc. In another embodiment, wherein the FRα-expressing cancer is non-small cell lung cancer (NSCLC), the progression refers to a progression from a more severe to a less severe stage as determined under the TNM classification system. See Spira; Greene; Sobin.

In further embodiments, the level of FRα may be used to calculate the likelihood that a subject is afflicted with an FRα-expressing cancer, the progression of an FRα-expressing cancer in a subject, the level of risk of developing an FRα-expressing cancer, the risk of cancer recurrence in a subject being treated for an FRα-expressing, the survival of a subject being treated for an FRα-expressing cancer, the efficacy of a treatment regimen for treating an FRα-expressing cancer, and the like, using the methods of the invention, which may include methods of regression analysis known to one of skill in the art. For example, suitable regression models include, but are not limited to CART (*e.g.*, Hill, T, and Lewicki, P. (2006) "STATISTICS Methods and Applications" StatSoft, Tulsa, OK), Cox (e.g., www.evidence-based-medicine.co.uk), exponential, normal and log normal (*e.g.,* www.obgyn.cam.ac.uk/mrg/statsbook/stsurvan.html), logistic (*e.g.,* www.en.wikipedia.org/wiki/Logistic_regression), parametric, non-parametric, semi-parametric (*e.g.,* www.socserv.mcmaster.ca/jfox/Books/Companion), linear (*e.g.,* www.en.wikipedia.org/wiki/Linear_regression), or additive (*e.g.,* www.en.wikipedia.org/wiki/Generalized_additive_model).

In one embodiment, a regression analysis includes the level of FRα. In further embodiments, a regression analysis may include additional clinical and/or molecular co-variates. Such clinical co-variates include, but are not limited to, age of the subject, tumor stage, tumor grade, tumor size, treatment regime, *e.g.,* chemotherapy and/or radiation therapy, clinical outcome (*e.g.,* relapse, disease-specific survival, therapy failure), and/or clinical outcome as a function of time after diagnosis, time after initiation of therapy, and/or time after completion of treatment. Molecular co-variates can include, but are not limited to additional molecular marker values. For example, in embodiments wherein the FRα-expressing cancer is ovarian cancer, such markers may include, *e.g.,* serum CA125 levels, serum DF3 levels, and/or plasma LPA levels.

In other aspects, the invention provides methods for monitoring the effectiveness of a therapy or treatment regimen. For example, the present invention provides methods for monitoring the efficacy of MORAb-003 treatment of ovarian cancer or lung cancer in a subject suffering from ovarian cancer or lung cancer. Specifically, the methods involve determining the level of folate receptor alpha (FRα) which is not bound to a cell, in a sample derived from said subject, wherein said subject has been previously administered MORAb-003; and comparing the level of folate receptor alpha (FRα) which is not bound to a cell with the level of FRα in a control sample, wherein an increase or no change in the level of FRα in the sample derived from said subject as compared with the level of FRα in the control sample is an indication that the MORAb-003 treatment is not efficacious; and wherein a decrease in the level of FRα in the sample derived from said subject as compared with the level of FRα in the control sample is an indication that the MORAb-003 treatment is efficacious.

For example, the control sample may be derived from a subject not subjected to the treatment regimen and a test sample may be derived from a subject subjected to at least a portion of the treatment regimen. Alternatively, the test sample and the control sample may be derived from the same subject. For example, the test sample may be a sample derived from a subject after administration of a therapeutic, such as MORAb-003. The control sample may be a sample derived from a subject prior to administration of therapeutic or at an earlier stage of therapeutic regimen. Accordingly, a decrease in the level of expression of FRα in the test sample, relative to the control sample, is an indication that therapy has decreased the progression of the FRα-expressing cancer, for example, ovarian cancer. For FRα-expressing cancers wherein a higher level of FRα is associated with a worse prognosis, such as *e.g.,* ovarian cancer or squamous cell carcinoma of the head and neck, a decrease in the level of expression of FRα in the test sample, relative to the control sample, is an indication that therapy is effective in slowing the progression of the FRα-expressing cancer, or in causing a regression of the cancer, in the subject afflicted with the FRα-expressing cancer. In a preferred embodiment, the FRα-expressing cancer is ovarian cancer.

In various embodiments of this aspect of the invention, the sample may be urine, serum, plasma or ascites. In particular embodiments, the sample is urine or serum. Moreover, the FRα may be determined by contacting the sample with an antibody that binds FRα, optionally, using antibodies as described herein and assay methods as described herein.

In various embodiments, the MORAb-003 treatment antibody is (a) an antibody that comprises the heavy chain amino acid sequence as set forth in SEQ ID NO:7 and the light chain amino acid sequence as set forth in SEQ ID NO:8; (b) an antibody that binds the same epitope as the MORAb-003 antibody; or (c) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3..

In a particular embodiment, the FRα-expressing cancer is ovarian cancer. In other embodiments, the FRα-expressing cancer is lung cancer. In more specific embodiments, the lung cancer is non-small cell lung cancer (NSCLC). In one such embodiment, the NSCLC is selected from the group consisting of adenocarcinoma, squamous cell lung carcinoma, large cell lung carcinoma, pleomorphic NSCLC, carcinoid tumor, salivary gland carcinoma, and unclassified carcinoma. In a preferred embodiment, the NSCLC is adenocarcinoma. In alternative embodiments, the lung cancer is small cell lung carcinoma (SCLC). In another embodiment, the lung cancer is bronchioalveolar carcinoma. In yet another embodiment, the lung cancer is a lung carcinoid tumor.

In another aspect, the invention provides methods of stratifying a subject with an FRα-expressing cancer into cancer therapy groups based on the determined level of FRα in a sample. In a preferred embodiment, the method involves stratifying a subject with an FRα-expressing cancer into one of at least four cancer therapy groups. In other embodiments, the method involves stratifying a subject with an FRα-expressing cancer into one of at least about two, about three, about four, about five, about six, about seven, about eight, about nine, or about ten cancer therapy groups.

According to the present invention, the levels of FRα may be associated with the severity, *i.e.*, the stage, of the FRα expressing cancer. For example, ovarian cancer is stratified into different stages based on the severity of the cancer, as set forth herein. Accordingly, the present invention provides methods for stratifying ovarian cancer into Stage I, for example, Stage IA, Stage 1B or Stage IC; Stage II, for example, Stage IIA, Stage IIB or Stage IIC; Stage III, for example, Stage IIIA, Stage IIIB or Stage IIIC; or Stage IV ovarian cancer.

SCLS or NSCLC may be stratified into different stages based on the severity of the cancer, as set forth herein. Accordingly, the present invention provides methods for stratifying the lung cancer, for example, SCLS or NSCLC, into the occult (hidden) stage; stage 0; Stage I, for example, stages IA and IB; Stage II, for example, stages IIA and IIB; Stage III, for example, stages IIIA and IIIB; or Stage IV lung cancer.

In yet another aspect, the present invention is predicated, at least in part, on the finding that FRα can serve as a predictive biomarker for treatment of FRα expressing cancers. Specifically, the methods of the present invention provide for assessing whether a subject will respond to treatment, for example, with MORAb-003, and whether and when to initiate treatment, for example, with MORAb-003, by assessing the levels of FRα in a subject.

In one aspect, the present invention provides a method for predicting whether a subject suffering from an FRα expressing cancer, for example, ovarian or lung cancer, will respond to treatment with MORAb-003, by determining the level of folate receptor alpha (FRα) which is not bound to a cell in a sample derived from said subject; and comparing the level of folate receptor alpha (FRα) which is not bound to a cell in the sample derived from said subject with the level of FRα in a control sample, wherein a difference between the level of FRα in the sample derived from said subject and the level of FRα in the control sample is an indication that the subject will respond to treatment with MORAb-003.

In certain embodiments, the degree of difference between the levels of FRα not bound to a cancer cell in the test sample as compared to the control sample is indicative that the subject will respond to treatment with MORAb-003. For example, a difference of at least about 2-, about 3-, about 4-, about 5-, about 6-, about 7-, about 8-, about 9-, about 10-, about 15-, about 20-, about 25-, about 100-, about 500-, about 1000-fold or greater than the standard error of the assessment method is indicative that the subject will respond to treatment with MORAb-003. Alternatively or in combination, a difference of at least about 5%, about 10%, about 15%, about 20%, about 25%, about 30%, about 40%, about 50%, about 60%, about 70%, about 80%, about 90%, about 100%, about 150%, about 200%, about 300%, about 400%, about 500%, about 600%, about 700%, about 800%, about 900% or about 1000% is indicative that the subject will respond to treatment with MORAb-003. Alternatively or in combination, a difference of at least about 1.5, and more preferably about two, about three, about four, about five or more standard deviations is indicative that the subject will respond to treatment with MORAb-003.

In various embodiments, the MORAb-003 treatment antibody is (a) an antibody that binds the same epitope as the MORAb-003 antibody; or (b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3.

In various embodiments, the sample is urine, plasma, serum or ascites. In particular embodiments, the sample is urine or serum. In further embodiments, the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer. In a particular embodiment, the FRα-expressing cancer is ovarian cancer. In another embodiment, the FRα-expressing cancer is non-small cell lung cancer, such as adenocarcinoma.

**B. Anti-FRα Antibody Based Assays for Detecting FRα-Expressing Cancers**

There are a variety of assay formats known to those of ordinary skill in the art for using an antibody to detect a polypeptide in a sample, including but not limited to enzyme linked immunosorbent assay (ELISA), radioimmunoassay (RIA), immunofluorimetry, immunoprecipitation, solution phase assay, equilibrium dialysis, immunodiffusion and other techniques. See, *e.g.,* Harlow and Lane, Antibodies: A Laboratory Manual, Cold Spring Harbor Laboratory, 1988; Weir, D. M., Handbook of Experimental Immunology, 1986, Blackwell Scientific, Boston. For example, the assay may be performed in a Western blot format, wherein a protein preparation from the biological sample is submitted to gel electrophoresis, transferred to a suitable membrane and allowed to react with the antibody. The presence of the antibody on the membrane may then be detected using a suitable detection reagent, as is well known in the art and described below.

In another embodiment, the assay involves the use of an antibody immobilized on a solid support to bind to the target FRα polypeptide and remove it from the remainder of the sample. The bound FRα polypeptide may then be detected using a second antibody reactive with a distinct FRα polypeptide antigenic determinant, for example, a reagent that contains a detectable reporter moiety. As a non-limiting example, according to this embodiment the immobilized antibody and the second antibody which recognize distinct antigenic determinants may be any two of the monoclonal antibodies described herein selected from MORAb-003, MOV18, 548908, 6D398 or variants thereof as described herein. Alternatively, a competitive assay may be utilized, in which FRα is labeled with a detectable reporter moiety and allowed to bind to the immobilized anti-FRα antibody after incubation of the immobilized antibody with the sample. The extent to which components of the sample inhibit the binding of the labeled polypeptide to the antibody is indicative of the reactivity of the sample with the immobilized antibody, and as a result, indicative of the level of FRα in the sample.

The solid support may be any material known to those of ordinary skill in the art to which the antibody may be attached, such as a test well in a microtiter plate, a nitrocellulose filter or another suitable membrane. Alternatively, the support may be a bead or disc, such as glass, fiberglass, latex or a plastic such as polystyrene or polyvinylchloride. The antibody may be immobilized on the solid support using a variety of techniques known to those in the art, which are amply described in the patent and scientific literature.

In certain preferred embodiments, the assay for detection of FRα in a sample is a two-antibody sandwich assay. This assay may be performed by first contacting a FRα specific antibody (*e.g.*, MORAb-003, MOV18, 548908, 6D398 or variants thereof as described herein) that has been immobilized on a solid support, commonly the well of a microtiter plate, with the biological sample, such that a soluble molecule naturally occurring in the sample and having an antigenic determinant that is reactive with the antibody is allowed to bind to the immobilized antibody (*e.g.,* a 30 minute incubation time at room temperature is generally sufficient) to form an antigen-antibody complex or an immune complex. Unbound constituents of the sample are then removed from the immobilized immune complexes. Next, a second antibody specific for FRα is added, wherein the antigen combining site of the second antibody does not competitively inhibit binding of the antigen combining site of the immobilized first antibody to FRα (*e.g.,* MORAb-003, MOV18, 548908, 6D398 or variants thereof as described herein, that is not the same as the monoclonal antibody immobilized on the solid support). The second antibody may be detectably labeled as provided herein, such that it may be directly detected. Alternatively, the second antibody may be indirectly detected through the use of a detectably labeled secondary (or "second stage") anti-antibody, or by using a specific detection reagent as provided herein. The subject invention method is not limited to any particular detection procedure, as those having familiarity with immunoassays will appreciate that there are numerous reagents and configurations for immunologically detecting a particular antigen (*e.g.,* FRα) in a two-antibody sandwich immunoassay.

In certain preferred embodiments of the invention using the two-antibody sandwich assay described above, the first, immobilized antibody specific for FRα is a polyclonal antibody and the second antibody specific for FRα is a polyclonal antibody. In certain other embodiments of the invention, the first, immobilized antibody specific for FRα is a monoclonal antibody and the second antibody specific for FRα is a polyclonal antibody. In certain other embodiments of the invention the first, immobilized antibody specific for FRα is a polyclonal antibody and the second antibody specific for FRα is a monoclonal antibody. In certain other embodiments of the invention, the first, immobilized antibody specific for FRα is a monoclonal antibody and the second antibody specific for FRα is a monoclonal antibody. For example, in these embodiments it should be noted that monoclonal antibodies MORAb-003, MOV18, 548908, 6D398 or variants thereof as described herein, as provided herein recognize distinct and noncompetitive antigenic determinants (*e.g.,*

epitopes) on FRα polypeptides, such that any pairwise combination of these monoclonal antibodies may be employed. In other preferred embodiments of the invention, the first, immobilized antibody specific for FRα and/or the second antibody specific for FRα may be any of the kinds of antibodies known in the art and referred to herein, for example, by way of illustration and not limitation, Fab fragments, $F(ab')_2$ fragments, immunoglobulin V-region fusion proteins or single chain antibodies. Those familiar with the art will appreciate that the present invention encompasses the use of other antibody forms, fragments, derivatives and the like in the methods disclosed and claimed herein.

In certain particularly preferred embodiments, the second antibody may contain a detectable reporter moiety or label such as an enzyme, dye, radionuclide, luminescent group, fluorescent group or biotin, or the like. The amount of the second antibody that remains bound to the solid support is then determined using a method appropriate for the specific detectable reporter moiety or label. For radioactive groups, scintillation counting or autoradiographic methods are generally appropriate. Antibody-enzyme conjugates may be prepared using a variety of coupling techniques (for review see, *e.g.,* Scouten, W. H., Methods in Enzymology 135:30-65, 1987). Spectroscopic methods may be used to detect dyes (including, for example, colorimetric products of enzyme reactions), luminescent groups and fluorescent groups. Biotin may be detected using avidin or streptavidin, coupled to a different reporter group (commonly a radioactive or fluorescent group or an enzyme). Enzyme reporter groups may generally be detected by the addition of substrate (generally for a specific period of time), followed by spectroscopic, spectrophotometric or other analysis of the reaction products. Standards and standard additions may be used to determine the level of mesothelin polypeptide in a sample, using well known techniques.

A method of screening for the presence of an FRα expressing cancer according to the present invention may be further enhanced by the detection of more than one tumor associated marker in a biological sample from a subject. Accordingly, in certain embodiments the present invention provides a method of screening that, in addition to detecting reactivity of FRα not bound to a cell, also includes detection of at least one additional soluble marker of a malignant condition using established methods as known in the art and provided herein. As noted above, there are currently a number of soluble tumor associated antigens that are detectable in samples of readily obtained biological fluids.

### C. Kits of the Invention

The invention also provides kits for assessing whether a subject is afflicted with an FRα-expressing cancer, for assessing the progression of an FRα-expressing cancer, for assessing the level of risk that a subject will develop an FRα-expressing cancer, or for monitoring the effectiveness of a therapy or treatment regimen for an FRα-expressing cancer. These kits include means for determining the level of expression of FRα and instructions for use of the kit to assess the progression of an FRα-expressing cancer, to assess the level of risk that a subject will develop an FRα-expressing cancer, or to monitor the effectiveness of a therapy or treatment regimen for an FRα-expressing cancer.

The kits of the invention may optionally comprise additional components useful for performing the methods of the invention. By way of example, the kits may comprise means for obtaining a sample from a subject, a control sample, *e.g.,* a sample from a subject having slowly progressing cancer and/or a subject not having cancer, one or more sample compartments, and instructional material which describes performance of a method of the invention and tissue specific controls/standards.

The means for determining the level of FRα include known methods in the art for assessing protein levels, as discussed above, and specific preferred embodiments, for example, utilizing the MORAb-003 antibody, as discussed herein. Thus, for example, in one embodiment, the level of FRα is assessed by contacting a sample derived from a subject (such as urine or serum) with a folate receptor alpha (FRα) binding agent. In a preferred embodiment, the binding agent is an antibody. Many of the types of antibodies that bind FRα are discussed above in the methods of the invention and may also be utilized in the kits of the invention.

The means for determining the level of FRα can further include, for example, buffers or other reagents for use in an assay for determining the level of FRα. The instructions can be, for example, printed instructions for performing the assay and/or instructions for evaluating the level of expression of FRα.

The kits of the inventions may also include means for isolating a sample from a subject. These means can comprise one or more items of equipment or reagents that can be used to obtain a fluid or tissue from a subject. The means for obtaining a sample from a subject may also comprise means for isolating blood components, such as serum, from a blood sample. Preferably, the kit is designed for use with a human subject.

### III. Screening Assays

In further embodiments, the invention also provides methods (also referred to herein as "screening assays") for identifying modulators, *i.e.*, candidate or test compounds or agents (*e.g.*, proteins, peptides, peptidomimetics, peptoids, small molecules or other drugs), which modulate the growth, progression and/or aggressiveness of cancer, *e.g.,* an FRα-expressing cancer, or a cancer cell, *e.g.,* an ovarian cancer cell, by monitoring and comparing the levels of FRα in a sample. Such assays typically comprise a test compound, or a combination of test compounds, whose activity against cancer or a cancer cell is to be evaluated. Compounds identified *via* assays such as those described herein may be useful, for example, for modulating, *e.g.,* inhibiting, ameliorating, treating, or preventing aggressiveness of an FRα-expressing cancer or a cancer cell, *e.g.,* an ovarian cancer cell. By monitoring the level of FRα in a sample, one can determine whether the FRα-expressing cancer is progressing or regressing and whether the test compound has the desired effect. For example, in embodiments wherein the FRα-expressing cancer is a cancer for which higher levels of FRα are associated with a worse prognosis, a decrease in the level of FRα after administration of the test compound(s) would be indicative of the efficacy of the test compound. By contrast, an increase in the level of FRα after administration of the test compound(s) would indicate that the test compound is not effective in treating ovarian cancer. By contrast, in embodiments wherein the FRα-expressing cancer is a cancer for which higher levels of FRα are associated with a better prognosis, an increase in the level of FRα after administration of the test compound(s) would be indicative of the efficacy of the test compound. By contrast, an decrease in the level of FRα after administration of the test compound(s) would indicate that the test compound is not effective in treating ovarian cancer.

The test compounds used in the screening assays of the present invention may be obtained from any available source, including systematic libraries of natural and/or synthetic compounds. Test compounds may also be obtained by any of the numerous approaches in combinatorial library methods known in the art, including biological libraries; peptoid libraries (libraries of molecules having the functionalities of peptides, but with a novel, non-peptide backbone which are resistant to enzymatic degradation but which nevertheless remain bioactive; see, *e.g.,* Zuckermann et al., 1994, J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, 1997, Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (*e.g*., Houghten, 1992, Biotechniques 13:412-421), or on beads (Lam, 1991, Nature 354:82-84), chips (Fodor, 1993, Nature 364:555-556), bacteria and/or spores, (Ladner, USP 5,223,409), plasmids (Cull et al, 1992, Proc Natl Acad Sci USA 89:1865-1869) or on phage (Scott and Smith, 1990, Science 249:386-390; Devlin, 1990, Science 249:404-406; Cwirla et al, 1990, Proc. Natl. Acad. Sci. 87:6378-6382; Felici, 1991, J. Mol. Biol. 222:301-310; Ladner, *supra*.).

The present invention is further illustrated by the following examples.

## EXAMPLES

### EXAMPLE 1. DETERMINATION OF FRα LEVELS IN URINE SAMPLES FROM HUMAN SUBJECTS WITH AND WITHOUT OVARIAN CANCER AS MEASURED BY ELECTROCHEMILUMINESCENCE IMMUNOASSAY (ECLIA).

***Materials and Methods***

Urine samples were obtained from human subjects, including subjects afflicted with ovarian cancer and normal control subjects not afflicted with ovarian cancer. The levels of FRα in urine samples were determined using an electrochemiluminescence immunoassay (ECLIA) according to the following procedure (see Namba et al. (1999) Analytical Science 15:1087-1093):

*i. Antibody coating to micro beads*

The monoclonal anti-folate receptor alpha antibody was coated over the surface of micro beads (Dynabeads M-450 Epoxy, Dynal). Thirty six milligrams of micro beads were mixed with 1.2 mL of antibody MOV18 (0.36 mg/mL, Enzo Life Science) in 0.15 mol/L phosphate buffer saline pH 7.8 (PBS), followed by gentle mixing for 16 hours at room temperature. The micro beads were then washed 5 times with 50 mM HEPES buffer containing 0.1% normal rabbit serum (NRS), 150 mmol/L NaCl, 0.01% Tween 20 pH 7.5 (wash buffer). Thereafter, the coated micro beads were suspended in 1.2 mL 50 mM HEPES buffer containing 20% NRS, 150 mmol/L NaCl and 0.01% Tween 20 pH 7.5 (reaction buffer) to block the unbound surface, followed by gentle mixing for 3.5 hours at room temperature. Finally, the micro beads were washed 5 times with wash buffer and re-suspended with 1.2 mL 50 mM HEPES buffer containing 10% NRS, 150 mmol/L NaCl, 10 mmol/L EDTA-2Na and 0.01% Tween 20 pH 7.5 (reaction buffer) so that the concentration of micro beads was 30 mg/mL. The micro beads were stored at 4°C until use.

*ii. Antibody labeling with Ruthenium-chelate-NHS (Ru)*

One milliliter of MORAB-003 (1 mg/mL) in PBS was mixed with 14 μL of Ru (10 mg/mL), initial molar ratio of antibody to Ru was 1:20, followed by shaking for 30 minutes at room temperature in the dark. The reaction was terminated by adding 25 μL of 2 mol/L glycine solution followed by incubation for 20 minutes. The labeled antibody was purified by gel filtration using Sephadex G-25 (GE Healthcare) eluted with PBS. The first eluted yellow fraction was collected and the concentration of antibody and Ru were determined by means of the Pierce BCA protein assay kit (Thermo Scientific) and absorption at 455 nm respectively. The final molar ratio was calculated by the formula: Final molar ratio = [(absorption at 455)/13700]/[Ab(mg/mL/150000)]. The labeled antibody was stored at 4°C until use.

*iii. One Step Immunoassay*

The antibody coated micro beads were set on the reagent table of the Picolumi 8220 (Sanko, Tokyo, Japan) after adjusting the concentration of the beads to 1.5 mg/ml (working solution) in reaction buffer. The Ru labeled antibody was set on the reagent table of the Picolumi 8220 after adjusting the concentration of antibody to 2 μg/ml (working solution) in reaction buffer.

Ten microliters of urine (diluted 1:51 in reaction buffer) or standard FRα (prepared in reaction buffer) and 100 μL of reaction buffer were dispensed into a reaction tube (Sanko, Tokyo, Japan) and set on Picolumi 8220.

The following steps were automatically run by Picolumi 8220. Twenty five microliters of beads (working solution) and 180 μL of Ru labeled antibody (working solution) were dispensed. After 26 minutes of incubation at 30 +/-2°C, the beads were washed and suspended with 300 μL of electrolyte solution (Sanko, Tokyo, Japan). The washed beads were subsequently transferred to the electrode and electrochemiluminescence (ECL) emission was measured.

All ECL measurements were carried out in duplicate.

***Results***

Table 1 depicts the urine levels of FRα in individual subjects with ovarian cancer and non-afflicted female control subjects.

**Table 1: FRα levels in urine of subjects with ovarian cancer and normal female control subjects**

| Group | Sample # | FRα (pg/mL) |
|---|---|---|
| ovarian cancer | 1 | 27800 |
| | 2 | 40242 |
| | 3 | 85580 |
| | 4 | 4994 |
| | 5 | 2017 |
| | 6 | 3781 |
| | 7 | 29469 |
| | 8 | 47456 |
| | 9 | 4479 |
| | 10 | 11920 |
| | 11 | 18352 |
| | 12 | 162017 |
| | 13 | 30630 |
| | 14 | 14431 |
| | 15 | 11801 |
| | 16 | 13470 |
| | 17 | 11563 |
| | 18 | 22185 |

(continued)

| Group | Sample # | FRα (pg/mL) |
|---|---|---|
| | 19 | 52106 |
| normal control | 20 | 8491 |
| | 21 | 4885 |
| | 22 | 3595 |
| | 23 | 21301 |
| | 24 | 22757 |
| | 25 | 16578 |
| | 26 | 6081 |
| | 27 | 4195 |
| | 28 | 12169 |
| | 29 | 20639 |

Figure 2 depicts the distribution of FRα levels in urine in subjects with ovarian cancer and in normal female control subjects, as set forth in Table 1.

Table 2 summarizes the number of subjects (n), mean, standard deviation (SD), maximum (Max.) and minimum (Min.) values for the levels of FRα in the ovarian cancer group and the normal female control group.

**Table 2: Summary of urine FRα measurement**

| | FRα (pg/mL) | |
|---|---|---|
| | ovarian cancer | normal female |
| N | 19 | 10 |
| Mean | 31279 | 12069 |
| SD | 37895 | 7654 |
| Max. | 162017 | 22757 |
| Min. | 2017 | 3595 |

***Discussion***

A high level of FRα was detected in the urine of subjects with ovarian cancer. Moreover, the levels of FRα differed significantly between ovarian cancer and normal female control groups (p=0.03, one-sided).

**EXAMPLE 2. DILUTION LINEARITY - DETERMINATION OF FRα LEVELS IN SERIALLY DILUTED URINE SAMPLES MEASURED BY ELECTROCHEMILUMINESCENCE IMMUNOASSAY (ECLIA)**

Dilution Linearity is a measure of accuracy of an assay. Two urine samples were serially diluted by a factor of 10 and 100. The FRα levels of each sample were measured as set forth in Example 1 and compared to assess the percent error. Percent error was calculated as follows:

$$\frac{[[(\text{FR}\alpha\text{ in diluted sample})*(\text{dilution factor})] - (\text{FR}\alpha\text{ in undiluted sample})]*100}{(\text{FR}\alpha\text{ in undiluted sample})}$$

The results are set forth in Table 3:

**Table 3: Dilution Linearity for Urine**

| Sample | Dilution factor | FRα (pg/mL) | Error (%) |
|---|---|---|---|
| 1 | 1 | 25037 | - |
| | 10 | 2601 | 4 |
| | 100 | 279 | 11 |
| | | | |
| 2 | 1 | 16649 | - |
| | 10 | 1696 | 2 |
| | 100 | 173 | 4 |

The foregoing results demonstrate dilution linearity in assessing the levels of FRα in human urine samples and that, within acceptable errors, urine can be diluted up to a factor of at least 100 while retaining accurate levels of FRα. Accordingly, dilution of the urine samples may be considered prior to determining levels of FRα.

**EXAMPLE 3: CENTRIFUGATION OF URINE SAMPLES - ADDRESSING REPRODUCIBILITY**

The reproducibility of the ECLIA assay for a particular urine sample was also tested. For example, as reflected in Table 4, ECLIA assays of the same sample resulted in varying results.

**Table 4. Reproducibility without sample centrifugation**

| | ECL Counts | |
|---|---|---|
| Sample | **Test 1** | **Test 2** |
| 1 | 29380 | 15046 |
| 2 | 20912 | 17227 |

The presence of insoluble material (precipitates) in urine samples was hypothesized to be responsible for the variability seen in measuring the levels of FRα. As a result, centrifugation of samples in order to remove urine sediment, prior to measurement of FRα levels, was considered as an option to enhance the accuracy and reproducibility of the assay.

Table 5 depicts the results obtained when three samples were centrifuged prior to performance of the ECLIA assay.

**Table 5. Reproducibility with sample centrifugation**

| | | FRα concentration (ng/mL) | |
|---|---|---|---|
| **Test** | **Sample 1** | **Sample 2** | **Sample 3** |
| **1** | 10.4 | 9.2 | 13.3 |
| **2** | 10.5 | 8.9 | 14.0 |
| **3** | 10.3 | 9.2 | 13.4 |
| **Mean** | 10.4 | 9.1 | 13.6 |
| **SD** | 0.1 | 0.1 | 0.3 |
| **CV(%)** | 1.0 | 1.1 | 2.2 |

As set forth above, the results indicate that centrifugation provided more consistent measurements of FRα concentration.

Additionally, two samples were subjected to (i) centrifugation (at 2000 x g for 2 min) and the supernatant removed for measurement of FRα (depicted as sample "A" below in Table 6) and (ii) centrifugation followed by vortexing (depicted as sample "B" below in Table 6), prior to measurement of FRα levels by the ECLIA assay set forth in Example 1. The results are reflected in Table 6 below.

**Table 6: Effect of centrifugation on FRα levels in urine**

| Sample | Sediment after centrifugation | A/B | FRα (pg/mL) | Difference (%) |
|---|---|---|---|---|
| 1 | Yes (++) | A | 13678 | - |
| | | B | 16559 | 21 |
| 2 | Yes (+) | A | 12271 | - |
| | | B | 13206 | 8 |

Difference (%) was determined as follows:

$$\frac{[(\text{Level of FR}\alpha\text{ in “B”}) - (\text{Level of FR}\alpha\text{ in “A”})] *100}{(\text{Level of FR}\alpha\text{ in “A”})}$$

As shown in Table 6, the levels of FRα as determined by the ECLIA assay vary depending on whether urine was clarified by centrifugation to remove precipitates or whether urine was vortexed to suspend or disperse sediments. Accordingly, in certain embodiments centrifuging or vortexing of urine samples may be performed prior to determining levels of FRα.

**EXAMPLE 4. DETERMINATION OF FRα LEVELS IN CENTRIFUGED URINE SAMPLES FROM HUMAN SUBJECTS WITH AND WITHOUT OVARIAN CANCER MEASURED BY ELECTROCHEMILUMINESCENCE IMMUNOASSAY (ECLIA).**

Based on the results of Example 3, the assay for assessing FRα levels in subjects was modified to introduce a centrifugation step. FRα levels were determined on the same samples utilized in Example 1, including the group of subjects with ovarian cancer and the group of normal female control subjects.

***Materials and Methods***

The methodology utilized was as described in Example 1, except that the urine samples were centrifuged for 10000 x g for 1 minute and the resulting supernatant subsequently diluted by 1:51 in reaction buffer.

***Results***

Table 7 depicts the levels of FRα in centrifuged and non-centrifuged urine samples from subjects afflicted with ovarian cancer and healthy female control subjects.

**Table 7: Urine FRα1 level in ovarian cancer and normal control group**

| | | FRα (pg/mL) | | |
|---|---|---|---|---|
| Group | Sample # | Without centrifugation | With centrifugation | sediment after centrifugation |
| ovarian cancer | 1 | 27800 | 23960 | + |
| | 2 | 40242 | 37852 | + |
| | 3 | 85580 | 78976 | + |
| | 4 | 4994 | 3766 | + |
| | 5 | 2017 | 1512 | - |
| | 6 | 3781 | 3443 | - |
| | 7 | 29469 | 25728 | + |
| | 8 | 47456 | 16556 | + |
| | 9 | 4479 | 3357 | - |

(continued)

| | | FRα (pg/mL) | | |
|---|---|---|---|---|
| Group | Sample # | Without centrifugation | With centrifugation | sediment after centrifugation |
| | 10 | 11920 | 5020 | + |
| | 11 | 18352 | 16695 | - |
| | 12 | 162017 | 82705 | + |
| | 13 | 30630 | 4496 | + |
| | 14 | 14431 | 8786 | + |
| | 15 | 11801 | 10582 | - |
| | 16 | 13470 | 5611 | + |
| | 17 | 11563 | 5463 | + |
| | 18 | 22185 | 14443 | + |
| | 19 | 52106 | 38327 | - |
| normal control | 20 | 8491 | 6867 | + |
| | 21 | 4885 | 3754 | - |
| | 22 | 3595 | 3529 | - |
| | 23 | 21301 | 15047 | + |
| | 24 | 22757 | 4850 | + |
| | 25 | 16578 | 14366 | + |
| | 26 | 6081 | 5201 | - |
| | 27 | 4195 | 3135 | - |
| | 28 | 12169 | 499 | + |
| | 29 | 20639 | 2439 | + |

According to the results set forth in Table 7, centrifugation resulted in a decrease in the measurement of FRα levels in some samples, as previously demonstrated in Table 6.

**EXAMPLE 5: DETECTION OF FRα IN URINE SEDIMENT BY IMMUNOBLOTTING**

Based on the results shown in Examples 3 and 4, the presence or absence of FRα in urine sediment/precipitate was assessed using western blotting.

***Materials and Methods***

Urine samples from 2 ovarian cancer patients for whom FRα concentrations were measured at 18,747 pg/mL and 145,564 pg/mL, respectively (See Table 10 *supra*), were subjected to the following procedures. Control samples consisted of HeLa cell lysate 10 μg, liver tissue lysate 20 μg, and ovarian cancer tissue lysate 20 μg.

- 900 μL urine was centrifuged for 2 minutes at 10000g
- supernatant was removed
- the remaining pellet was dissolved in 15 μL of PAGE sample buffer (containing 292 mM LDS) and subsequently boiled at 70°C for 10 min
- The entire sample (approx. 20 μL) was loaded onto the NuPAGE bis-tris gel (Invitrogen)
- After electrophoresis, proteins were transferred to PVDF membrane
- 1% skim milk / 0.05% Tween 20 / PBS was added for blocking
- The membrane was washed with 0.05% Tween 20 / PBS
- 0.5 mL of monoclonal antibody 548908 (R&D Systems) at 2 μg/ml was added and incubated for 60 minutes at room temperature

- The membrane was washed with 0.05% Tween 20 / PBS
- 10 mL of anti-mouse IgG-HRP (DAKO p0447, 1:2000) was added and allowed to incubate for 60 minutes
- The membrane was washed with 0.05% Tween 20 / PBS
- Pierce ECL Substrate was added to the membrane
- The membrane was removed from the substrates and then imaged using the LAS-3000 (FUJIFILM) system

***Results***

The resulting immunoblot is shown in Figure 3. In this figure, lanes 1-5 correspond to FRα detected from the following sources:

(1) urine from ovarian cancer patient with a measured FRα level of 18,747 pg/mL
(2) urine from ovarian cancer patient with a measured FRα level of 145,564 pg/mL
(3) HeLa cell lysate: 10 μg
(4) Liver tissue lysate: 20 μg
(5) ovarian cancer tissue lysate: 20 μg

Lane 6 in the western blot represents molecular weight markers and demonstrates that the observed band in lanes 1, 2, 3 and 5 runs at the expected molecular weight for FRα.

Lanes 3 and 5 are positive control samples and lane 4 is a negative control sample. The faint band on lane 1 and the clear band on lane 2 demonstrate that FRα can be detected in the urine sediment of ovarian cancer patients by western blotting.

**EXAMPLE 6. DETERMINATION OF FRα LEVELS IN GUANIDINE-TREATED NORMAL HUMAN URINE SAMPLES BY ELECTROCHEMILUMINESCENCE IMMUNOASSAY (ECLIA).**

Based on the results of Example 4 in which centrifugation resulted in decreased FRα levels, and the results of Example 5 where the urine sediment obtained from centrifugation was shown to contain immunoreactive FRα, methods were sought to solubilize the sediments of urine to obtain more quantitative and accurate measurements of FRα.

In this regard, treatment of normal female urine samples with guanidine prior to assessing FRα levels was attempted.

The methodology utilized was as described in Example 1, except that the urine samples were mixed in a 1:1 ratio with either 6 M guanidine in buffer (PBS) or buffer alone. Subsequently, the urine samples were diluted by 1:51 in reaction buffer.

The results of this assay are shown in Table 8.

**Table 8: Normal urine FRα level with or without guanidine treatment**

| Sample | | Guanidine treatment | FRα (pg/mL) |
|---|---|---|---|
| | Std Ag | Yes | 83964 |
| | | No | 82512 |
| Normal Urine | 1 | Yes | 9431 |
| | | No | 7796 |
| | 2 | Yes | 5713 |
| | | No | 4066 |
| | 3 | Yes | 9687 |
| | | No | 9428 |

The results of this experiment indicate that guanidine does not interfere with FRα measurements. As can be seen for the pure antigen control (Std Ag), this methodology of guanidine treatment and subsequent dilution has no effect on the measurement of FRα. Further, it will be noted that in all three (3) urine samples assessed, the levels of FRα were higher in the samples treated (solubilized) with guanidine relative to the samples not treated with guanidine.

The reliability of guanidine pre-treatment of urine samples was further assessed by exposing three samples to guanidine and measuring the FRα concentration of each guanidine treated sample 3 times using the ECLIA assay. The results are reflected in Table 9 below:

**Table 9: Intra-assay reproducibility of guanidine treated urine**

| | FRα (pg/mL) | | |
|---|---|---|---|
| **Test** | **Sample 1** | **Sample 2** | **Sample 3** |
| **1** | 9210 | 5477 | 9889 |
| **2** | 9638 | 5405 | 10047 |
| **3** | 10192 | 5812 | 10944 |
| **Mean** | 9680 | 5565 | 10293 |
| **SD** | 492 | 217 | 569 |
| **CV(%)** | 5.1 | 3.9 | 5.5 |

As set forth above, the results indicate that guanidine treatment of urine prior to FRα assay provided consistent measurements of FRα concentration with very low CV's.

**EXAMPLE 7. DETERMINATION OF FRα LEVELS IN GUANIDINE-TREATED URINE SAMPLES FROM HUMAN SUBJECTS WITH AND WITHOUT OVARIAN CANCER MEASURED BY ELECTROCHEMILUMINESCENCE IMMUNOASSAY (ECLIA).**

Based on the results of Example 6 in which guanidine treatment was shown not to interfere with FRα assays, a modified assay protocol was employed to measure FRα in the urine samples from the subjects with and without ovarian cancer in Example 1.

The following assay protocol was employed:

***Materials and Methods***

The methodology utilized was as described in Example 1, except that the urine samples were mixed in a 1:1 ratio with a 6 M guanidine buffer and subsequently diluted by 1:26 in reaction buffer.

***Results***

Table 10 depicts the levels of FRα in guanidine treated urine samples from subjects afflicted with ovarian cancer and healthy female control subjects.

**Table 10: Urine FRα level in ovarian cancer and normal control group**

| Group | Sample # | FRα (pg/mL) |
|---|---|---|
| ovarian cancer | 1 | 27015 |
| | 2 | 37315 |
| | 3 | 79579 |
| | 4 | 285 |
| | 5 | 1864 |
| | 6 | 2902 |
| | 7 | 27914 |
| | 8 | 51864 |
| | 9 | 2699 |
| | 10 | 9455 |
| | 11 | 18396 |
| | 12 | 145564 |
| | 13 | 19046 |
| | 14 | 10440 |
| | 15 | 10977 |
| | 16 | 9199 |
| | 17 | 18223 |
| | 18 | 18747 |
| | 19 | 51098 |
| normal control | 20 | 8012 |
| | 21 | 3797 |
| | 22 | 3323 |
| | 23 | 20976 |
| | 24 | 6941 |
| | 25 | 14512 |
| | 26 | 7286 |
| | 27 | 2789 |
| | 28 | 2617 |
| | 29 | 7233 |

Figure 4 shows the distribution of FRα levels in the urine of ovarian cancer afflicted subjects and normal female control subjects using the modified protocol with guanidine treatment. A statistically significant difference between groups was observed. Table 11 summarizes these results.

**Table 11: Summary of urine FRα measurement**

| | FRα (pg/mL) | |
|---|---|---|
| | ovarian cancer | normal control |
| n | 19 | 10 |
| Mean | 28557 | 7749 |
| SD | 34990 | 5850 |

(continued)

| | FRα (pg/mL) | |
|---|---|---|
| | ovarian cancer | normal control |
| Max. | 145564 | 20976 |
| Min. | 285 | 2617 |

Using the data from this experiment, a receiver operating characteristic (ROC) analysis was performed. Figure 5 shows an ROC curve of the sensitivity and specificity of the ECLIA measurement of FRα levels in urine after the urine was treated with guanidine. AUC is the area under the curve, which measures the accuracy of the test in separating ovarian cancer from control subjects.

Using an arbitrary cutoff value of 9100 pg FRα/mL, the AUC was 0.70 with a positive predictive value of 70% and a negative predictive value of 80%, as shown in Table 12. Using this cutoff value, 15/19 ovarian cancer patients had a concentration of FRα above 9100 pg/mL and 8/10 normal subjects had a concentration of FRα less than 9100 pg/mL.

**Table 12: Guanidine treatment for urine measurement**

| | ovarian cancer | control |
|---|---|---|
| Number of Samples | 19 | 10 |
| Positive | 15 | 2 |
| Predictive value (%) | 78.9 | 80.0 |

**EXAMPLE 8: CREATININE CORRECTION OF FRα CONCENTRATIONS DETERMINED IN GUANIDINE-TREATED URINE SAMPLES BY ELECTROCHEMILUMINESCENCE IMMUNOASSAY (ECLIA)**

Concentrations of FRα were previously determined using ECLIA of guanidine-treated urine samples from ovarian cancer patients and normal female controls (See Example 7, Table 10). Here, these FRα concentrations were corrected for urine creatinine levels in order to normalize for the glomerular filtration rate. The resulting values were subjected to an ROC analysis.

***Methods***

The urine creatinine level was determined by the Ministry of Health, Labour and Welfare approved test kit, determiner L CRE (Kyowa Medex, Japan). The corrected value for urine FRα concentration was calculated as follows:

$$\text{Urine FR}\alpha\text{ Creatinine correction (ng/g)}$$
$$= (\text{Urine FR}\alpha\text{ (ng/L) x 1000}) / (\text{Urine Creatinine (mg/dL) x10})$$
$$= (\text{Urine FR}\alpha\text{ (ng/L) x 1000}) / \text{Urine Creatinine (mg/L)}$$
$$= \text{Urine FR}\alpha\text{ (ng/L)} / \text{Urine Creatinine (g/L)}$$
$$= \text{Urine FR}\alpha\text{ (ng)} / \text{Urine Creatinine (g)}$$

or

$$= 1/1000\text{ x Urine FR}\alpha\text{ }(\mu\text{g}) / \text{Urine Creatinine (g)}$$

*Results*

Table 13 presents the resulting creatinine-corrected FRα levels.

**Table 13: Creatinine-corrected FRα levels determined using ECLIA of guanidine-treated urine samples**

| Group | Sample # | FRα (pg/mL) | Corrected FRα (μg FRα/ g creatinine) |
|---|---|---|---|
| ovarian cancer | 1 | 27015 | 11.6 |
| | 2 | 37315 | 37.8 |
| | 3 | 79579 | 33.9 |
| | 4 | 285 | 0.6 |
| | 5 | 1864 | 6.7 |
| | 6 | 2902 | 7.1 |
| | 7 | 27914 | 54.9 |
| | 8 | 51864 | 17.1 |
| | 9 | 2699 | 13.5 |
| | 9 | 9455 | 14.5 |
| | 10 | 18396 | 23.1 |
| | 11 | 145564 | 66.0 |
| | 12 | 19046 | 9.1 |
| | 13 | 10440 | 8.5 |
| | 14 | 10977 | 7.4 |
| | 15 | 9199 | 5.9 |
| | 16 | 18223 | 13.0 |
| | 17 | 18747 | 9.6 |
| normal control | 18 | 3797 | 7.7 |
| | 19 | 3323 | 7.9 |
| | 20 | 20976 | 10.7 |
| | 21 | 6941 | 4.3 |
| | 22 | 14512 | 8.6 |
| | 23 | 7286 | 13.8 |
| | 24 | 2789 | 4.3 |
| | 25 | 2617 | 1.9 |
| | 26 | 7233 | 3.1 |
| | | | |

Figure 6 shows the distribution of FRα levels in ovarian cancer (OC) and normal female control subjects after correction for urine creatinine levels. There is a statistically significant difference between ovarian cancer patients and controls in creatinine-corrected levels of FRα ($p=0.007$).

The summary data for ovarian cancer and normal control subjects are provided in Table 14.

**Table 14: Summary statistics for creatinine-corrected FRα levels**

| | FRα (μg/g-creatinine) | |
|---|---|---|
| | **ovarian cancer** | **normal control** |
| **n** | 18 | 9 |
| **Mean** | 18.9 | 6.9 |
| **SD** | 17.9 | 3.9 |

(continued)

| | FRα (μg/g-creatinine) | |
|---|---|---|
| | **ovarian cancer** | **normal control** |
| **Max.** | 66.0 | 13.8 |
| **Min.** | 0.6 | 1.9 |

The creatinine-corrected FRα levels were further subjected to an ROC analysis. The ROC curve is shown in Figure 7. Table 15 presents the sensitivity, specificity, and area under the curve (AUC) for various cutoff values of the creatinine-corrected test.

**Table 15: Sensitivity, specificity, and AUC for various cutoff values of the creatinine-corrected FRα test**

| Cut-off | Sensitivity | Specificity | AUC |
|---|---|---|---|
| 3.0 | 94.4% | 11.1% | 0.67 |
| 4.0 | 94.4% | 22.2% | 0.70 |
| 5.0 | 94.4% | 44.4% | 0.78 |
| 6.0 | 88.9% | 44.4% | 0.74 |
| 9.0 | 66.7% | 77.8% | 0.70 |

As previously noted, there is a clear discrimination between urines of ovarian cancer patients and those from healthy female control subjects.

**EXAMPLE 9: ENZYME IMMUNOASSAY (EIA) AND OPTIMIZATION THEREOF**

**1. Enzyme Immunoassay (EIA)**

**Antibody coating to microtiter plates**

The monoclonal anti-folate receptor alpha antibody was coated on the surface of microtiter plates (Nunc-immunoplate, Thermo Scientific) as follows. One hundred microliter of antibody (absorbance 0.02 at 280 nm) in 50 mmol/L carbonate buffer pH 9.4 was dispensed into wells, followed by coating for 16 hours at 4°C. The microplates were then washed 2 times with PBS containing 0.05% Tween20 (PBS-T). Thereafter 0.15 mL of PBS containing 20% normal rabbit serum pH 7.8 was dispensed into wells to block the unbounded surface, followed by blocking for 1 hour at room temperature. Finally, the microplates were washed 2 times with PBS-T. The antibody coated plates were dried and kept at 4°C in aluminum bags until use.

**Biotin labeling**

Biotin labeling was conducted according to the manufacturers recommendations for the EZ-Link Sulfo-NHS-LC-LC-Biotin (Product No. 21338, Thermo Scientific). Briefly, 1 mg of antibody in 0.4 mL of PBS was mixed with 0.013 mL of 10 mM Sulfo-NHS-LC-LC-Biotin, with an initial molar ratio of antibody to biotin of 1:20, followed by incubation for 30 min at room temperature. The biotin coupled antibody was purified by gel filtration using a PD-10 column (GE Healthcare) eluted with PBS to remove non-reacted biotin. In order to determine the level of biotin incorporation, the EZ Biotin quantitation kit (Product No. 28005, Thermo Scientific) was used. The biotin labeled antibody was stored at -80°C until use.

**Two step immunoassay**

For the first reaction, 40 μL of plasma or standard antigen and 60 μL of 50 mM HEPES buffer containing 10% NRS, 150 mmol/L NaCl, 10 mmol/L EDTA-2Na, 0.01% Tween 20 pH 7.5 (reaction buffer) was dispensed into antibody coated wells. The plate was incubated for 18 hours at 4°C, and subsequently washed 5 times with PBS-T. For the second reaction, 100 μL of 10 μg/mL biotin labeled antibody in reaction buffer was dispensed. The plate was incubated for 1 hour at room temperature, and subsequently washed 5 times with PBS-T. 100 μL of horse radish peroxidase labeled streptavidin (Pierce) was dispensed. After 30 minutes incubation at room temperature, the plates were washed 5 times with PBS-T. Finally, for the color development, 100 μL of TMB solution (KPL) was dispensed and left for 15 minutes in dark. After stopping color development by adding 100 μL of 1N HCl, the absorption at 450 nm was read using a plate reader. All washing steps were automatically done by auto-plate washer (AMW-8, BioTec, Japan), and all EIA measurements were carried out in duplicate.

Figure 8 depicts the EIA assay using MOV18 as the capture antibody and biotinylated MORAb-003 as the detector antibody.

**2. Optimization of EIA procedures**

The above EIA procedures were arrived at in part based on the following experiments designed to optimize the procedure.

First, avidin-HRP, biotin labeled antibody and HRP labeled antibody were compared. Compared with HRP labeled antibody, biotin labeled antibody and avidin-HRP provided a higher signal; therefore, biotin labeled antibody and avidin-HRP were employed.

Second, one and two-step incubation procedures were compared. As depicted in Figure 9, a two-step incubation procedure yielded a higher signal and was thus employed.

Third, to optimize the second incubation time, incubation times of one to four hours were compared. The results indicated that one hour incubation times provided the highest signal to noise ratio and therefore an incubation time of one hour was subsequently employed.

Fourth, in order to optimize the working concentration of biotin labeled antibody, HRP labeled antibody and sample volume, various concentrations were employed as set forth in the above description of the EIA assay. The optimal values concentrations are described above.

**EXAMPLE 10: COMPARISON OF FRα IN HUMAN PLASMA USING ELECTROCHEMILUMINESCENCE IMMUNOASSAY (ECLIA) AND ENZYME IMMUNOASSAY (EIA)**

The levels of FRα were measured in human plasma samples taken from ovarian cancer patients and healthy female controls using the electrochemiluminescence assay (ECLIA) described in Example 1 and Figure 1 (using MORAb-003 as the capture antibody and ruthenium (Ru)-labeled MOV-18 as the labeled detector antibody) and the enzyme immunoassay (EIA) described in Example 9 and Figure 8. In both assays, 40 μL of plasma was assayed.

Table 16 shows the plasma levels of FRα in ovarian cancer and normal control subjects, as determined using the EIA and ECLIA.

**Table 16: Plasma concentrations of FRα determined using EIA and ECLIA methods**

| Group | Sample # | FRα concentration (pg/mL) | |
|---|---|---|---|
| | | EIA | ECLIA |
| Ovarian cancer | 1 | 10 | 73 |
| | 2 | <10 | 200 |
| | 3 | 56 | 286 |
| | 4 | 44 | 286 |
| | 5 | 353 | 1606 |
| | 6 | 83 | 494 |
| Healthy control | 7 | 110 | 127 |
| | 8 | 162 | 112 |
| | 9 | 88 | 252 |
| | 10 | 180 | 254 |
| | 11 | 262 | 471 |
| | 12 | 206 | 396 |

With only one exception, the results for all of the subjects indicated that the concentrations of FRα detected in serum using EIA are lower than the levels detected using ECLIA, demonstrating that the EIA assay, as formatted, is not as sensitive as the ECLIA assay when this particular combination of capture (MOV-18) and detector antibodies (MORAb-003) is used. Therefore, further experiments with other types of antibodies were conducted to develop a more sensitive EIA procedure.

## EXAMPLE 11: FEASIBILITY OF DIFFERENT TYPES OF ANTIBODIES FOR EIA MEASUREMENT OF FRα IN HUMAN PLASMA

### 1. Preliminary experimentation of Antibody Combinations

Various combinations of capture/detector antibodies were considered. Preliminary experimentation rendered the results set forth in Table 17.

**Table 17**

| | | Capture antibody | | | | | | | |
|---|---|---|---|---|---|---|---|---|---|
| | | MORAB-003 | | MOV18 | | 548908(R&D) | | 6D398 | |
| Biotin-labeled detection antibody | 003 | Blank<br>Std.<br>1-S | Low<br>-<br>- | Blank<br>Std.<br>1-S | Low<br>+++<br>++ | Blank<br>Std.<br>1-S | Low<br>++<br>++ | Blank<br>Std.<br>1-S | Low<br>++<br>+ |
| | MOV18 | Blank<br>Std.<br>1-S | Low<br>+<br>++ | Blank<br>Std.<br>1-S | High<br>-<br>+++ | Blank<br>Std.<br>1-S | High<br>-<br>++ | Blank<br>Std.<br>1-S | High<br>-<br>+++ |
| | 548908 | | | | | | | Blank<br>Std.<br>1-S | Low<br>-<br>++ |

### 2. Comparison of EIA assays using various antibody combinations and comparison to the ECLIA assay

The levels of FRα in plasma from ovarian cancer patients and normal healthy female controls were measured using an enzyme-linked immunosorbent assay (EIA) with different combinations of capture and biotin-labeled antibodies and compared with the levels of FRα measured using the ECLIA assay.

*Materials and Methods*

The ECLIA method was as described in Example 1 and depicted in Figure 1 (using the MORAb-003 antibody as the capture antibody and the Mov-18 antibody as the labeled detector antibody). The EIA method was as described in Example 9, except that three different combinations of capture/detector antibodies were employed, as depicted in Figure 10: MOV18/MORAb-003, 548908/MORAb-003 and 6D398/MORAb-003. The antibodies 548908 and 6D398 are commercially available. The 548908 antibody was obtained from R&D Systems (North Las Vegas, NV) and the 6D398 antibody was obtained from US Biological (Swampscott, MA 01907).

*Results*

The concentrations of FRα (pg/mL) determined using the EIA and ECLIA methods are shown in Table 18. In addition, the concentrations of FRα (pg/ml) determined by EIA using various combinations of capture/detector antibodies are depicted graphically in Figure 11.

**Table 18: Plasma concentrations of FRα (pg/mL) determined using the EIA and ECLIA methods with various combinations of capture and detector antibodies.**

| Group | Sample # | **EIA** 548908-MORAb-003 | **EIA** 6D398-MORAb-003 | **EIA** MOV18-MORAb-003 | **ECLIA** MOV18-MORAb-003 |
|---|---|---|---|---|---|
| **Ovarian cancer** | 1 | 176 | 2 | 10 | 217 |
| | 2 | 85 | <0 | - | 165 |
| | 3 | 257 | 35 | - | 296 |
| | 4 | 117 | 42 | 44 | 322 |
| | 5 | 2048 | 370 | 353 | 1335 |
| | 6 | 447 | 63 | 83 | 390 |
| **Normal control** | 7 | 247 | 66 | 110 | 137 |
| | 8 | 213 | 110 | 162 | 185 |
| | 9 | 367 | 78 | 88 | 219 |
| | 10 | 364 | 152 | 180 | 228 |
| | 11 | 804 | 224 | 262 | 388 |
| | 12 | 473 | 194 | 206 | 318 |

The data in Table 18 indicate that the measurements of FRα levels with EIA using the 54908-MORAb-003 combination yields results that are most similar to the results obtained using the ECLIA assay. Quantitive analyses were performed, confirming this observation. Further, these data demonstrate that the detection of FRα is highly dependent on the antibodies and antibody combination employed. Accordingly, different antibody combinations can be employed for the determination of FRα in biological fluids. In addition, since the data obtained from the EIA and the ECLIA assay formats are similar, various assay formats can be used for the determination of FRα.

For each of the three combinations of capture and detector antibodies used for the EIA method, a regression analysis was performed, and the concentrations of FRα (pg/mL) determined with EIA were correlated with the concentrations determined with the ECLIA assay. The results of this analysis are shown in Table 19 and in Figure 12.

**Table 19: Correlations of plasma concentrations of FRα measured by ECLIA with concentrations measured by EIA using three combinations of capture and detector antibodies**

| Capture antibody-detector antibody | 548098-MORAb-003 | 6D398-MORAb-003 | MOV18-MORAB-003 |
|---|---|---|---|
| r | 0.960 | 0.781 | 0.715 |
| Slope | 1.595 | 0.285 | 0.223 |
| Intercept | -87.06 | -0.64 | 58.62 |

The results for EIA using the 548098-MORAb-003 capture-detector combination correlated highly (r=0.96) with the results for ECLIA.

**EXAMPLE 12: PLASMA LEVELS OF FRα DETERMINED BY EIA AND ECLIA IN SAMPLES FROM OVARIAN CANCER PATIENTS**

Measurements of serum FRα levels were determined in a group of ovarian cancer patients (n=17) and normal controls (n=35) using ECLIA and EIA. For the EIA measurements, the 548908 capture/ MORAb-003 detector antibody combination was employed. The EIA procedure was otherwise as described in Example 9. The ECLIA procedure was as described in Example 1. The results are shown in Table 20.

**Table 20: Plasma FRα concentrations in ovarian cancer patients and normal controls, as determined using EIA and ECLIA**

| Group | Sample # | EIA | ECLIA |
|---|---|---|---|
| | | pg/mL | pg/mL |
| Ovarian cancer | 1 | 245 | 217 |
| | 2 | 247 | 223 |
| | 3 | 194 | 229 |
| | 4 | 2613 | 1335 |
| | 5 | 154 | 153 |
| | 6 | 319 | 215 |
| | 7 | 516 | 390 |
| | 8 | 370 | 271 |
| | 9 | 933 | 449 |
| | 10 | 4768 | 4502 |
| | 11 | 385 | 266 |
| | 12 | 251 | 322 |
| | 13 | 404 | 349 |
| | 14 | 338 | 371 |
| | 15 | 4147 | 2344 |

(continued)

| Group | Sample # | EIA | ECLIA |
|---|---|---|---|
| | | pg/mL | pg/mL |
| | 16 | 179 | 165 |
| | 17 | 380 | 296 |
| **Control** | 18 | 232 | 181 |
| | 19 | 372 | 173 |
| | 20 | 332 | 189 |
| | 21 | 380 | 203 |
| | 22 | 376 | 290 |
| | 23 | 406 | 217 |
| | 24 | 281 | 182 |
| | 25 | 348 | 191 |
| | 26 | 490 | 247 |
| | 27 | 253 | 137 |
| | 28 | 368 | 185 |
| | 29 | 338 | 195 |
| | 30 | 289 | 219 |
| | 31 | 338 | 206 |
| | 32 | 406 | 226 |
| | 33 | 365 | 228 |
| | 34 | 501 | 280 |
| | 35 | 806 | 388 |
| | 36 | 613 | 286 |
| | 37 | 380 | 250 |
| | 38 | 420 | 281 |
| | 39 | 393 | 280 |
| | 40 | 552 | 284 |
| | 41 | 664 | 318 |
| | 42 | 429 | 261 |
| | 43 | 499 | 286 |
| | 44 | 310 | 218 |
| | 45 | 281 | 217 |
| | 46 | 215 | 202 |
| | 47 | 293 | 217 |
| | 48 | 380 | 256 |
| | 49 | 270 | 195 |
| | 50 | 393 | 234 |
| | 51 | 425 | 308 |
| | 52 | 226 | 199 |

(continued)

| Group | Sample # | EIA | ECLIA |
|---|---|---|---|
| | | pg/mL | pg/mL |
| | | | |

Figure 13 shows the distribution of plasma FRα concentrations in subjects with ovarian cancer and normal female control subjects as determined using EIA.

Table 21 shows summary descriptive statistics for the plasma FRα concentrations in ovarian cancer and normal female control subjects as determined using EIA.

**Table 21: Summary of FRα plasma concentrations in ovarian cancer and normal female control subjects as determined using EIA.**

| | FRα (pg/mL) | |
|---|---|---|
| | **Ovarian cancer** | **Normal control** |
| **n** | **17** | **35** |
| **Mean** | **967** | **389** |
| **SD** | **1438** | **126** |
| **Max.** | **4768** | **806** |
| **Min.** | **154** | **215** |

Figure 14 further depicts the correlation between FRα plasma concentrations determined using EIA and ECLIA. The correlation is high (r=0.95).

**EXAMPLE 13. DETERMINATION OF FRα LEVELS IN MATCHED URINE AND SERUM SAMPLES FROM LUNG CANCER PATIENTS AND OVARIAN CANCER PATIENTS AS MEASURED BY ELECTROCHEMILUMINESCENCE IMMUNOASSAY (ECLIA)**

FRα levels were determined in matched urine and serum samples from lung cancer and ovarian cancer patients using ECLIA where the samples were taken from the same patient. The correlation between serum and urine FRα levels was also determined.

***Materials and Methods***

The ECLIA methodology utilized is as described in Example 1. Guanidine was used to solubilize urine sediments as described in Example 6.

***Results***

The results of the ECLIA assays of serum and urine from lung cancer and ovarian cancer patients are presented in Table 22.

**Table 22: FRα concentrations in matched urine and serum samples of lung cancer patients and ovarian cancer patients, as determined by ECLIA**

| | | Serum | Urine |
|---|---|---|---|
| **Group** | **set ID** | **pg/mL** | **pg/mL** |
| **Lung cancer** | 1 | 146 | 2009 |
| | 2 | 153 | 4496 |
| | 3 | 206 | - |
| | 4 | 70 | 3562 |

(continued)

| | | Serum | Urine |
|---|---|---|---|
| Group | set ID | pg/mL | pg/mL |
| | 5 | 195 | 12381 |
| | 6 | 352 | 21873 |
| | 7 | 198 | 11296 |
| | 8 | 120 | 18570 |
| | 9 | 275 | 4455 |
| | 10 | 163 | 8662 |
| | 11 | 145 | 5294 |
| | 12 | 178 | - |
| | 13 | 165 | 1106 |
| | 14 | 187 | 7446 |
| | 15 | 168 | 11167 |
| | 16 | 217 | 24448 |
| | 17 | 142 | 6724 |
| | 18 | 177 | 14514 |
| | 19 | 236 | 822 |
| | 20 | 101 | 4826 |
| | 21 | 145 | 7723 |
| | 22 | 213 | 9887 |
| | 23 | 143 | 7422 |
| | 24 | 253 | 3376 |
| | 25 | 421 | 8045 |
| **ovarian cancer** | 26 | 282 | 9414 |
| | 27 | 1605 | 7651 |
| | 28 | 240 | 13059 |
| | 29 | 695 | 10549 |

Summary data for serum and urine FRα levels of lung cancer patients is presented in Table 23.

**Table 23: Summary statistics for FRα concentrations in matched serum and urine samples of lung cancer patients, as determined by ECLIA**

| Lung cancer | FRα (pg/mL) | |
|---|---|---|
| | Serum | Urine |
| **n** | 25 | 23 |
| **Mean** | 191 | 8700 |
| **SD** | 75 | 6291 |
| **Max.** | 421 | 24448 |
| **Min.** | 70 | 822 |

Summary data for serum and urine FRα levels of ovarian cancer patients is presented in Table 24.

**Table 24: Summary statistics for FRα concentrations in matched serum and urine samples of ovarian cancer patients, as determined by ECLIA**

| Ovarian cancer | FRα (pg/mL) | |
|---|---|---|
| | Serum | Urine |
| n | 4 | 4 |
| Mean | 705 | 10168 |
| SD | 634 | 2266 |
| Max. | 1605 | 13059 |
| Min. | 240 | 7651 |

Figure 15 shows correlations between ECLIA measures of FRα levels in matched serum and urine samples taken from the same patient. The correlation for lung cancer patients was r=0.24 (upper panel) and the correlation for ovarian cancer patients was r=-0.76 (lower panel).

These data demonstrate the relative lack of correlation between FRα concentrations measured in urine versus serum, especially as shown for lung cancer patients. Further, these data demonstrate that FRα is basically non-detectable above background levels in the serum of lung cancer patients versus normal controls whereas FRα is detectable in the urine of these patients.

## EXAMPLE 14. ASSESSMENT OF LEVELS OF FRα IN SERUM SAMPLES FROM PATIENTS WITH OVARIAN CANCER, PATIENTS WITH LUNG CANCER, AND NORMAL CONTROLS

FRα levels in the serum of patients with ovarian cancer, patients with lung cancer, and normal controls were assessed. Serum FRα levels were assessed using ECLIA with two different pairs of capture-detector antibodies: **Pair 1,** in which 9F3 was the capture antibody and 24F12 was the detector antibody, and **Pair 2,** in which 26B3 was the capture antibody and 19D4 was the detector antibody.

The FRα pairs were tested with full calibrator curves and 196 individual serums diluted 1:4. In one experiment, 26B3 was used as the capture antibody after CR processing on a plate lot (75 μg/mL, +B, +T) and 19D4 was used as the detector antibody at 1.0μg/mL. In another experiment, 9F3 was used as the capture antibody and 24F12 was used as the detector antibody at 1.0μg/mL. Each were CR processed (lot 10070) with an label to protein ratio (L/P) of 13.3. Diluent 100 (Meso Scale Discovery, Gaithersburg, Maryland) + human anti-mouse antibody (HAMA) +mIgG was used for samples and calibrator. Diluent 3 (Meso Scale Discovery, Gaithersburg, Maryland) was used for detections.

The following protocol was employed for the ECLIA. Samples were added at 50μL/well. The samples were shaken for 2 hours and subsequently washed with Phosphate Buffered Saline (PBS) solution with the detergent Tween 20 (PBST). The detector antibody was added at 25μL/well. The samples were shaken for 2 hours and then were washed with PBST. Finally, the electrochemiluminescence (ECL) emission of the samples was read with 2X MSD® Buffer T.

The results are shown in Table 25 below.

**Table 25: FRα levels in serum of patients with ovarian cancer, patients with lung cancer, and normal controls**

| | | LLOQ[1] = 1pg/mL | | LLOQ[1] = 5 pg/mL | | | | | |
|---|---|---|---|---|---|---|---|---|---|
| | | **FRα - Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **1** | **Ovarian Serum** | 3760 | 4% | 3585 | 4% | III | 2 | F | Adenocarcinoma-Ovary |
| **2** | **Ovarian Serum** | 223 | 3% | 273 | 2% | III | 3 | F | Adenocarcinoma-Ovary |
| **3** | **Ovarian Serum** | 950 | 1% | 3346 | 8% | IIIC | | F | Papillary Serous Carcinoma |
| **4** | **Ovarian Serum** | 3827 | 4% | 968 | 0% | III | 2 | F | Adenocarcinoma-Ovary |
| **5** | **Ovarian Serum** | 251 | 6% | 468 | 2% | IV | 2 | F | Adenocarcinoma-Ovary |
| **6** | **Ovarian Serum** | 199 | 6% | 328 | 1% | IIIC | 2 | F | Cystadenocarcinoma |
| **7** | **Ovarian Serum** | 166 | 1% | 257 | 5% | IC | 2 | F | Cystadenocarcinoma |
| **8** | **Ovarian Serum** | 182 | 4% | 248 | 2% | IIIC | 2 | F | Cystadenocarcinoma |
| **9** | **Ovarian Serum** | 155 | 6% | 265 | 2% | IIIC | 2 | F | Cystadenocarcinoma |
| **10** | **Ovarian Serum** | 145 | 9% | 253 | 5% | IIIC | 2 | F | Cystadenocarcinoma |
| **11** | **Ovarian Serum** | 142 | 5% | 186 | 1% | IIB | 1 | F | Serous adenocarcinoma |
| **12** | **Ovarian Serum** | 299 | 5% | 456 | 2% | IB | 3 | F | Serous adenocarcinoma |
| **13** | **Ovarian Serum** | 315 | 0% | 768 | 8% | IIIB | high grade | F | Serous cystadenocarcinoma of the ovary |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **14** | **Ovarian Serum** | 168 | 6% | 351 | 1% | I | high grade | F | Serous cystadenocarcinoma of the ovary |
| **15** | **Ovarian Serum** | 187 | 8% | 263 | 1% | I | Well to moderately differentiated | F | Papillary serous cystadenocarcinoma of the ovary |
| **16** | **Ovarian Serum** | 423 | 3% | 229 | 5% | IA | poorly differentiated | F | Mucinous cystadenocarcinoma of the ovary |
| **17** | **Ovarian Serum** | 86 | 7% | 289 | 1% | I | moderately differentiated | F | Papillary cystadenocarcinoma of the ovary |
| **18** | **Ovarian Serum** | 57 | 3% | 255 | 1% | I | well differentiated | F | Mucinous cystadenocarcinoma of the ovary |
| **19** | **Ovarian Serum** | 108 | 1% | 393 | 1% | III | moderately differentiated | F | Papillary mucinous cystadenocarcinoma of the ovary |
| **20** | **Ovarian Serum** | 207 | 3% | 328 | 1% | missing | poorly differentiated | F | Papillary cystadenocarcinoma of the ovary |
| **21** | **Ovarian Serum** | 66 | 0% | 254 | 1% | missing | high grade | F | Adenocarcinoma of the ovary |
| **22** | **Ovarian Serum** | 108 | 2% | 197 | 2% | III | low grade | F | Papillary cystadenocarcinoma of the ovary |
| **23** | **Ovarian Serum** | 201 | 2% | 457 | 1% | II | high grade | F | Papillary serous cystadenocarcinoma of the ovary |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **24** | **Ovarian Serum** | 477 | 1% | 808 | 11% | III | high grade | F | Serous cystadenocarcinoma of the ovary |
| **25** | **Ovarian Serum** | 423 | 3% | 613 | 3% | n/a | n/a | F | transitional cell carcinoma |
| **26** | **Ovarian Serum** | 249 | 8% | 432 | 1% | n/a | 2 | F | ovarian carcinoma - endometrioid type |
| **27** | **Ovarian Serum** | 152 | 7% | 235 | 2% | IV | 2 | F | ovarian carcinoma - serous papillary type |
| **28** | **Ovarian Serum** | 1409 | 5% | 1287 | 3% | III C | 3 | F | ovarian carcinoma - serous type |
| **29** | **Ovarian Serum** | 443 | 2% | 547 | 2% | III C | 2 | F | ovarian carcinoma - serous papillary type |
| **30** | **Ovarian Serum** | 208 | 1% | 298 | 13% | IA | 1 | F | ovarian carcinoma - serous type |
| **31** | **Ovarian Serum** | 114 | 9% | 344 | 1% | III B | 2 | F | ovarian carcinoma - serous papillary type |
| **32** | **Ovarian Serum** | 223 | 4% | 157 | 133% | III C | 1 | F | ovarian carcinoma - serous papillary type |
| **33** | **Ovarian Serum** | 5034 | 1% | 4405 | 1% | III C | 3 | F | transitional cell carcinoma |
| **34** | **Ovarian Serum** | 32966 | 6% | 23228 | 4% | IV | n/a | F | ovarian carcinoma - serous papillary type |
| **35** | **Ovarian Serum** | 94 | 6% | 188 | 3% | n/a | 3 | F | clear cell adenocarcinoma |
| **36** | **Ovarian Serum** | 866 | 6% | 1317 | 1% | III C | 3 | F | poorly differentiated adenocarcinoma |

(continued)

| | | LLOQ[1] = 1pg/mL | | LLOQ[1] = 5 pg/mL | | | | | |
|---|---|---|---|---|---|---|---|---|---|
| | | FRα- Pair 2 | | FRα Pair 1 | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **37** | **Ovarian Serum** | 2916 | 8% | 3121 | 0% | IV | 3 | F | ovarian carcinoma - serous papillary type |
| **38** | **Ovarian Serum** | 679 | 4% | 1037 | 17% | IV | 3 | F | ovarian carcinoma - serous type |
| **39** | **Ovarian Serum** | 294 | 8% | 478 | 3% | III C | 3 | F | ovarian carcinoma - serous papillary type |
| **40** | **Ovarian Serum** | 2037 | 4% | 12 | 74% | III C | 3 | F | ovarian carcinoma - serous type |
| **41** | **Ovarian Serum** | 16289 | 6% | 10431 | 3% | III C | 2 | F | ovarian carcinoma - serous papillary type |
| **42** | **Ovarian Serum** | 386 | 6% | 736 | 6% | n/a | 3 | F | ovarian carcinoma - serous papillary type |
| **43** | **Ovarian Serum** | 1474 | 6% | 2382 | 3% | IIIC | | F | Papillary serous cystadenocarcinoma of the pelvic |
| **44** | **Ovarian Serum** | 169 | 2% | 438 | 1% | I | 3 | F | Adenocarcinoma of the ovary |
| **45** | **Ovarian Serum** | 257 | 4% | 635 | 10% | I | high grade | F | Adenocarcinoma of the ovary |
| **46** | **Ovarian Serum** | 184 | 6% | 393 | 0% | IIIC | poorly differentiated | F | Adenocarcinoma of the ovary |
| **47** | **Ovarian Serum** | 251 | 6% | 659 | 1% | IIA | 2 | F | Adenocarcinoma of the ovary |
| **48** | **Ovarian Serum** | 165 | 2% | 394 | 7% | I | 2 | F | Adenocarcinoma of the ovary |
| **49** | **Ovarian Serum** | 64 | 7% | 245 | 6% | IIIB | undifferentiated | F | Serous cystadenocarcinoma of the ovary |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **50** | **Ovarian Serum** | 90 | 3% | 203 | 3% | IIIC | | F | Serous adenocarcinoma nos |
| **51** | **Ovarian Serum** | 167 | 3% | 376 | 11% | IIIC | high grade | F | Serous papillary adenocarcinoma nos |
| **52** | **Ovarian Serum** | 112 | 4% | 260 | 5% | IIIC | high grade | F | Serous adenocarcinoma nos |
| **53** | **Ovarian Serum** | 198 | 2% | 340 | 6% | IV | high grade | F | Serous cystadenocarcinoma, nos |
| **54** | **Ovarian Serum** | 134 | 3% | 363 | 6% | I | moderately differentiated | F | Papillary mucinous cystadenocarcinoma of the ovary |
| **55** | **Ovarian Serum** | 118 | 5% | 304 | 10% | IB | high grade | F | Papillary serous cystadenocarcinoma of the ovary |
| **56** | **Ovarian Serum** | 107 | 1% | 326 | 4% | IIIB | high grade | F | Papillary serous cystadenocarcinoma of the ovary |
| **57** | **Ovarian Serum** | 728 | 1% | 1670 | 2% | IIIB | | F | Papillary serous cystadenocarcinoma of the ovary |
| **58** | **Ovarian Serum** | 2138 | 1% | 3257 | 2% | IIIC | | F | Papillary adenocarcinoma of the ovary |
| **59** | **Ovarian Serum** | 167 | 4% | 410 | 1% | III | | F | Papillary serous cystadenocarcinoma of the ovary |
| **60** | **Ovarian Serum** | 3054 | 4% | 3285 | 14% | IIIC | high grade | F | Serous carcinoma of the ovary |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **61** | **Ovarian Serum** | 97 | 3% | 215 | 0% | IIIB | high grade | F | Papillary serous cystadenocarcinoma of the ovary |
| **63** | **Lung Serum** | 61 | 3% | 366 | 10% | IA | moderate to poorly differentiated | M | Adenocarcinoma of the lung |
| **64** | **Lung Serum** | 106 | 1% | 319 | 4% | IB | | M | Adenocarcinoma of the lung |
| **65** | **Lung Serum** | 49 | 2% | 257 | 1% | IB | moderately differentiated | M | Adenocarcinoma of the lung |
| **66** | **Lung Serum** | 75 | 2% | 301 | 2% | II | 3 | M | Adenocarcinoma |
| **67** | **Lung Serum** | 79 | 3% | 283 | 8% | II | 2 | M | Adenocarcinoma |
| **68** | **Lung Serum** | 145 | 2% | 492 | 0% | II | 2 | F | Adenocarcinoma |
| **69** | **Lung Serum** | 142 | 2% | 367 | 6% | IV | n/a | F | adenocarcinoma |
| **70** | **Lung Serum** | 103 | 2% | 231 | 7% | IV | n/a | F | adenocarcinoma |
| **71** | **Lung Serum** | 153 | 1% | 311 | 3% | III B | n/a | F | adenocarcinoma |
| **72** | **Lung Serum** | 54 | 3% | 124 | 6% | III A | missing | M | large and solid cell carcinoma |
| **73** | **Lung Serum** | 183 | 3% | 391 | 10% | III B | missing | F | adenocarcinoma |

(continued)

| | | LLOQ[1] = 1pg/mL | | LLOQ[1] = 5 pg/mL | | | | | |
|---|---|---|---|---|---|---|---|---|---|
| | | **FRα - Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **74** | **Lung Serum** | 91 | 2% | 199 | 7% | missing | 3 | M | poorly differentiated non-keratinizing squamous cell carcinoma |
| **75** | **Lung Serum** | 89 | 7% | 221 | 2% | III B | 3 | F | poorly differentiated adenocarcinoma |
| **76** | **Lung Serum** | 139 | 1% | 330 | 1% | I A | 2 | F | moderately differentiated adenocarcinoma |
| **77** | **Lung Serum** | 197 | 3% | 452 | 7% | III A | 2 | F | moderately differentiated adenocarcinoma |
| **78** | **Lung Serum** | 52 | 3% | 183 | 5% | III A | n/a | F | pleomorphic carcinoma |
| **79** | **Lung Serum** | 80 | 3% | 249 | 8% | III A | n/a | M | pleomorphic carcinoma |
| **80** | **Lung Serum** | 72 | 1% | 158 | 7% | IIIA | 2 | F | moderately differentiated adenocarcinoma |
| **81** | **Lung Serum** | 130 | 12% | 221 | 1% | IA | 3 | M | large and solid cell carcinoma |
| **82** | **Lung Serum** | 81 | 6% | 155 | 1% | IB | missing | M | large and solid cell carcinoma |
| **83** | **Lung Serum** | 127 | 4% | 278 | 3% | IIIB | missing | F | large and solid cell carcinoma |
| **84** | **Lung Serum** | 129 | 3% | 240 | 2% | missing | missing | F | large and solid cell carcinoma |
| **85** | **Lung Serum** | 135 | 2% | 231 | 7% | III B | 2 | M | moderately differentiated adenocarcinoma |
| **86** | **Lung Serum** | 235 | 1% | 330 | 0% | IV | 2 | M | adenocarcinoma |

(continued)

| | | LLOQ[1] = 1pg/mL | | LLOQ[1] = 5 pg/mL | | | | | |
|---|---|---|---|---|---|---|---|---|---|
| | | FRα- Pair 2 | | FRα Pair 1 | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **87** | **Lung Serum** | 243 | 5% | 396 | 2% | IV | 3 | F | poorly differentiated adenocarcinoma |
| **88** | **Lung Serum** | 204 | 3% | 572 | 6% | IA | moderately differentiated | F | Squamous cell carcinoma of the lung |
| **89** | **Lung Serum** | 54 | 9% | 214 | 4% | IB | moderately differentiated | M | Adenocarcinoma of the lung |
| **90** | **Lung Serum** | 116 | 7% | 270 | 0% | IB | moderately differentiated | F | Mucinous adenocarcinoma of the lung |
| **91** | **Lung Serum** | 117 | 3% | 292 | 5% | IIA | moderately differentiated | M | Adenocarcinoma of the lung |
| **92** | **Lung Serum** | 248 | 1% | 578 | 2% | missing | moderately differentiated | M | Adenocarcinoma of the lung |
| **93** | **Lung Serum** | 86 | 2% | 300 | 9% | IB | poorly differentiated | M | Adenocarcinoma of the lung |
| **94** | **Lung Serum** | 33 | 6% | 117 | 5% | IIIA | moderate to poorly differentiated | M | Adenocarcinoma of the lung |
| **95** | **Lung Serum** | 36 | 3% | 196 | 3% | IIIA | poorly differentiated | M | Adenocarcinoma of the lung |
| **96** | **Lung Serum** | 237 | 3% | 722 | 4% | IB | well differentiated | M | Adenocarcinoma of the lung |
| **97** | **Lung Serum** | 82 | 9% | 286 | 2% | IB | poorly differentiated | M | Adenocarcinoma of the lung |
| **98** | **Lung Serum** | 112 | 1% | 431 | 0% | IB | Well to moderately differentiated | F | Adenocarcinoma of the lung |

(continued)

| | | LLOQ[1] = 1pg/mL | | LLOQ[1] = 5 pg/mL | | | | | |
|---|---|---|---|---|---|---|---|---|---|
| | | FRα- Pair 2 | | FRα Pair 1 | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **99** | **Lung Serum** | 137 | 5% | 379 | 0% | IA | moderately differentiated | F | Alveolar adenocarcinoma of the lung |
| **100** | **Lung Serum** | 65 | 2% | 181 | 9% | IA | moderate to poorly differentiated | M | Adenocarcinoma of the lung |
| **101** | **Lung Serum** | 119 | 6% | 280 | 4% | IB | | M | Adenocarcinoma of the lung |
| **102** | **Normal Serum** | 187 | 6% | 391 | 2% | | | F | |
| **103** | **Normal Serum** | 337 | 4% | 560 | 6% | | | F | |
| **104** | **Normal Serum** | 203 | 4% | 405 | 3% | | | F | |
| **105** | **Normal Serum** | 97 | 3% | 311 | 2% | | | F | |
| **106** | **Normal Serum** | 210 | 11% | 393 | 6% | | | F | |
| **107** | **Normal Serum** | 135 | 9% | 271 | 6% | | | M | |
| **108** | **Normal Serum** | 145 | 2% | 225 | 3% | | | F | |
| **109** | **Normal Serum** | 182 | 4% | 257 | 5% | | | M | |
| **110** | **Normal Serum** | 186 | 5% | 297 | 2% | | | M | |
| **111** | **Normal Serum** | 129 | 8% | 197 | 4% | | | M | |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **112** | **Normal Serum** | 133 | 4% | 254 | 4% | | | M | |
| **113** | **Normal Serum** | 136 | 1% | 298 | 9% | | | M | |
| **114** | **Normal Serum** | 189 | 3% | 321 | 1% | | | M | |
| **115** | **Normal Serum** | 167 | 1% | 257 | 6% | | | M | |
| **116** | **Normal Serum** | 159 | 1% | 315 | 1% | | | M | |
| **117** | **Normal Serum** | 166 | 3% | 270 | 1% | | | M | |
| **118** | **Normal Serum** | 197 | 2% | 339 | 0% | | | M | |
| **119** | **Normal Serum** | 148 | 1% | 389 | 4% | | | M | |
| **120** | **Normal Serum** | 198 | 4% | 833 | 9% | | | M | |
| **121** | **Normal Serum** | 101 | 3% | 137 | 0% | | | M | |
| **122** | **Normal Serum** | 111 | 8% | 266 | 1% | | | M | |
| **123** | **Normal Serum** | 96 | 0% | 172 | 2% | | | M | |
| **124** | **Normal Serum** | 224 | 1% | 249 | 7% | | | M | |

(continued)

| | | LLOQ[1] = 1pg/mL | | LLOQ[1] = 5 pg/mL | | | | | |
|---|---|---|---|---|---|---|---|---|---|
| | | FRα- Pair 2 | | FRα Pair 1 | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **125** | **Normal Serum** | 203 | 8% | 312 | 1% | | | M | |
| **126** | **Normal Serum** | 277 | 10% | 388 | 5% | | | M | |
| **127** | **Normal Serum** | 191 | 4% | 272 | 4% | | | F | |
| **128** | **Normal Serum** | 206 | 4% | 297 | 3% | | | F | |
| **129** | **Normal Serum** | 204 | 15% | 194 | 3% | | | F | |
| **130** | **Normal Serum** | 156 | 1% | 106 | 2% | | | F | |
| **131** | **Normal Serum** | 177 | 3% | 195 | 3% | | | F | |
| **132** | **Normal Serum** | 109 | 0% | 148 | 5% | | | M | |
| **134** | **Normal Serum** | 116 | 1% | 281 | 1% | | | F | |
| **135** | **Normal Serum** | 182 | 7% | 250 | 6% | | | M | |
| **136** | **Normal Serum** | 324 | 2% | 475 | 7% | | | F | |
| **137** | **Normal Serum** | 122 | 18% | 191 | 1% | | | F | |
| **138** | **Normal Serum** | 135 | 7% | 185 | 1% | | | F | |

(continued)

| | | LLOQ[1] = 1pg/mL | | LLOQ[1] = 5 pg/mL | | | | | |
|---|---|---|---|---|---|---|---|---|---|
| | | FRα - Pair 2 | | FRα Pair 1 | | | | | |
| MSD Sample Testing Number | Sample Type | Adjusted backfit conc[2] (pg/mL) | %CV | Adjusted backfit conc2 (pg/mL) | %CV | stage | grade | gender | comments |
| 139 | Normal Serum | 264 | 4% | 372 | 2% | | | F | |
| 140 | Normal Serum | 105 | 4% | 188 | 5% | | | M | |
| 141 | Normal Serum | 374 | 0% | 649 | 0% | | | M | |
| 142 | Normal Serum | 93 | 7% | 162 | 7% | | | M | |
| 143 | Normal Serum | 143 | 7% | 326 | 4% | | | F | |
| 144 | Normal Serum | 108 | 3% | 202 | 2% | | | F | |
| 145 | Normal Serum | 153 | 8% | 341 | 3% | | | F | |
| 146 | Normal Serum | 448 | 8% | 400 | 5% | | | F | |
| 147 | Normal Serum | 109 | 4% | 196 | 3% | | | F | |
| 148 | Normal Serum | 142 | 6% | 218 | 1% | | | F | |
| 149 | Normal Serum | 174 | 4% | 309 | 9% | | | F | |
| 150 | Normal Serum | 185 | 5% | 270 | 2% | | | F | |
| 151 | Normal Serum | 180 | 3% | 241 | 0% | | | M | |

(continued)

| | | LLOQ[1] = 1pg/mL | | LLOQ[1] = 5 pg/mL | | | | | |
|---|---|---|---|---|---|---|---|---|---|
| | | **FRα - Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **152** | **Normal Serum** | 125 | 5% | 314 | 4% | | | F | |
| **153** | **Normal Serum** | 270 | 0% | 449 | 2% | | | F | |
| **154** | **Normal Serum** | 127 | 9% | 232 | 1% | | | M | |
| **155** | **Normal Serum** | 251 | 3% | 415 | 6% | | | F | |
| **156** | **Normal Serum** | 121 | 1% | 349 | 0% | | | M | |
| **157** | **Normal Serum** | 137 | 8% | 223 | 0% | | | M | |
| **158** | **Normal Serum** | 77 | 3% | 173 | 6% | | | M | |
| **159** | **Normal Serum** | 143 | 4% | 223 | 7% | | | F | |
| **160** | **Normal Serum** | 121 | 5% | 411 | 8% | | | M | |
| **161** | **Normal Serum** | 99 | 8% | 199 | 3% | | | F | |
| **162** | **Normal Serum** | 158 | 2% | 236 | 0% | | | F | |
| **163** | **Normal Serum** | 138 | 7% | 235 | 3% | | | F | |
| **164** | **Normal Serum** | 175 | 18% | 290 | 2% | | | F | |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **165** | **Normal Serum** | 339 | 4% | 589 | 8% | | | M | |
| **166** | **Normal Serum** | 155 | 4% | 372 | 1% | | | F | |
| **167** | **Normal Serum** | 166 | 0% | 278 | 1% | | | M | |
| **168** | **Normal Serum** | 231 | 7% | 377 | 3% | | | M | |
| **169** | **Normal Serum** | 148 | 10% | 255 | 3% | | | F | |
| **170** | **Normal Serum** | 172 | 2% | 312 | 4% | | | M | |
| **171** | **Normal Serum** | 146 | 6% | 344 | 1% | | | M | |
| **172** | **Normal Serum** | 158 | 3% | 306 | 4% | | | M | |
| **173** | **Normal Serum** | 145 | 2% | 274 | 6% | | | F | |
| **174** | **Normal Serum** | 163 | 12% | 279 | 1% | | | M | |
| **175** | **Normal Serum** | 83 | 5% | 196 | 0% | | | M | |
| **176** | **Normal Serum** | 102 | 7% | 282 | 5% | | | M | |
| **177** | **Normal Serum** | 140 | 3% | 330 | 6% | | | M | |

(continued)

| | | LLOQ[1] = 1pg/mL | | LLOQ[1] = 5 pg/mL | | | | | |
|---|---|---|---|---|---|---|---|---|---|
| | | **FRα- Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **178** | **Normal Serum** | 174 | 9% | 277 | 15% | | | F | |
| **179** | **Normal Serum** | 295 | 3% | 281 | 8% | | | M | |
| **180** | **Normal Serum** | 67 | 4% | 308 | 13% | | | F | |
| **181** | **Normal Serum** | 115 | 3% | 324 | 0% | | | F | |
| **182** | **Normal Serum** | 128 | 5% | 287 | 0% | | | F | |
| **183** | **Normal Serum** | 128 | 1% | 112 | 79% | | | M | |
| **184** | **Normal Serum** | 76 | 3% | 147 | 53% | | | F | |
| **185** | **Normal Serum** | 264 | 7% | 377 | 4% | | | F | |
| **186** | **Normal Serum** | 146 | 13% | 258 | 3% | | | M | |
| **187** | **Normal Serum** | 132 | 0% | 264 | 1% | | | F | |
| **188** | **Normal Serum** | 92 | 4% | 249 | 1% | | | M | |
| **189** | **Normal Serum** | 89 | 11% | 251 | 4% | | | F | |
| **190** | **Normal Serum** | 135 | 5% | 268 | 4% | | | M | |

(continued)

| | | LLOQ[1] = 1pg/mL | | LLOQ[1] = 5 pg/mL | | | | | |
|---|---|---|---|---|---|---|---|---|---|
| | | **FRα - Pair 2** | | **FRα Pair 1** | | | | | |
| **MSD Sample Testing Number** | **Sample Type** | **Adjusted backfit conc[2] (pg/mL)** | **%CV** | **Adjusted backfit conc2 (pg/mL)** | **%CV** | **stage** | **grade** | **gender** | **comments** |
| **191** | **Normal Serum** | 177 | 4% | 394 | 3% | | | F | |
| **192** | **Normal Serum** | 184 | 8% | 367 | 2% | | | F | |
| **193** | **Normal Serum** | 156 | 3% | 387 | 5% | | | F | |
| **194** | **Normal Serum** | 118 | 8% | 275 | 4% | | | M | |
| **195** | **Normal Serum** | 74 | 7% | 217 | 6% | | | M | |
| **196** | **Normal Serum** | 185 | 8% | 373 | 5% | | | F | |
| **197** | **Normal Serum** | 159 | 3% | 378 | 2% | | | F | |
| **198** | **Normal Serum** | 94 | 2% | 245 | 1% | | | F | |

[1] LLOQ is the lower limit of quantitation
[2] The adjusted backfit concentration is adjusted to take into account the sample dilution.

Based on the foregoing data, it was apparent that the 9F3, 2412, 26B3 and 19D4 antibodies were useful in detecting levels of FRα in biological samples, for example serum, derived from a subject. Moreover, the particular combinations of (i) 9F3 as a capture antibody and 24F12 as a detector antibody and (ii) 26B3 as a capture antibody and 19D4 as a detector antibody were capable and particularly effective of assessing levels of FRα in biological samples.

**EXAMPLE 15. ASSESSMENT OF LEVELS OF FRα IN URINE USING THREE DIFFERENT DETECTOR AND CAPTURE ANTIBODY PAIRS**

The ability of three anti-FRα antibody pairs in detecting the levels of FRα in urine samples was assessed. The antibody pairs utilized were as follows: (1) 26B3 as detector antibody and 9F3 as capture antibody, and (2) 24F12 as detector antibody and 9F3 as capture antibody.

**Method**

Two antibody pairs were tested with full calibrator curves and urine pretreated with a 1:1 dilution for 2 minutes in either 6M guanidine, 3M guanidine or PBS control. The following urine samples were tested: three human urine pools diluted 1:80, and five human individual urines diluted 1:80 (one male, four female).

Plates were Biodotted at 150μg/mL, +B, +T, on 4spot STD ((Meso Scale Discovery, Gaithersburg, Maryland)), one capture per well. Detects were run at 1 μg/mL. Diluent 100 + HAMA + mIgG was used for samples and calibrator. Diluent 3 was used for detections. Diluents were commercially available diluents obtained from Meso Scale Discovery.

The following protocol was employed for the ECLIA. Samples were added at 50μL/well. The samples were shaken for 2 hours. The samples were washed with Phosphate Buffered Saline (PBS) solution with the detergent Tween 20 (PBST). The detector antibody was added at 25μL/well. The samples were shaken for 2 hours and subsequently washed with PBST. The electrochemiluminescence (ECL) emission of the samples was read with 2X MSD Buffer T.

The results of these experiments are shown in Tables 26-27.

**Table 26:** Detection of FRα levels in urine using 26B3 as detector antibody and 9F3 as capture antibody

| **Detect** | 26B3 | | | | | | | |
|---|---|---|---|---|---|---|---|---|
| **Capture** | 9F3 | | | | | | | |
| | 6M Guanidine | | | 3M Guanidine | | | PBS Control | |
| Sample ID | Adjusted Backfit conc pg/mL | %CV | % of control | Adjusted Backfit conc pg/mL | %CV | % of control | Adjusted Backfit conc pg/mL | %CV |
| Urine pool 1 | 13,252 | 2% | 114% | 14,505 | 10% | 124% | 11,655 | 17% |
| Urine pool 2 | 14,827 | 5% | 133% | 17,039 | 4% | 152% | 11,187 | 5% |
| Urine pool 3 | 11,280 | 5% | 119% | 9,065 | 10% | 96% | 9,479 | 9% |
| Urine Ind 1 | 1,747 | 3% | 99% | 1,754 | 6% | 100% | 1,760 | 12% |
| Urine Ind 2 | 40,505 | 7% | 145% | 46,622 | 5% | 167% | 27,920 | 13% |
| Urine Ind 3 | 1,623 | 1% | 117% | 1,496 | 5% | 108% | 1,381 | 4% |
| Urine Ind 4 | 12,091 | 2% | 86% | 14,941 | 5% | 107% | 13,996 | 13% |
| Urine Ind 5 | 22,829 | 2% | 128% | 24,607 | 8% | 137% | 17,899 | 5% |
| | | | | | | | | |
| | Average | 3% | 118% | Average | 7% | **124%** | Average | 10% |

(continued)

| Detect | 26B3 | | | | | | | |
|---|---|---|---|---|---|---|---|---|
| Capture | 9F3 | | | | | | | |
| | 6M Guanidine | | | 3M Guanidine | | | PBS Control | |
| Sample ID | Adjusted Backfit conc pg/mL | %CV | % of control | Adjusted Backfit conc pg/mL | %CV | % of control | Adjusted Backfit conc pg/mL | %CV |
| | | | | | difference from 6M condition | 6% | | |

**Table 27:** Detection of FRα levels in urine using 24F12 as detector antibody and 9F3 as capture antibody

| Detect | 24F12 | | | | | | | |
|---|---|---|---|---|---|---|---|---|
| Capture | 9F3 | | | | | | | |
| | 6M Guanidine | | | 3M Guanidine | | | PBS Control | |
| Sample ID | Adjusted Backfit conc pg/mL | %CV | % of control | Adjusted Backfit conc pg/mL | %CV | % of control | Adjusted Backfit conc pg/mL | %CV |
| Urine pool 1 | 10,883 | 2% | 53% | 14,689 | 9% | 72% | 20,504 | 10% |
| Urine pool 2 | 11,763 | 8% | 60% | 16,487 | 7% | 85% | 19,468 | 1% |
| Urine pool 3 | 7,456 | 9% | 40% | 9,362 | 17% | 50% | 18,677 | 7% |
| Urine Ind 1 | 1,376 | 1% | 39% | 1,894 | 7% | 54% | 3,501 | 3% |
| Urine Ind 2 | 29,567 | 0% | 61% | 37,843 | 13% | 78% | 48,607 | 5% |
| Urine Ind 3 | 1,621 | 4% | 61% | 2,153 | 2% | 81% | 2,667 | 1% |
| Urine Ind 4 | 10,470 | 6% | 58% | 14,116 | 5% | 78% | 18,175 | 6% |
| Urine Ind 5 | 19,390 | 6% | 68% | 22,076 | 23% | 78% | 28,421 | 4% |
| | | | | | | | | |
| | Average | 5% | 55% | Average | 10% | **72%** | Average | 5% |
| | | | | | difference from 6M condition | 17% | | |

Based on the foregoing data, it was apparent that the 9F3, 24F12, 26B3 and 19D4 antibodies were useful in detecting levels of FRα in biological samples derived from a subject. Moreover, the combinations of (1) 26B3 as detector antibody and 9F3 as capture antibody and (2) 24F12 as detector antibody and 9F3 as capture antibody were capable and particularly effective of assessing levels of FRα in biological samples.

A second set of experiments, following the protocol described above and using the same two pairs of antibodies, were conducted utilizing four female human individual urines diluted 1:80. The urine was pretreated with a 1:1 dilution for 2 minutes in either 3M guanidine or PBS control. The results are shown in Tables 28-29.

**Table 28:** Detection of FRα levels in urine using 26B3 as detector antibody and 9F3 as capture antibody

| **Detect** | 26B3 | | | | |
|---|---|---|---|---|---|
| **Capture** | 9F3 | | | | |
| | 3M Guanidine | | | PBS Control | |
| Sample ID | Adjusted Backfit conc pg/mL | %CV | % of control | Adjusted Backfit conc pg/mL | %CV |
| Urine Ind 2 | 33,824 | 4% | 98% | 34,569 | 2% |
| Urine Ind 3 | 2,086 | 4% | 99% | 2,107 | 3% |
| Urine Ind 4 | 15,283 | 5% | 97% | 15,696 | 2% |
| Urine Ind 5 | 24,955 | 4% | 92% | 26,991 | 3% |
| | | | | | |
| | Average | 4% | 97% | Average | 3% |

**Table 29:** Detection of FRα levels in urine using 24F12 as detector antibody and 9F3 as capture antibody

| **Detect** | 24F12 | | | | |
|---|---|---|---|---|---|
| **Capture** | 9F3 | | | | |
| | 3M Guanidine | | | PBS Control | |
| Sample ID | Adjusted Backfit conc pg/mL | %CV | % of control | Adjusted Backfit conc pg/mL | %CV |
| Urine Ind 2 | 38,455 | 4% | 106% | 36,414 | 6% |
| Urine Ind 3 | 2,447 | 2% | 109% | 2,250 | 2% |
| Urine Ind 4 | 15,303 | 3% | 81% | 18,964 | 8% |
| Urine Ind 5 | 27,216 | 0% | 95% | 28,651 | 5% |
| | Average | 2% | 98% | Average | 5% |

The results of this second set of experiments further confirm the results of the first set of experiments and demonstrate that the level of FRα which is not bound to a cell can be reliably assessed, for example, in urine, using assays such as the ECLIA assay and using the 26B3, 9F3, 24F12 antibodies. Further, the results demonstrate that such assays can effectively detect FRα using pairs of detector and capture antibodies that bind FRα (such as, *e.g.*, 26B3 as detector antibody and 9F3 as capture antibody, and 24F12 as detector antibody and 9F3 as capture antibody).

## EXAMPLE 16. ASSESSMENT OF LEVELS OF FRα IN SERUM AND PLASMA

The levels of FRα were assessed in samples of serum and plasma on two separate days. The subjects from whom the samples were derived were either normal subjects or patients with ovarian or lung cancer.

The protocol for assessing FRα levels was the same as set forth in Example 14 above. The pairs of antibodies used for assessing FRα levels were also the same as in Example 14, *i.e.*, **Pair 1,** in which 9F3 was the capture antibody and 24F12 was the detector antibody, and **Pair 2,** in which 26B3 was the capture antibody and 19D4 was the detector antibody.

The results are provided in Table 30.

**Table 30: Levels of FR$\alpha$ as assessed in serum and plasma samples on different days**

| | | | | Day 1 | Day 2 | Day 1 | Day 2 | Day 1 | Day 2 | Day 1 | Day 2 |
|---|---|---|---|---|---|---|---|---|---|---|---|
| | | | | **FR$\alpha$/pair 1** | **FR$\alpha$/pair 1** | **FR$\alpha$/pair 1** | **FR$\alpha$/pair 1** | **FR$\alpha$/pair 2** | **FR$\alpha$/pair 2** | **FR$\alpha$/pair 2** | **FR$\alpha$/pair 2** |
| **Donor ID** | **Disease** | **Biosample Confirmed Diagnosis** | **Stage** | **Serum (pg/ml)** | **Serum (pg/ml)** | **Plasma (pg/ml)** | **Plasma (pg/ml)** | **Serum (pg/ml)** | **Serum (pg/ml)** | **Plasma (pg/mL)** | **Plasma (pg/mL)** |
| 17168 | ovary | Serum FRA - Pair 1 versus Pair 2 | I | 1236 | 1282 | 1466 | 1183 | 1298 | 1384 | 1410 | 1336 |
| 46464 | ovary | Serous carcinoma | IIIC | 1589 | 1848 | 2027 | 2147 | 1966 | 2018 | 2066 | 2210 |
| 47219 | ovary | Adenocarcinoma | missing | 447 | 432 | 1208 | 748 | 435 | 446 | 695 | 577 |
| 47721 | ovary | Papillary serous cystadenocarcinoma | IIIB | 1307 | 1400 | 2291 | 2100 | 1642 | 1479 | 1940 | 1807 |
| 48185 | ovary | Adenocarcinoma | missing | 1058 | 1038 | 883 | 652 | 918 | 872 | 811 | 781 |
| 48254 | ovary | Adenocarcinoma | IIIC | 511 | 495 | 3030 | 2569 | 506 | 445 | 1332 | 1370 |
| 48258 | ovary | Adenocarcinoma | missing | 471 | 552 | 1978 | 1070 | 629 | 547 | 978 | 798 |
| 48282 | ovary | Adenocarcinoma | missing | 375 | 388 | 688 | 536 | 446 | 407 | 540 | 428 |
| 48698 | ovary | Carcinoma, undifferentiated | IIIB | 279 | 231 | 308 | 225 | 340 | 255 | 328 | 228 |
| 49028 | ovary | Serous carcinoma | IIIC | 727 | 590 | 158 | 169 | 468 | 526 | 192 | 192 |
| 49030 | ovary | Serous carcinoma | IIIC | 215 | 205 | 695 | 485 | 362 | 291 | 734 | 590 |
| 49033 | ovary | Serous cystadenocarcinoma | IIIC | 579 | 457 | 805 | 717 | 511 | 451 | 629 | 668 |
| 49071 | ovary | Serous cystadenocarcinoma | IV | 335 | 338 | 1884 | 1462 | 559 | 457 | 1224 | 1028 |
| 49092 | ovary | Papillary cystadenocarcinoma | missing | 272 | 228 | 1235 | 615 | 399 | 334 | 678 | 535 |
| 49258 | ovary | Papillary serous cystadenocarcinoma | IIIB | 201 | 190 | 343 | 142 | 328 | 253 | 368 | 244 |
| 49335 | ovary | Serous cystadenocarcinoma | IIIB | 1904 | 1698 | 1560 | 1485 | 1879 | 1503 | 1868 | 1627 |
| 49369 | ovary | Serous carcinoma | IIIC | 2451 | 2805 | 2589 | 2641 | 3402 | 2659 | 2898 | 2605 |
| 49551 | ovary | Serous carcinoma | III | 408 | 385 | 364 | 342 | 505 | 432 | 436 | 418 |
| 50009 | ovary | Serous carcinoma | IIIC | 2887 | 4127 | 2641 | 3811 | 4466 | 3914 | 3533 | 3519 |

(continued)

| | | | | Day 1 | Day 2 | Day 1 | Day 2 | Day 1 | Day 2 | Day 1 | Day 2 |
|---|---|---|---|---|---|---|---|---|---|---|---|
| | | | | **FRα/pair 1** | **FRα/pair 1** | **FRα/pair 1** | **FRα/pair 1** | **FRα/pair 2** | **FRα/pair 2** | **FRα/pair 2** | **FRα/pair 2** |
| **Donor ID** | **Disease** | **Biosample Confirmed Diagnosis** | **Stage** | **Serum (pg/ml)** | **Serum (pg/ml)** | **Plasma (pg/ml)** | **Plasma (pg/ml)** | **Serum (pg/ml)** | **Serum (pg/ml)** | **Plasma (pg/mL)** | **Plasma (pg/mL)** |
| 50370 | ovary | Serous cystadenocarcinoma | IIIB | 619 | 570 | 788 | 611 | 813 | 762 | 708 | 758 |
| 50378 | ovary | Papillary serous cystadenocarcinoma | I | 410 | 387 | 330 | 256 | 388 | 464 | 381 | 387 |
| 50460 | ovary | Papillary serous cystadenocarcinoma | IIIB | 254 | 274 | 688 | 414 | 284 | 272 | 489 | 420 |
| 50467 | ovary | Clear cell adenocarcinoma | I | 427 | 314 | 414 | 300 | 272 | 313 | 292 | 309 |
| 50635 | ovary | Serous cystadenocarcinoma | IA | 247 | 227 | 462 | 367 | 355 | 345 | 392 | 392 |
| 51503 | ovary | Papillary cystadenocarcinoma | I | 291 | 250 | 925 | 651 | 315 | 326 | 818 | 498 |
| 51504 | ovary | Mucinous cystadenoma, borderline malignancy | I | 224 | 184 | 2438 | 1395 | 225 | 240 | 916 | 856 |
| 51506 | ovary | Papillary mucinous cystadenocarcinoma | III | 270 | 258 | 713 | 324 | 335 | 384 | 457 | 424 |
| 52949 | ovary | Adenocarcinoma | missing | 446 | 436 | 288 | 257 | 435 | 483 | 395 | 390 |
| 52952 | ovary | Papillary serous cystadenocarcinoma | missing | 480 | 415 | 451 | 376 | 310 | 351 | 299 | 321 |
| 52957 | ovary | Papillary serous cystadenocarcinoma | III | 373 | 334 | 274 | 221 | 318 | 374 | 303 | 330 |
| 52978 | ovary | Papillary mucinous cystadenocarcinoma | II | 348 | 318 | 533 | 429 | 525 | 559 | 596 | 624 |
| 52980 | ovary | Serous cystadenocarcinoma | III | 627 | 630 | 1447 | 1019 | 834 | 905 | 964 | 996 |
| DLSN-057 | normal | | | 309 | 402 | 413 | 394 | 515 | 527 | 509 | 531 |
| DLSN-056 | normal | | | 273 | 254 | 483 | 498 | 480 | 458 | 522 | 524 |
| DLSN-052 | normal | | | 282 | 289 | 293 | 298 | 446 | 483 | 438 | 401 |

(continued)

| | | | | Day 1 | Day 2 | Day 1 | Day 2 | Day 1 | Day 2 | Day 1 | Day 2 |
|---|---|---|---|---|---|---|---|---|---|---|---|
| | | | | **FRα/pair 1** | **FRα/pair 1** | **FRα/pair 1** | **FRα/pair 1** | **FRα/pair 2** | **FRα/pair 2** | **FRα/pair 2** | **FRα/pair 2** |
| **Donor ID** | **Disease** | **Biosample Confirmed Diagnosis** | **Stage** | **Serum (pg/ml)** | **Serum (pg/ml)** | **Plasma (pg/ml)** | **Plasma (pg/ml)** | **Serum (pg/ml)** | **Serum (pg/ml)** | **Plasma (pg/mL)** | **Plasma (pg/mL)** |
| DLSN-049 | normal | | | 282 | 256 | 351 | 320 | 363 | 394 | 430 | 359 |
| DLSN-048 | normal | | | 362 | 399 | 454 | 434 | 746 | 722 | 824 | 639 |
| DLSN-047 | normal | | | 188 | 176 | 208 | 167 | 275 | 263 | 251 | 212 |
| DLSN-046 | normal | | | 295 | 321 | 276 | 240 | 354 | 310 | 315 | 257 |
| DLSN-045 | normal | | | 259 | 259 | 259 | 189 | 292 | 279 | 273 | 235 |
| DLSN-044 | normal | | | 244 | 236 | 246 | 254 | 284 | 273 | 284 | 271 |
| DLSN-042 | normal | | | 245 | 199 | 210 | 185 | 328 | 301 | 285 | 258 |
| DLSN-040 | normal | | | 392 | 376 | 408 | 406 | 481 | 499 | 502 | 413 |
| DLSN-039 | normal | | | 463 | 470 | 469 | 446 | 617 | 599 | 567 | 528 |
| DLSN-037 | normal | | | 256 | 231 | 256 | 223 | 367 | 338 | 351 | 289 |
| DLSN-029 | normal | | | 285 | 270 | 348 | 337 | 453 | 417 | 418 | 351 |
| DLSN-023 | normal | | | 265 | 254 | 286 | 298 | 458 | 402 | 370 | 386 |
| DLSN-020 | normal | | | 237 | 238 | 311 | 287 | 530 | 496 | 504 | 446 |
| DLSN-011 | normal | | | 344 | 328 | 210 | 207 | 688 | 603 | 297 | 267 |
| DLSN-039 | normal | | | 196 | 178 | 204 | 183 | 259 | 219 | 242 | 248 |
| DLSN-047 | normal | | | | 336 | | 385 | | 489 | | 429 |
| DLSN-052 | normal | | | | 226 | | 208 | | 346 | | 315 |
| DLSL-012 | lung | | | | 340 | | 220 | | 529 | | 375 |
| DLSL-015 | lung | | | | 199 | | 180 | | 236 | | 292 |
| DLSL-023 | lung | | | | 581 | | 347 | | 568 | | 523 |
| DLSL-031 | lung | | | | 400 | | 251 | | 415 | | 343 |
| DLSL-034 | lung | | | | 133 | | 138 | | 299 | | 331 |

(continued)

| | | | | Day 1 | Day 2 | Day 1 | Day 2 | Day 1 | Day 2 | Day 1 | Day 2 |
|---|---|---|---|---|---|---|---|---|---|---|---|
| | | | | **FRα/pair 1** | **FRα/pair 1** | **FRα/pair 1** | **FRα/pair 1** | **FRα/pair 2** | **FRα/pair 2** | **FRα/pair 2** | **FRα/pair 2** |
| **Donor ID** | **Disease** | **Biosample Confirmed Diagnosis** | **Stage** | **Serum (pg/ml)** | **Serum (pg/ml)** | **Plasma (pg/ml)** | **Plasma (pg/ml)** | **Serum (pg/ml)** | **Serum (pg/ml)** | **Plasma (pg/mL)** | **Plasma (pg/mL)** |
| 1 | lung | | | | 238 | | 339 | | 462 | | 460 |
| 3 | lung | | | | 120 | | 185 | | 203 | | 334 |
| 6 | lung | | | | 295 | | 1646 | | 431 | | 868 |
| 18 | lung | | | | 328 | | 288 | | 466 | | 418 |
| 18639 | lung | | | | 124 | | 333 | | 128 | | 216 |
| 18640 | lung | | | | 246 | | 275 | | 221 | | 221 |
| 50666 | lung | | | | 372 | | 357 | | 405 | | 338 |
| 2 | lung | | | | 214 | | 327 | | 274 | | 269 |
| 4 | lung | | | | 347 | | 625 | | 526 | | 575 |
| 7 | lung | | | | 224 | | 308 | | 421 | | 452 |
| 12 | lung | | | | 409 | | 424 | | 778 | | 725 |
| 13 | lung | | | | 509 | | 590 | | 1251 | | 1360 |
| 14 | lung | | | | 250 | | 250 | | 409 | | 357 |
| 15 | lung | | | | 502 | | 466 | | 606 | | 594 |
| 16 | lung | | | | 153 | | 274 | | 246 | | 275 |
| 19 | lung | | | | 357 | | 905 | | 428 | | 576 |
| DLS4-0002 | lung | | | | 234 | | 245 | | 410 | | 347 |
| DLS4-0004 | lung | | | | 472 | | 520 | | 507 | | 538 |
| DLSO-007 | ovary | | | | 389 | | 593 | | 574 | | 544 |
| DLSO-008 | ovary | | | | 175 | | 295 | | 386 | | 364 |
| DLSO-009 | ovary | | | | 232 | | 331 | | 299 | | 349 |
| DLSO-014 | ovary | | | | 265 | | 300 | | 430 | | 467 |

(continued)

| | | | | Day 1 | Day 2 | Day 1 | Day 2 | Day 1 | Day 2 | Day 1 | Day 2 |
|---|---|---|---|---|---|---|---|---|---|---|---|
| | | | | **FRα/pair 1** | **FRα/pair 1** | **FRα/pair 1** | **FRα/pair 1** | **FRα/pair 2** | **FRα/pair 2** | **FRα/pair 2** | **FRα/pair 2** |
| **Donor ID** | **Disease** | **Biosample Confirmed Diagnosis** | **Stage** | **Serum (pg/ml)** | **Serum (pg/ml)** | **Plasma (pg/ml)** | **Plasma (pg/ml)** | **Serum (pg/ml)** | **Serum (pg/ml)** | **Plasma (pg/mL)** | **Plasma (pg/mL)** |
| DLSO-020 | ovary | | | | 536 | | 261 | | 453 | | 389 |
| DLSO-026 | ovary | | | | | | | | | | |
| DLSO-027 | ovary | | | | 290 | | 140 | | 269 | | 218 |
| DLSO-028 | ovary | | | | 357 | | 207 | | 397 | | 18 |
| DLSO-029 | ovary | | | | 412 | | 236 | | 369 | | 361 |
| DLSO-030 | ovary | | | | 387 | | 498 | | 595 | | 616 |
| DLSO-031 | ovary | | | | 348 | | 496 | | 353 | | 413 |
| DLSO-034 | ovary | | | | 197 | | 222 | | 308 | | 346 |
| DLSO-035 | ovary | | | | 144 | | 540 | | 298 | | 587 |
| DLSO-023 | ovary | | | | 404 | | 216 | | 423 | | 337 |
| DLSO-025 | ovary | | | | 306 | | 156 | | 301 | | 272 |
| DLSO-026 | ovary | | | | 231 | | 293 | | 406 | | 378 |
| DLSO-032 | ovary | | | | 151 | | 190 | | 240 | | 255 |
| DLSO-018 | ovary | | | | 155 | | 340 | | 303 | | 343 |
| DLSO-019 | ovary | | | | 350 | | 330 | | | | |
| DLSO-021 | ovary | | | | 359 | | 365 | | 346 | | 434 |
| DLSO-024 | ovary | | | | 260 | | 140 | | 338 | | 318 |
| 10001627 | ovary | | | | 1481 | | 1396 | | 1583 | | 1655 |
| 11025393 | ovary | | | | 5241 | | 4069 | | 4998 | | 5486 |
| 11025394 | ovary | | | | 473 | | 371 | | 488 | | 518 |
| 110025395 | ovary | | | | 3215 | | 2920 | | 3466 | | 4043 |
| 110025397 | ovary | | | | 109 | | 145 | | 245 | | 232 |

(continued)

| | | | | Day 1 | Day 2 | Day 1 | Day 2 | Day 1 | Day 2 | Day 1 | Day 2 |
|---|---|---|---|---|---|---|---|---|---|---|---|
| | | | | **FRα/pair 1** | **FRα/pair 1** | **FRα/pair 1** | **FRα/pair 1** | **FRα/pair 2** | **FRα/pair 2** | **FRα/pair 2** | **FRα/pair 2** |
| **Donor ID** | **Disease** | **Biosample Confirmed Diagnosis** | **Stage** | **Serum (pg/ml)** | **Serum (pg/ml)** | **Plasma (pg/ml)** | **Plasma (pg/ml)** | **Serum (pg/ml)** | **Serum (pg/ml)** | **Plasma (pg/mL)** | **Plasma (pg/mL)** |
| 110025398 | ovary | | | | 476 | | 497 | | 613 | | 543 |
| 110025399 | ovary | | | | 166 | | 145 | | 234 | | 229 |
| 110025402 | ovary | | | | 219 | | 221 | | 389 | | 357 |
| 110025403 | ovary | | | | 7529 | | 6990 | | 13033 | | 11888 |
| 110025405 | ovary | | | | 510 | | 508 | | 836 | | 835 |
| 5 | ovary | | | | 164 | | 328 | | 252 | | 375 |
| 8 | ovary | | | | 251 | | 709 | | 473 | | 573 |
| 9 | ovary | | | | 249 | | 743 | | 482 | | 561 |
| 10 | ovary | | | | 288 | | 569 | | 412 | | 446 |
| 11 | ovary | | | | 180 | | 417 | | 310 | | 397 |
| 17 | ovary | | | | 296 | | 760 | | 342 | | 628 |
| 110025392 | ovary | | | | 1094 | | 2210 | | 907 | | 1294 |

Based on the foregoing data, it was apparent that the 9F3, 2412, 26B3 and 19D4 antibodies were useful in detecting levels of FRα in biological samples, for example, serum or plasma, derived from a subject. Moreover, the particular combinations of (i) 9F3 as a capture antibody and 24F12 as a detector antibody and (ii) 26B3 as a capture antibody and 19D4 as a detector antibody were capable and particularly effective in assessing levels of FRα in biological samples.

For assays conducted using both Pair 1 and Pair 2, there was a high correlation between serum and plasma FRα levels. Figure 16 shows the correlation in serum versus plasma FRα levels for assays conducted using Pair 1 (see Example 16). The $R^2$ value was 0.8604. Figure 17 shows the correlation in serum versus plasma FRα levels for assays conducted using Pair 2 (see Example 16). The $R^2$ value was 0.9766.

For both serum and plasma samples, there was a high correlation between FRα levels measured using Pair 1 and Pair 2. Figure 18 shows the correlation in serum FRα levels for assays conducted using Pair 1 versus Pair 2 (see Example 16). The $R^2$ value was 0.9028. Figure 19 shows the correlation in plasma FRα levels for assays conducted using pair 1 versus pair 2 (see Example 16). The $R^2$ value was 0.8773.

The results also showed that there was a high correlation between FRα levels measured on different days. Figure 20 shows the interday correlation in serum FRα levels for assays conducted using pair 2. The $R^2$ value was 0.9839.

## EQUIVALENTS

Those skilled in the art will recognize, or be able to ascertain using no more than routine experimentation, many equivalents of the specific embodiments of the invention described herein.

## Claims

1. A method of assessing whether a subject is afflicted with an FRα-expressing cancer, the method comprising determining the level of folate receptor alpha (FRα) which is not bound to a cell, in a urine sample derived from said subject; and
comparing the level of folate receptor alpha (FRα) which is not bound to a cell with the level of FRα in a control sample, wherein a difference between the level of FRα in the urine sample derived from said subject and the level of FRα in the control sample is an indication that the subject is afflicted with an FRα-expressing cancer;
wherein the level of FRα which is not bound to a cell in the urine sample derived from said subject is assessed by contacting the sample with an antibody that binds FRα.

2. The method of claim 1, wherein the FRα-expressing cancer is selected from the group consisting of lung cancer, mesothelioma, ovarian cancer, renal cancer, brain cancer, cervical cancer, nasopharyngeal cancer, squamous cell carcinoma of the head and neck, endometrial cancer, breast cancer, bladder cancer, pancreatic cancer, bone cancer, pituitary cancer, colorectal cancer and medullary thyroid cancer; optionally wherein the FRα-expressing cancer is ovarian cancer or lung cancer; optionally wherein the FRα-expressing cancer is non-small cell lung cancer; optionally wherein the non-small cell lung cancer is adenocarcinoma.

**3.** The method of claim 1, wherein the presence of FRα in said urine sample at a concentration of greater than 9500 pg/mL, 10,000 pg/mL, 11,000 pg/mL, 12,000 pg/mL, 13,000 pg/mL, 14,000 pg/mL, 15,000 pg/mL, 16,000 pg/mL, 17,000 pg/mL, 18,000 pg/mL, 19,000 pg/mL, or 20,000 pg/mL is an indication that the subject is afflicted with ovarian cancer.

**4.** The method of claim 1, wherein the antibody is selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) the MOV18 antibody;
(d) an antibody that binds the same epitope as the MOV18 antibody;
(e) the 548908 antibody;
(f) an antibody that binds the same epitope as the 548908 antibody;
(g) the 6D398 antibody;
(h) an antibody that binds the same epitope as the 6D398 antibody;
(i) an antibody that binds the same epitope as the 26B3 antibody;
(j) an antibody comprising SEQ ID NO:55 (GYFMN) as CDRH1, SEQ ID NO:56 (RIFPYNGDTFYNQKFKG) as CDRH2, SEQ ID NO:57 (GTHYFDY) as CDRH3, SEQ ID NO:51 (RTSENIFSYLA) as CDRL1 , SEQ ID NO:52 (NAKTLAE) as CDRL2 and SEQ ID NO:53 (QHHYAFPWT) as CDRL3;
(k) the 26B3 antibody;
(l) an antibody that binds the same epitope as the 19D4 antibody;
(m) an antibody comprising SEQ ID NO:39 (HPYMH) as CDRH1, SEQ ID NO:40 (RIDPANGNTKYDPKFQG) as CDRH2, SEQ ID NO:41 (EEVADYTMDY) as CDRH3, SEQ ID NO:35 (RASESVDTYGNNFIH) as CDRL1, SEQ ID NO:36 (LASNLES) as CDRL2 and SEQ ID NO:37 (QQNNGDPWT) as CDRL3;
(n) the 19D4 antibody;
(o) an antibody that binds the same epitope as the 9F3 antibody;
(p) an antibody comprising SEQ ID NO:31 (SGYYWN) as CDRH1, SEQ ID NO:32 (YIKSDGSNNYNPSLKN) as CDRH2, SEQ ID NO:33 (EWKAMDY) as CDRH3, SEQ ID NO:27 (RASSTVSYSYLH) as CDRL1, SEQ ID NO:28 (GTSNLAS) as CDRL2 and SEQ ID NO:29 (QQYSGYPLT) as CDRL3;
(q) the 9F3 antibody;
(r) an antibody that binds the same epitope as the 24F12 antibody;
(s) an antibody comprising SEQ ID NO:47 (SYAMS) as CDRH1, SEQ ID NO:48 (EIGSGGSYTYYPDTVTG) as CDRH2, SEQ ID NO:49 (ETTAGYFDY) as CDRH3, SEQ ID NO:43 (SASQGINNFLN) as CDRL1, SEQ ID NO:44 (YTSSLHS) as CDRL2 and SEQ ID NO:45 (QHFSKLPWT) as CDRL3;
(t) the 24F12 antibody;
(u) an antibody that comprises a variable region light chain selected from the group consisting of LK26HuVK (SEQ ID NO: 13); LK26HuVKY (SEQ ID NO: 14); LK26HuVKPW (SEQ ID NO: 15); and LK26HuVKPW,Y (SEQ ID NO: 16);
(v) an antibody that comprises a variable region heavy chain selected from the group consisting of LK26HuVH (SEQ ID NO: 17); LK26HuVH FAIS,N (SEQ ID NO: 18); LK26HuVH SLF (SEQ ID NO: 19); LK26HuVH I,I (SEQ ID NO: 20); and LK26KOLHuVH (SEQ ID NO: 21);
(w) an antibody that comprises the heavy chain variable region LK26KOLHuVH (SEQ ID NO: 21) and the light chain variable region LK26HuVKPW,Y (SEQ ID NO: 16);
(x) an antibody that comprises the heavy chain variable region LK26HuVH SLF (SEQ ID NO: 19) and the light chain variable region LK26HuVKPW,Y (SEQ ID NO: 16); and
(y) an antibody that comprises the heavy chain variable region LK26HuVH FAIS,N (SEQ ID NO: 18) and the light chain variable region LK26HuVKPW,Y (SEQ ID NO: 16).

**5.** The method of claim 1, wherein the antibody is (a) selected from the group consisting of a murine antibody, a human antibody, a humanized antibody, a bispecific antibody, a chimeric antibody, a Fab, Fab'2, ScFv, SMIP, affibody, avimer, versabody, nanobody, and a domain antibody; or (b) labeled; optionally wherein the antibody is labeled with a label selected from the group consisting of a radio-label, a biotin-label, a chromophore-label, a fluorophore-label, an ECL label and an enzyme-label.

**6.** The method of claim 1, wherein the level of FRα is determined by using a technique selected from the group consisting of western blot analysis, radioimmunoassay, immunofluorimetry, immunoprecipitation, equilibrium dialysis, immunodiffusion, solution phase assay, electrochemiluminescence immunoassay (ECLIA) and ELISA assay.

**7.** The method of claim 1, wherein the control sample comprises a standardized control level of FRα in a healthy subject.

**8.** The method of claim 1, wherein the urine sample is (a) treated with guanidine prior to determining the level of FRα in the urine sample; or (b) diluted prior to determining the level of FRα in the urine sample; or (c) centrifuged, vortexed, or both, prior to determining the level of FRα in the urine sample.

**9.** The method of claim 1, wherein the level of FRα in the urine sample derived from said subject is assessed by contacting the urine sample with a pair of antibodies selected from the group consisting of

(a) MOV18 antibody immobilized to a solid support and labeled MORAB-003 antibody,
(b) 9F3 antibody immobilized to a solid support and labeled 24F12 antibody,
(c) 26B3 antibody immobilized to a solid support and labeled 19D4 antibody, and
(d) 9F3 antibody immobilized to a solid support and labeled 26B3 antibody.

**10.** Use of a kit according to the method of any of claims 1-9 for assessing whether a subject is afflicted with an FRα-expressing cancer in a subject, the kit comprising
means for determining the level of folate receptor alpha (FRα) which is not bound to a cell in a urine sample derived from said subject; and
instructions for use of the kit to assess whether the subject is afflicted with an FRα-expressing cancer or to assess the progression of an FRα-expressing cancer.

**Patentansprüche**

**1.** Verfahren zur Bestimmung, ob ein Individuum von einem FRα-exprimierenden Krebs befallen ist, welches umfasst Bestimmen der Menge an Folat-Rezeptor alpha (FRα), der nicht an eine Zelle gebunden ist, in einer von dem Individuum abgeleiteten Urinprobe; und
Vergleichen der Menge an Folat-Rezeptor alpha (FRα), der nicht an eine Zelle gebunden ist mit der Menge an FRα in einer Kontroll-Probe, worin ein Unterschied zwischen der Menge an FRα in der von dem Individuum abgeleiteten Urinprobe and der Menge an FRα in der Kontroll-Probe ein Anzeichen dafür ist, dass das Individuum von einem FRα-exprimierenden Krebs befallen ist;
worin die Menge an FRα, der nicht an eine Zelle gebunden ist, in der von dem Individuum abgeleiteten Urinprobe durch Inkontaktbringen der Probe mit einem Antikörper bestimmt wird, der an FRα bindet.

**2.** Verfahren nach Anspruch 1, worin der FRα-exprimierende Krebs ausgewählt ist aus der Gruppe bestehend aus Lungen-Krebs, Mesotheliomie, Eierstock-Krebs, Nieren-Krebs, Hirn-Krebs, Cervix-Krebs, Nasopharynx-Krebs, Plattenepithelkarzinom des Kopfs und Nackens, Endometrial-Krebs, Brust-Krebs, Blasen-Krebs, Pankreas-Krebs, Knochen-Krebs, Hirnanhangsdrüsen-Krebs, Darm-Krebs und medulläres Schilddrüsen-Karzinom; wahlweise worin der FRα-exprimierende Krebs Eierstock-Krebs oder Lungen-Krebs ist; wahlweise worin der FRα-exprimierende Krebs nicht-kleinzelliges Lungenkarzinom ist; wahlweise worin das nicht-kleinzellige Lungenkarzinom Adenokarzinom ist.

**3.** Verfahren nach Anspruch 1, worin die Anwesenheit von FRα in der Urinprobe bei einer Konzentration von mehr als 9.500 pg/ml, 10.000 pg/ml, 11.00 pg/ml, 12.000 pg/ml, 13.000 pg/ml, 14.000 pg/ml, 15.000 pg/ml, 16.000 pg/ml, 17.000 pg/ml, 18.000 pg/ml, 19.000 pg/ml, oder 20.000 pg/ml ein Anzeichen dafür ist dass das Individuum von Eierstock-Krebs befallen ist.

**4.** Verfahren nach Anspruch 1, worin der Antikörper ausgewählt ist aus der Gruppe bestehend aus:

(a) einem Antikörper, der an das gleiche Epitop bindet, wie der MORAb-003 Antikörper;
(b) einem Antikörper, umfassend die SEQ ID NR:1 (GFTFSGYGLS) als CDRH1, SEQ ID NR:2 (MISSGGSYTYVADSVKG) als CDRH2, SEQ ID NR:3 (HGDDPAWFAY) als CDRH3, SEQ ID NR:4 (SVSSSISSNNLH) als CDRL1, SEQ ID NR:5 (GTSNLAS) als CDRL2 and SEQ ID NR:6 (QQWSSYPYMYT) als CDRL3;
(c) dem MOV18 Antikörper;
(d) einem Antikörper, der an das gleiche Epitop bindet wie der MOV18 Antikörper;
(e) dem 548908 Antikörper;
(f) einem Antikörper, der an das gleiche Epitop bindet wie der 548908 Antikörper;
(g) dem 6D398 Antikörper;
(h) einem Antikörper, der an das gleiche Epitop bindet wie der 6D398 Antikörper;
(i) einem Antikörper, der an das gleiche Epitop bindet wie der2683 Antikörper;
j) einem Antikörper umfassend SEQ ID NR:55 (GYFMN) als CDRH1, SEQ ID NR:56 (RIFPYNGDTFYNQKFKG) als CDRH2, SEQ ID NR:57 (GTHYFOY) als CDRH3, SEQ ID NR:51 (RTSENIFSYLA) als CDRL1 , SEQ ID NR:52 (NAKTLAE) als CDRL2 and SEQ ID NR:53 (QHHYAFPWT) als CDRL3;
(k) dem 2683 Antikörper;
(i) einem Antikörper, der an das gleiche Epitop bindet wie der 1904 Antikörper;
(m) einem Antikörper umfassend SEQ ID NR:39 (HPYMH) als CDRH1, SEQ ID NR:40 (RIDPANGNTKYOPKFQG) als CDRH2, SEQ ID NR:41 (EEVADYTMOY) als CDRH3, SEQ ID NR:35 (RASESVDTYGNNFIH) als CDRL1, SEQ ID NR:36 (LASNLES) als CDRL2 and SEQ ID NR:37 (QQNNGDPWT) als CDRL3;
(n) dem 19D4 Antikörper;
(o) einem Antikörper, der an das gleiche Epitop bindet wie der 9F3 Antikörper;
(p) einem Antikörper umfassend SEQ ID NR:31 (SGYYWN) als CDRH1, SEQ ID NR:32 (YIKSOGSNNYNPSLKN) als CDRH2, SEQ ID NR:33 (EWKAMDY) als CDRH3, SEQ ID NR:27 (RASSTVSYSYLH) als CDRL1, SEQ ID NR:28 (GTSNLAS) als CDRL2 and SEQ ID NR:29 (QQYSGYPLT) als CDRL3;
(q) dem 9F3 Antikörper;
(r) einem Antikörper, der an das gleiche Epitop bindet wie der 24F12 Antikörper;
(s) einem Antikörper umfassend SEQ ID NR:47 (SYAMS) als CDRH1, SEQ ID NR:48 (EIGSGGSYTYVPDTVTG) als CDRH2, SEQ ID NR:49 (ETTAGYFDY) als CDRH3, SEQ ID NR:43 (SASQGINNFLN) als CDRL1, SEQ ID N0:44 (YTSSLHS) als CDRL2 and SEQ ID NR:45 (QHFSKLPWT) als CDRL3;
(t) dem 24F12 Antikörper;
(u) einem Antikörper, der eine leichte Kette der variablen Region umfasst, ausgewählt aus der Gruppe bestehend aus LK26HuVK (SEQ ID NR: 13); LK26HuVKY (SEQ ID NR: 14); LK26HuVKPW (SEQ ID NR: 15); and LK26HuVKPW,Y (SEQ ID NR: 16);
(v) einem Antikörper, der eine variable Region der schweren Kette umfasst, ausgewählt aus der Gruppe bestehend aus LK26HuVH (SEQ ID NR: 17); LK26HuVH FAIS,N (SEQ ID NR: 18); LK26HuVH SLF (SEQ ID NR: 19); LK26HuVH I,I (SEQ ID NR: 20); und LK26KOLHuVH (SEQ ID NR: 21);
(w) einem Antikörper, der die variable Region der schweren Kette LK26KOLHuVH (SEQ ID NR: 21) und die leichte Kette der variablen Region LK26HuVKPW,Y (SEQ ID NR: 16) umfasst;
(x) einem Antikörper, der die variable Region der schweren Kette LK26HuVH SLF (SEQ ID NR: 19) und die leichte Kette der variablen Region LK26HuVKPW,Y (SEQ ID NR: 16) umfasst; und
(y) einem Antikörper, der die variable Region der schweren Kette LK26HuVH FAIS,N (SEQ ID NR: 18) und die leichte Kette der variablen Region LK26HuVKPW,Y (SEQ ID NR: 16) umfasst.

**5.** Verfahren nach Anspruch 1, worin der Antikörper (a) ausgewählt ist aus der Gruppe bestehend aus einem Maus-Antikörper, einem humanen Antikörper, einem humanisierten Antikörper, einem bispezifischen Antikörper, einem chimären Antikörper, einem Fab, Fab'2, ScFv, SMIP, Affikörper, Avimer, Versakörper, Nanokörper, and einer Antikörperdomäne; oder (b) markiert ist;
wahlweise worin der Antikörper mit einem Marker markiert ist ausgewählt aus der Gruppe bestehend aus einem Radio-Marker, einem Biotin-Marker, einem Chromophor-Marker, einem Fluorophor-Marker, einem ECL-Marker und einem Enzym-Marker.

**6.** Verfahren nach Anspruch 1, worin die Menge an FRα unter Verwendung einer Technik bestimmt wird, ausgewählt aus der Gruppe bestehend aus Western Blot Analyse, Radioimmunassay, Immunfluorimetrie, Immunausfällung, Gleichgewichtsdialyse, Immundiffusion, Lösungsphasen-Assay, Elektrochemilumineszenz-Immunassay (ECLIA) und ELISA-Assay.

**7.** Verfahren nach Anspruch 1, worin die Kontroll-Probe eine standardisierte Kontroll-Menge an FRα in einem gesunden Individuum umfasst.

**8.** Verfahren nach Anspruch 1, worin die Urinprobe (a) vor Bestimmung der Menge an FRα in der Urinprobe mit Guanidin behandelt wird; oder (b) vor der Bestimmung der Menge an FRα in der Urinprobe verdünnt wird; oder (c) vor Bestimmung der Menge an FRα in der Urinprobe zentrifugiert, gevortexed oder beides wird.

**9.** Verfahren nach Anspruch 1, worin die von dem Individuum abgeleitete Menge an FRα in der Urinprobe durch Inkontaktbringen der Urinprobe mit einem Antikörper-Paar bestimmt wird, ausgewählt aus der Gruppe bestehend aus

(a) einem auf einem festen Träger immobilisierten MOV18 Antikörper und einem markierten MORA8-003 Antikörper,
(b) einem auf einem festen Träger immobilisierten 9F3 Antikörper und einem markierten 24F12 Antikörper,
(c) einem auf einem festen Träger immobilisierten 2683 Antikörper und einem markierten 1904 Antikörper, und
(d) einem auf einem festen Träger immobilisierten 9F3 Antikörper und einem markierten 2683 Antikörper.

**10.** Verwendung eines Kits gemäß einem Verfahren nach einem der Ansprüche 1-9 zur Bestimmung, ob ein Individuum von einem FRα-exprimierenden Krebs befallen ist, worin der Kit umfasst
Mittel zur Bestimmung der Menge an Folat-Rezeptor alpha (FRα), der nicht an eine Zelle gebunden ist, in einer von dem Individuum abgeleiteten Urinprobe; und
Instruktionen zur Verwendung des Kit zur Bestimmung, ob das Individuum von einem FRα-exprimierenden Krebs befallen ist, oder zur Bestimmung des Fortschreitens eines FRα-exprimierenden Krebs.

**Revendications**

**1.** Procédé d'estimation si un sujet est atteint d'un cancer exprimant le FRα, le procédé comprenant
la détermination du taux de récepteur alpha des folates (FRα) qui n'est pas lié à une cellule, dans un échantillon d'urine dérivé dudit sujet ; et
la comparaison du taux de récepteur alpha des folates (FRα) qui n'est pas lié à une cellule au taux de FRα dans un échantillon témoin, une différence entre le taux de FRα dans l'échantillon d'urine dérivé dudit sujet et le taux de FRα dans l'échantillon témoin étant une indication que le sujet est atteint d'un cancer exprimant le FRα ;
dans lequel le taux de FRα qui n'est pas lié à une cellule dans l'échantillon d'urine dérivé dudit sujet est estimé par la mise en contact de l'échantillon avec un anticorps qui lie le FRα.

**2.** Procédé selon la revendication 1, dans lequel le cancer exprimant le FRα est choisi dans le groupe constitué du cancer du poumon, du mésothéliome, du cancer ovarien, du cancer rénal, du cancer du cerveau, du cancer du col de l'utérus, du cancer nasopharyngé, du carcinome à cellules squameuses de la tête et du cou, du cancer de l'endomètre, du cancer du sein, du cancer de la vessie, du cancer pancréatique, du cancer des os, du cancer de l'hypophyse, du cancer colorectal et du cancer médullaire de la thyroïde ; éventuellement dans lequel le cancer exprimant le FRα est le cancer ovarien ou le cancer du poumon ; éventuellement dans lequel le cancer exprimant le FRα est le cancer du poumon non à petites cellules ; éventuellement dans lequel le cancer du poumon non à petites cellules est l'adénocarcinome.

**3.** Procédé selon la revendication 1, dans lequel la présence de FRα dans ledit échantillon d'urine à une concentration supérieure à 9500 pg/ml, 10 000 pg/ml, 11 000 pg/ml, 12 000 pg/ml, 13 000 pg/ml, 14 000 pg/ml, 15 000 pg/ml, 16 000 pg/ml, 17 000 pg/ml, 18 000 pg/ml, 19 000 pg/ml, ou 20 000 pg/ml est une indication que le sujet est atteint d'un cancer ovarien.

**4.** Procédé selon la revendication 1, dans lequel l'anticorps est choisi dans le groupe constitué de :

(a) un anticorps qui lie le même épitope que l'anticorps MORAb-003 ;
(b) un anticorps comprenant SEQ ID NO : 1 (GFTFSGYGLS) en tant que CDRH1, SEQ ID NO : 2 (MISSGGSYTYYADSVKG) en tant que CDRH2, SEQ ID NO : 3 (HGDDPAWFAY) en tant que CDRH3, SEQ ID NO : 4 (SVSSSISSNNLH) en tant que CDRL1, SEQ ID NO : 5 (GTSNLAS) en tant que CDRL2 et SEQ ID NO : 6 (QQWSSYPYMYT) en tant que CDRL3 ;
(c) l'anticorps MOV18 ;
(d) un anticorps qui lie le même épitope que l'anticorps MOV18 ;
(e) l'anticorps 548908 ;
(f) un anticorps qui lie le même épitope que l'anticorps 548908 ;
(g) l'anticorps 6D398 ;
(h) un anticorps qui lie le même épitope que l'anticorps 6D398 ;

(i) un anticorps qui lie le même épitope que l'anticorps 26B3 ;
(j) un anticorps comprenant SEQ ID NO : 55 (GYFMN) en tant que CDRH1, SEQ ID NO : 56 (RIFPYNGDTFYNQKFKG) en tant que CDRH2, SEQ ID NO : 57 (GTHYFDY) en tant que CDRH3, SEQ ID NO : 51 (RTSENIFSYLA) en tant que CDRL1, SEQ ID NO : 52 (NAKTLAE) en tant que CDRL2 et SEQ ID NO : 53 (QHHYAFPWT) en tant que CDRL3 ;
(k) l'anticorps 26B3 ;
(l) un anticorps qui lie le même épitope que l'anticorps 19D4 ;
(m) un anticorps comprenant SEQ ID NO : 39 (HPYMH) en tant que CDRH1, SEQ ID NO : 40 (RIDPANGNTKYDPKFQG) en tant que CDRH2, SEQ ID NO : 41 (EEVADYTMDY) en tant que CDRH3, SEQ ID NO : 35 (RASESVDTYGNNFIH) en tant que CDRL1, SEQ ID NO : 36 (LASNLES) en tant que CDRL2 et SEQ ID NO : 37 (QQNNGDPWT) en tant que CDRL3 ;
(n) l'anticorps 19D4 ;
(o) un anticorps qui lie le même épitope que l'anticorps 9F3 ;
(p) un anticorps comprenant SEQ ID NO : 31 (SGYYWN) en tant que CDRH1, SEQ ID NO : 32 (YIKSDGSNNYNPSLKN) en tant que CDRH2, SEQ ID NO : 33 (EWKAMDY) en tant que CDRH3, SEQ ID NO : 27 (RASSTVSYSYLH) en tant que CDRL1, SEQ ID NO : 28 (GTSNLAS) en tant que CDRL2 et SEQ ID NO : 29 (QQYSGYPLT) en tant que CDRL3 ;
(q) l'anticorps 9F3 ;
(r) un anticorps qui lie le même épitope que l'anticorps 24F12 ;
(s) un anticorps comprenant SEQ ID NO : 47 (SYAMS) en tant que CDRH1, SEQ ID NO : 48 (EIGSGGSYTYYPDTVTG) en tant que CDRH2, SEQ ID NO : 49 (ETTAGYFDY) en tant que CDRH3, SEQ ID NO : 43 (SASQGINNFLN) en tant que CDRL1, SEQ ID NO : 44 (YTSSLHS) en tant que CDRL2 et SEQ ID NO : 45 (QHFSKLPWT) en tant que CDRL3 ;
(t) l'anticorps 24F12 ;
(u) un anticorps qui comprend une région variable de chaîne légère choisie dans le groupe constitué de LK26HuVK (SEQ ID NO : 13) ; LK26HuVKY (SEQ ID NO : 14) ; LK26HuVKPW (SEQ ID NO : 15) ; et LK26HuVKPW, Y (SEQ ID NO : 16) ;
(v) un anticorps qui comprend une région variable de chaîne lourde choisie dans le groupe constitué de LK26HuVH (SEQ ID NO : 17) ; LK26HuVH FAIS,N (SEQ ID NO : 18) ; LK26HuVH SLF (SEQ ID NO : 19) ; LK26HuVH I, I (SEQ ID NO : 20) ; et LK26KOLHuVH (SEQ ID NO : 21) ;
(w) un anticorps qui comprend la région variable de chaîne lourde LK26KOLHuVH (SEQ ID NO : 21) et la région variable de chaîne légère LK26HuVKPW,Y (SEQ ID NO : 16) ;
(x) un anticorps qui comprend la région variable de chaîne lourde LK26HuVH SLF (SEQ ID NO : 19) et la région variable de chaîne légère LK26HuVKPW,Y (SEQ ID NO : 16) ; et
(y) un anticorps qui comprend la région variable de chaîne lourde LK26HuVH FAIS,N (SEQ ID NO : 18) et la région variable de chaîne légère LK26HuVKPW,Y (SEQ ID NO : 16).

**5.** Procédé selon la revendication 1, dans lequel l'anticorps est (a) choisi dans le groupe constitué d'un anticorps murin, un anticorps humain, un anticorps humanisé, un anticorps bispécifique, un anticorps chimérique, un Fab, Fab'2, ScFv, SMIP, affibody, avimer, versabody, nanobody, et un anticorps à domaine unique ; ou (b) marqué ; éventuellement dans lequel l'anticorps est marqué avec un marqueur choisi dans le groupe constitué d'un radiomarqueur, un marqueur biotine, un marqueur chromophore, un marqueur fluorophore, un marqueur ECL et un marqueur enzymatique.

**6.** Procédé selon la revendication 1, dans lequel le taux de FR$\alpha$ est déterminé en utilisant une technique choisie dans le groupe constitué de l'analyse Western blot, une analyse radioimmunologique, l'immunofluorimétrie, l'immunoprécipitation, la dialyse à l'équilibre, l'immunodiffusion, l'analyse en phase de solution, l'analyse immunologique par électrochimioluminescence (ECLIA) et l'analyse ELISA.

**7.** Procédé selon la revendication 1, dans lequel l'échantillon témoin comprend un taux témoin standardisé de FR$\alpha$ chez un sujet en bonne santé.

**8.** Procédé selon la revendication 1, dans lequel l'échantillon d'urine est (a) traité avec de la guanidine avant la détermination du taux de FR$\alpha$ dans l'échantillon d'urine ; ou (b) dilué avant la détermination du taux de FR$\alpha$ dans l'échantillon d'urine ; ou (c) centrifugé, passé au vortex, ou les deux, avant la détermination du taux de FR$\alpha$ dans l'échantillon d'urine.

**9.** Procédé selon la revendication 1, dans lequel le taux de FR$\alpha$ dans l'échantillon d'urine dérivé dudit sujet est estimé par la mise en contact de l'échantillon d'urine avec une paire d'anticorps choisis dans le groupe constitué de

(a) l'anticorps MOV18 immobilisé sur un support solide et l'anticorps MORAB-003 marqué,
(b) l'anticorps 9F3 immobilisé sur un support solide et l'anticorps 24F12 marqué,
(c) l'anticorps 26B3 immobilisé sur un support solide et l'anticorps 19D4 marqué, et
(d) l'anticorps 9F3 immobilisé sur un support solide et l'anticorps 26B3 marqué.

**10.** Utilisation d'un kit selon le procédé de l'une quelconque des revendications 1 à 9 pour estimer si un sujet est atteint d'un cancer exprimant le FRα chez un sujet,
le kit comprenant
un moyen pour déterminer le taux de récepteur alpha des folates (FRα) qui n'est pas lié à une cellule dans un échantillon d'urine dérivé dudit sujet ; et
des instructions pour l'utilisation du kit pour estimer si le sujet est atteint d'un cancer exprimant le FRα ou pour estimer la progression d'un cancer exprimant le FRα.

Figure 1

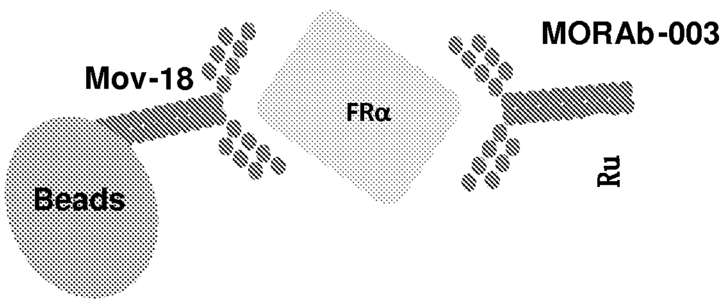

**Figure 2**

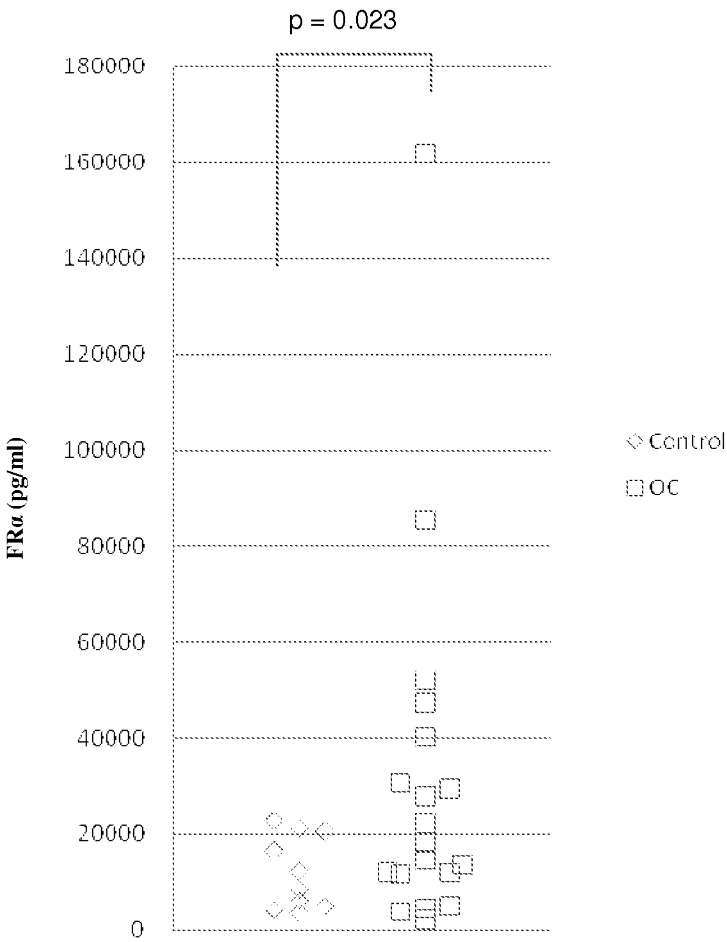
p = 0.023
180000
160000
140000
120000
100000
80000
60000
40000
20000
0
FRα (pg/ml)
Control
OC

Figure 3

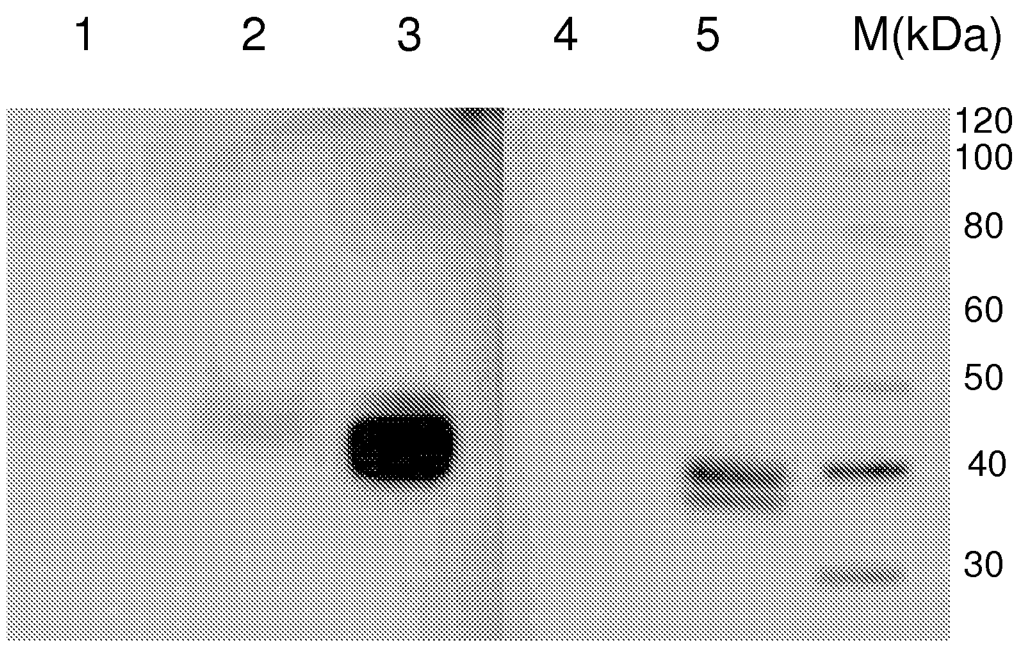

**Figure 4**

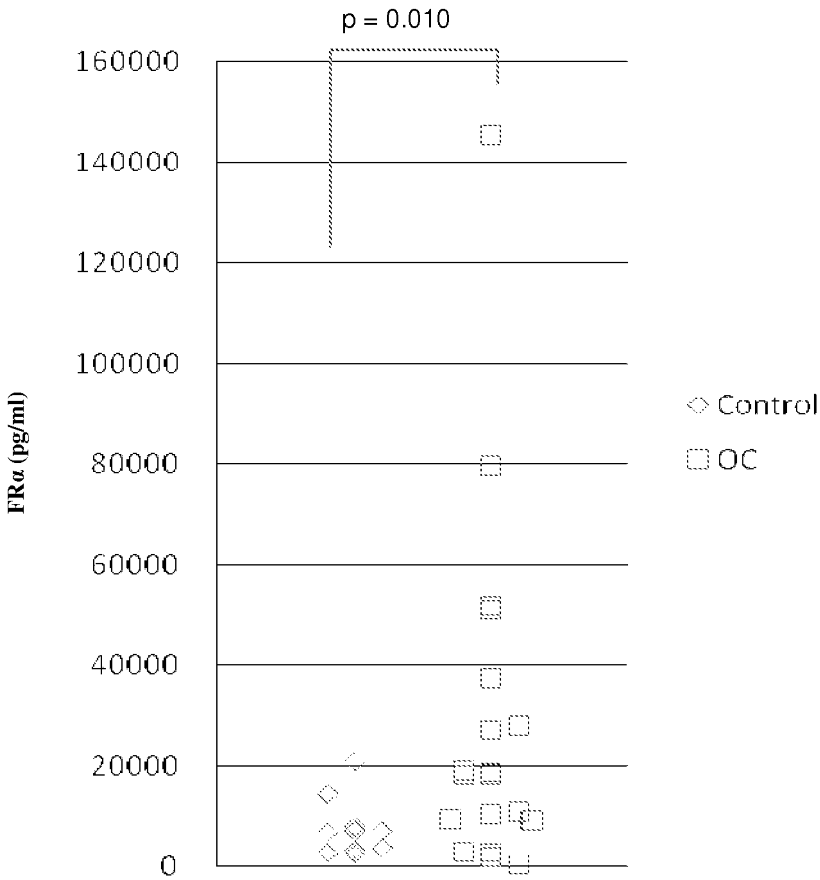
p = 0.010
160000
140000
120000
100000
80000
60000
40000
20000
0
FRα (pg/ml)
Control
OC

Figure 5

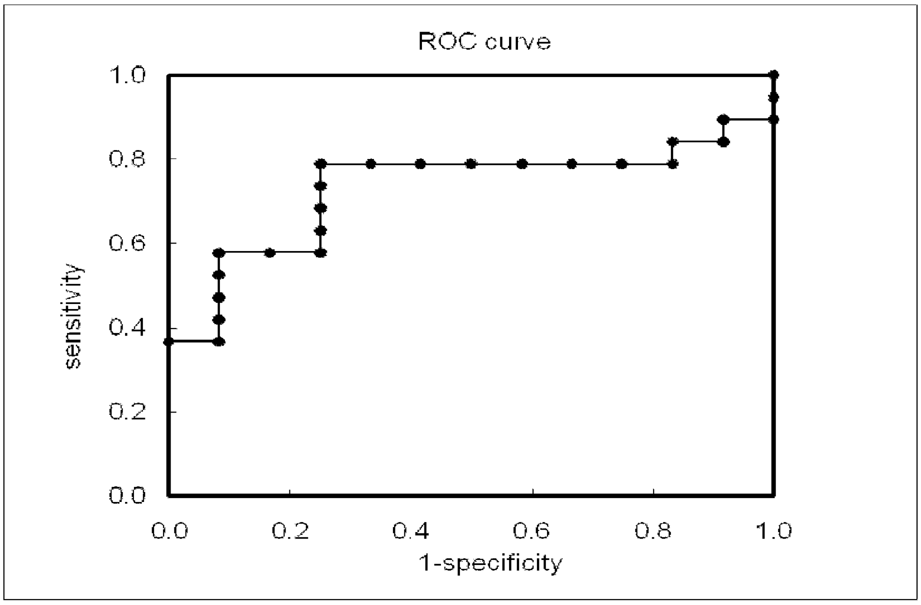


| | FRα (pg/mL) |
|---|---|
| Cut off | 9100 |

**Figure 6**

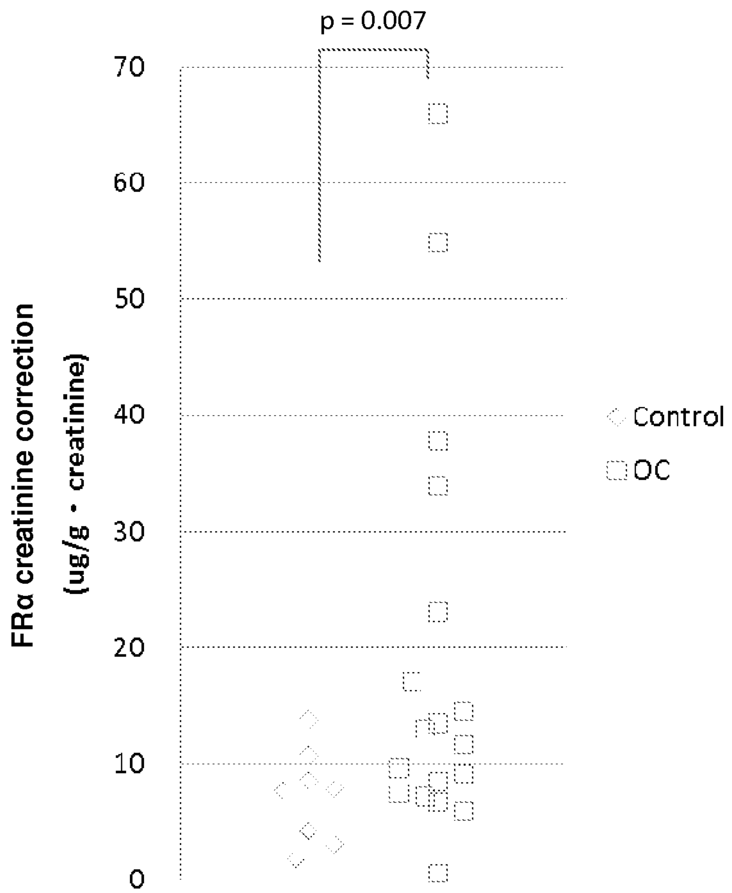


Fig. Distribution of FRα level revised by creatinine value in urine

Figure 7

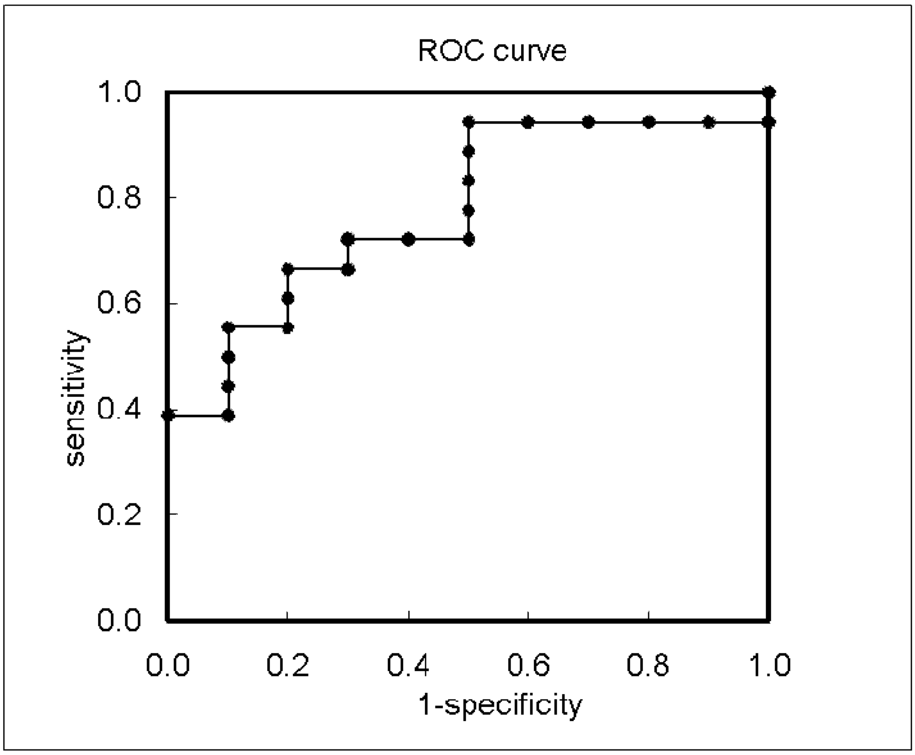
ROC curve
1.0
0.8
0.6
0.4
0.2
0.0
sensitivity
0.0
0.2
0.4
0.6
0.8
1.0
1-specificity

Figure 8

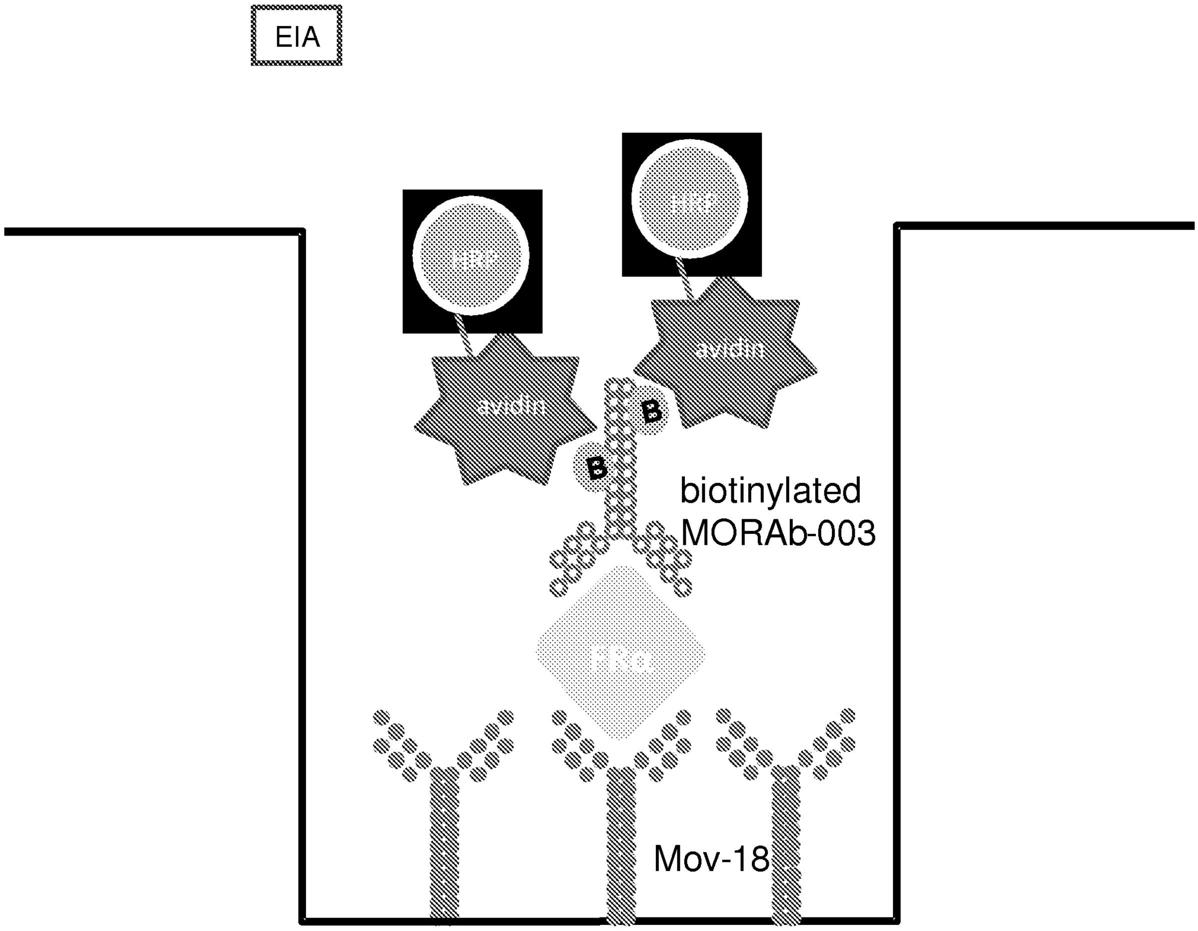
EIA
HRP
HRP
avidin
avidin
B
B
biotinylated
MORAb-003
FRα
Mov-18

## Figure 9

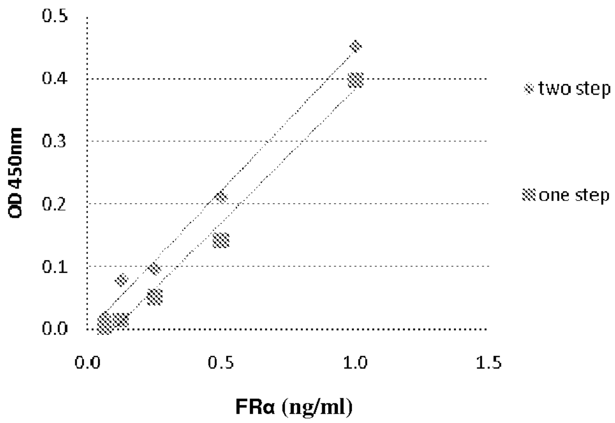


| | Two step | One step |
|---|---|---|
| Normal serum No. 1-S | 0.36pg/mL | 0.01pg/mL |

Figure 10

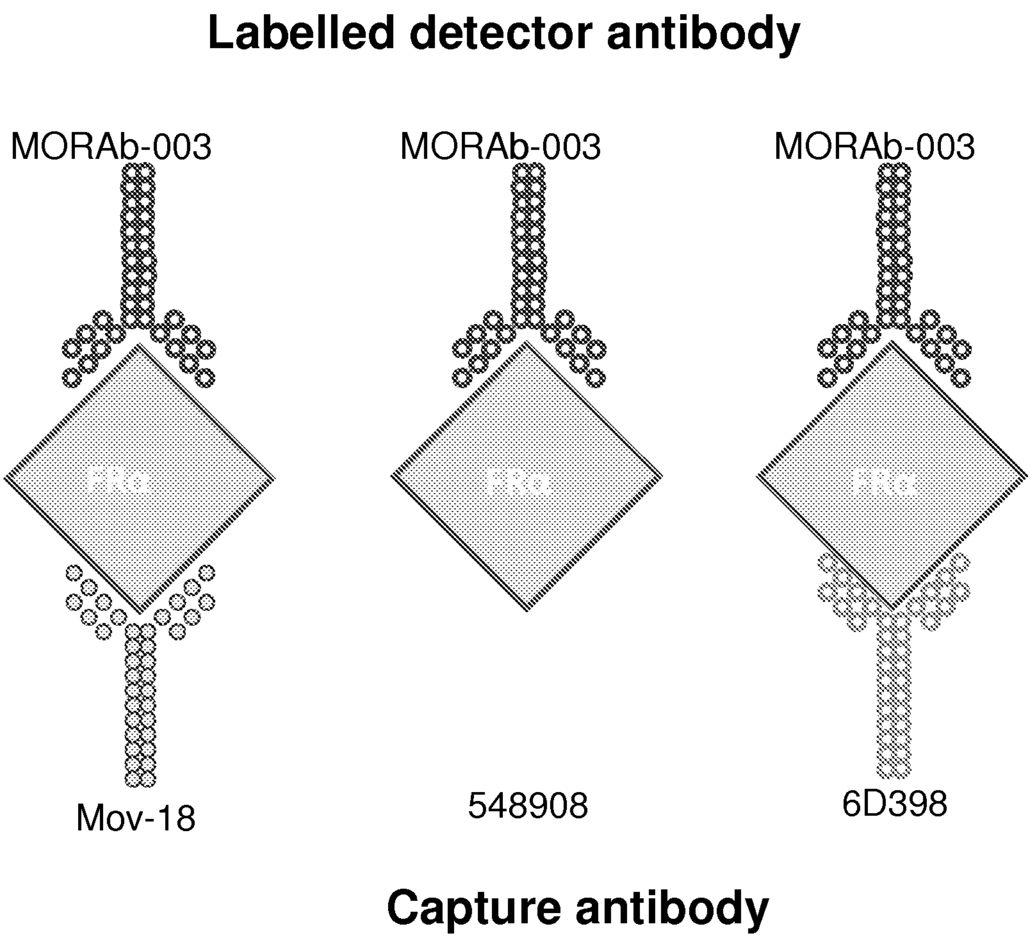
Labelled detector antibody
MORAb-003
MORAb-003
MORAb-003
FRα
FRα
FRα
Mov-18
548908
6D398
Capture antibody

# Figure 11

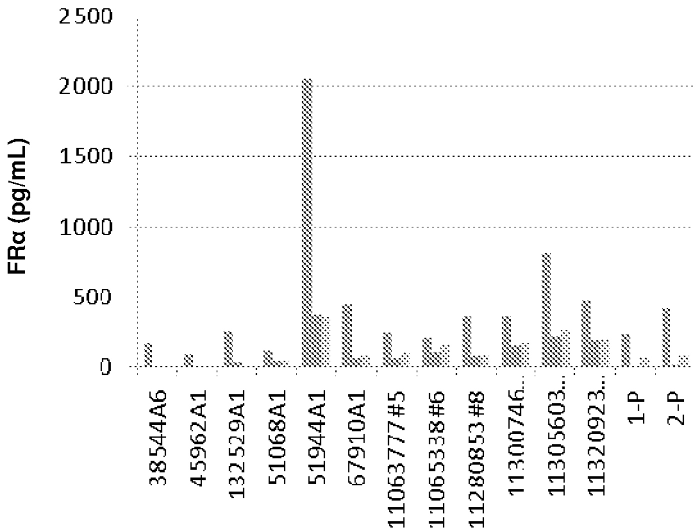
2500
2000
1500
1000
500
0
FRα (pg/mL)
38544A6
45962A1
132529A1
51068A1
51944A1
67910A1
11063777#5
11065338#6
11280853#8
11300746...
11305603...
11320923...
1-P
2-P

Figure 12

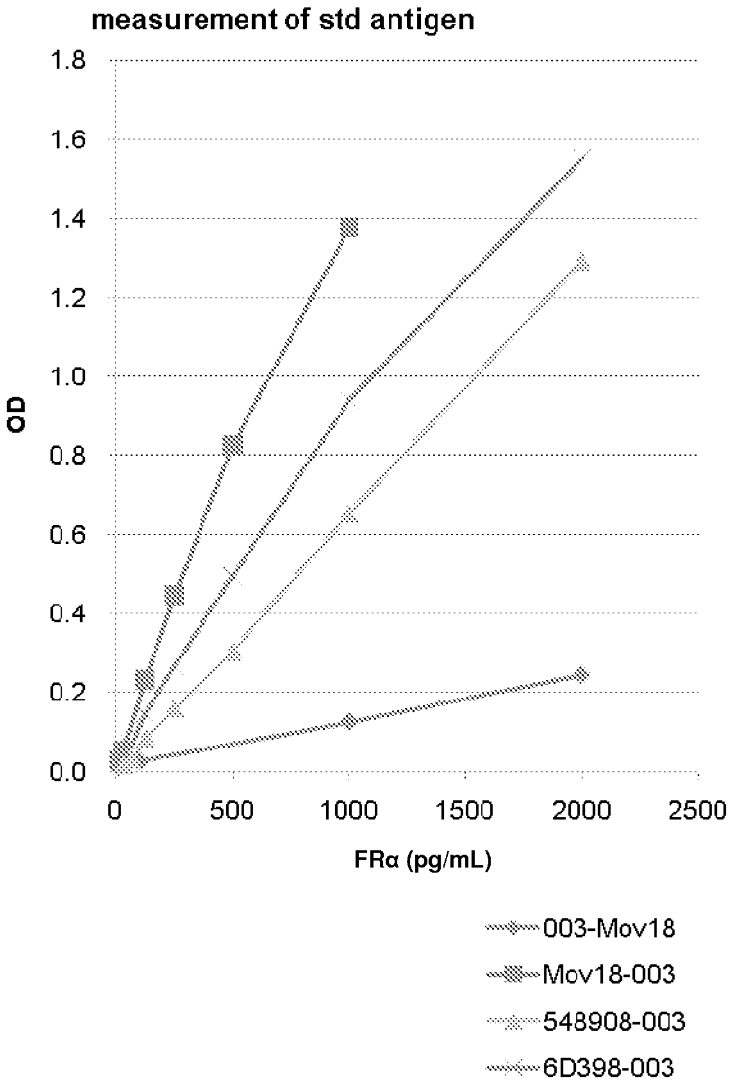
measurement of std antigen
1.8
1.6
1.4
1.2
1.0
0.8
0.6
0.4
0.2
0.0
OD
0
500
1000
1500
2000
2500
FRα (pg/mL)
003-Mov18
Mov18-003
548908-003
6D398-003

Figure 13

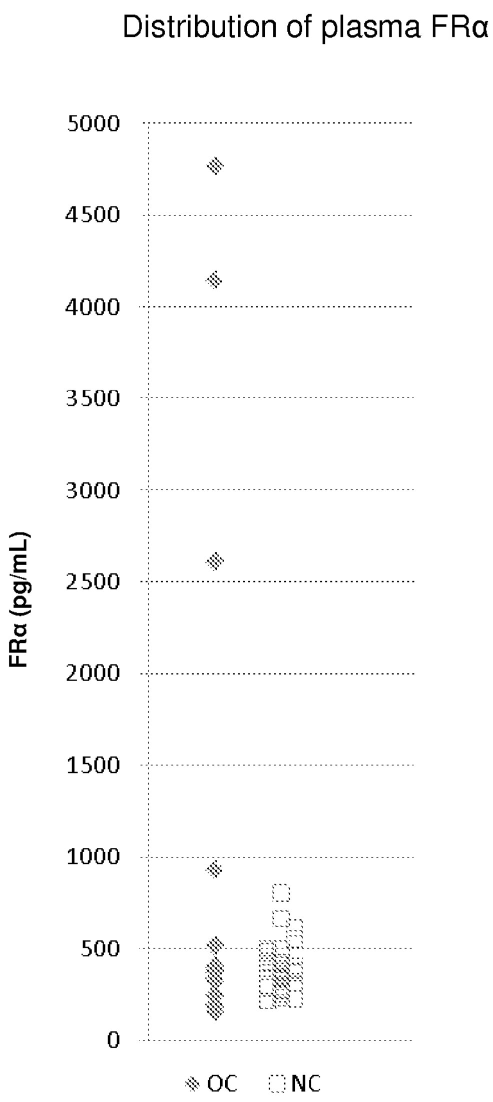
Distribution of plasma FRα
FRα (pg/mL)
5000
4500
4000
3500
3000
2500
2000
1500
1000
500
0
OC
NC

Figure 14

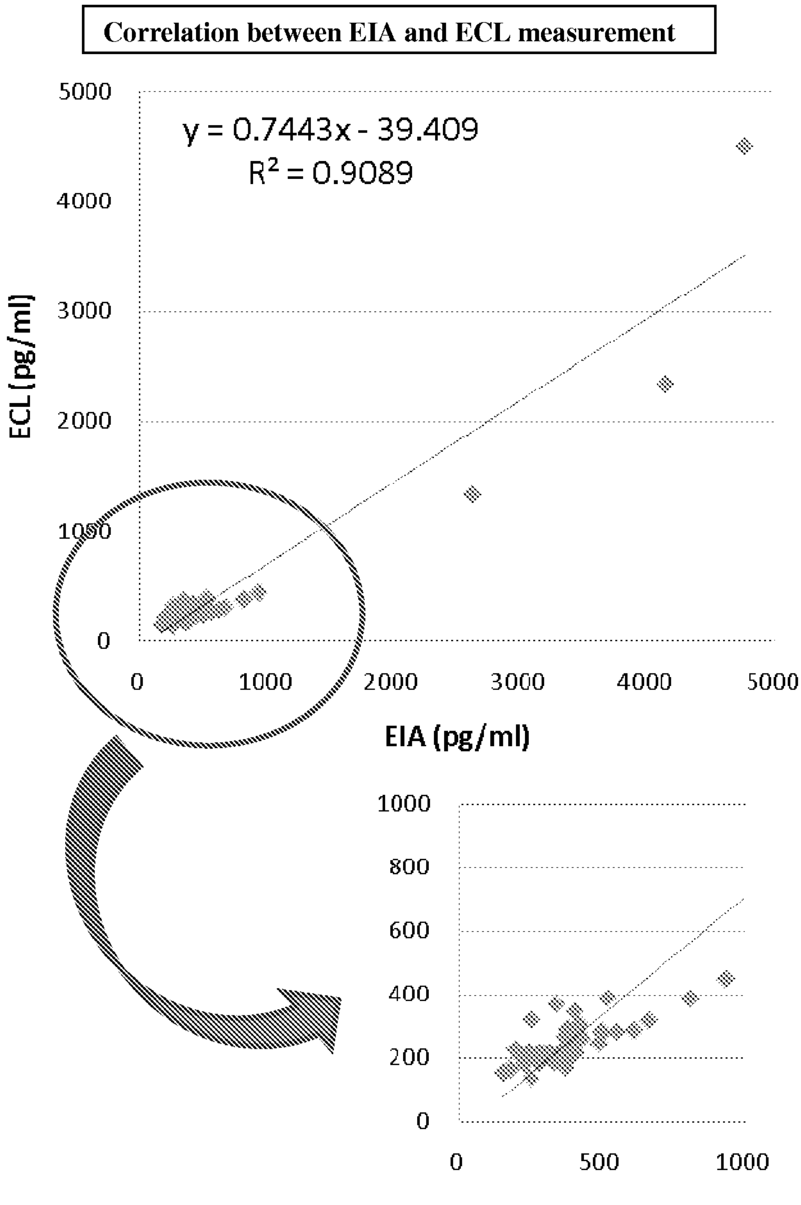
Correlation between EIA and ECL measurement
y = 0.7443x - 39.409
R² = 0.9089
ECL (pg/ml)
EIA (pg/ml)
0
1000
2000
3000
4000
5000
0
200
400
600
800
1000
500

Figure 15

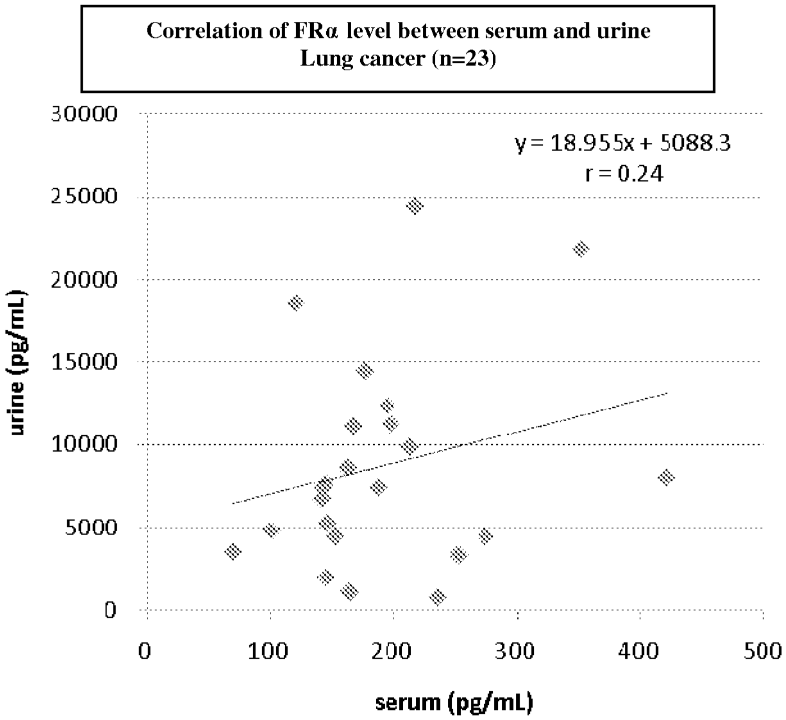
Correlation of FRα level between serum and urine
Lung cancer (n=23)
y = 18.955x + 5088.3
r = 0.24
urine (pg/mL)
serum (pg/mL)
30000
25000
20000
15000
10000
5000
0
0
100
200
300
400
500

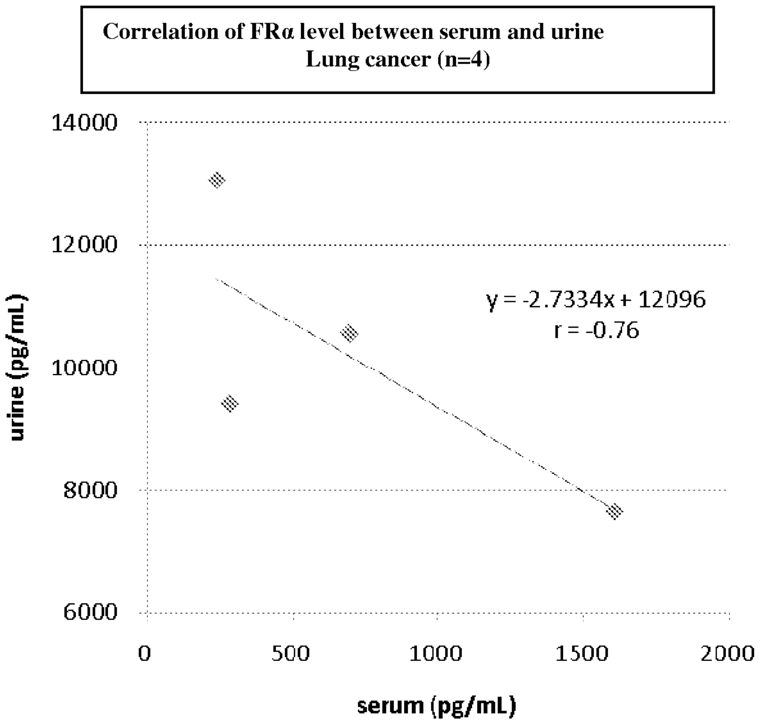
Correlation of FRα level between serum and urine
Lung cancer (n=4)
y = -2.7334x + 12096
r = -0.76
urine (pg/mL)
serum (pg/mL)
14000
12000
10000
8000
6000
0
500
1000
1500
2000

Figure 16

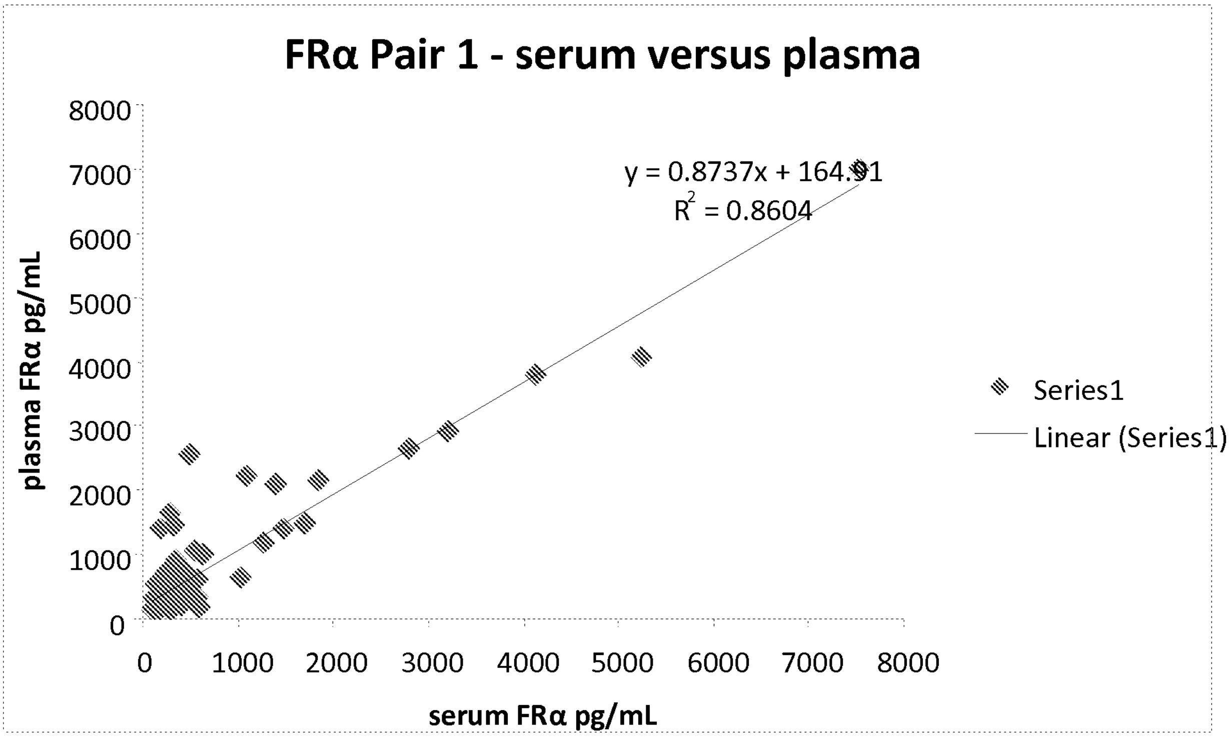
FRα Pair 1 - serum versus plasma
8000
7000
6000
5000
4000
3000
2000
1000
0
0
1000
2000
3000
4000
5000
6000
7000
8000
plasma FRα pg/mL
serum FRα pg/mL
y = 0.8737x + 164.91
R2 = 0.8604
Series1
Linear (Series1)

Figure 17

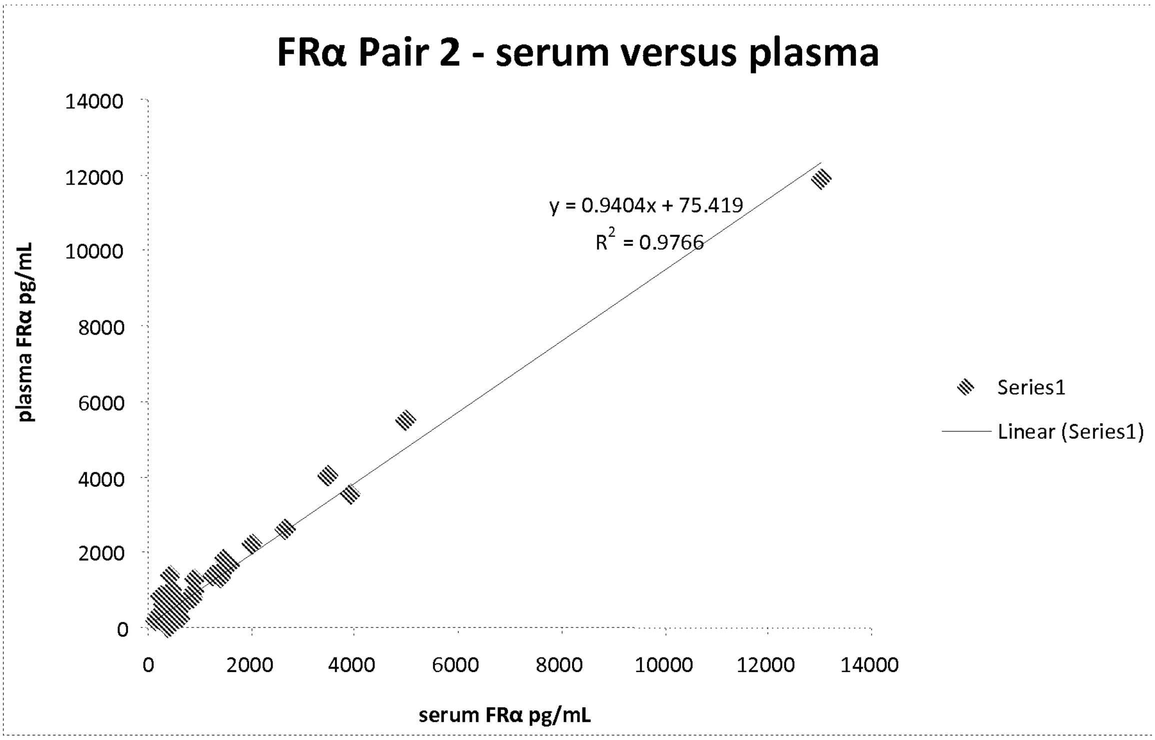
FRα Pair 2 - serum versus plasma
14000
12000
10000
8000
6000
4000
2000
0
plasma FRα pg/mL
y = 0.9404x + 75.419
R2 = 0.9766
0
2000
4000
6000
8000
10000
12000
14000
serum FRα pg/mL
Series1
Linear (Series1)

Figure 18

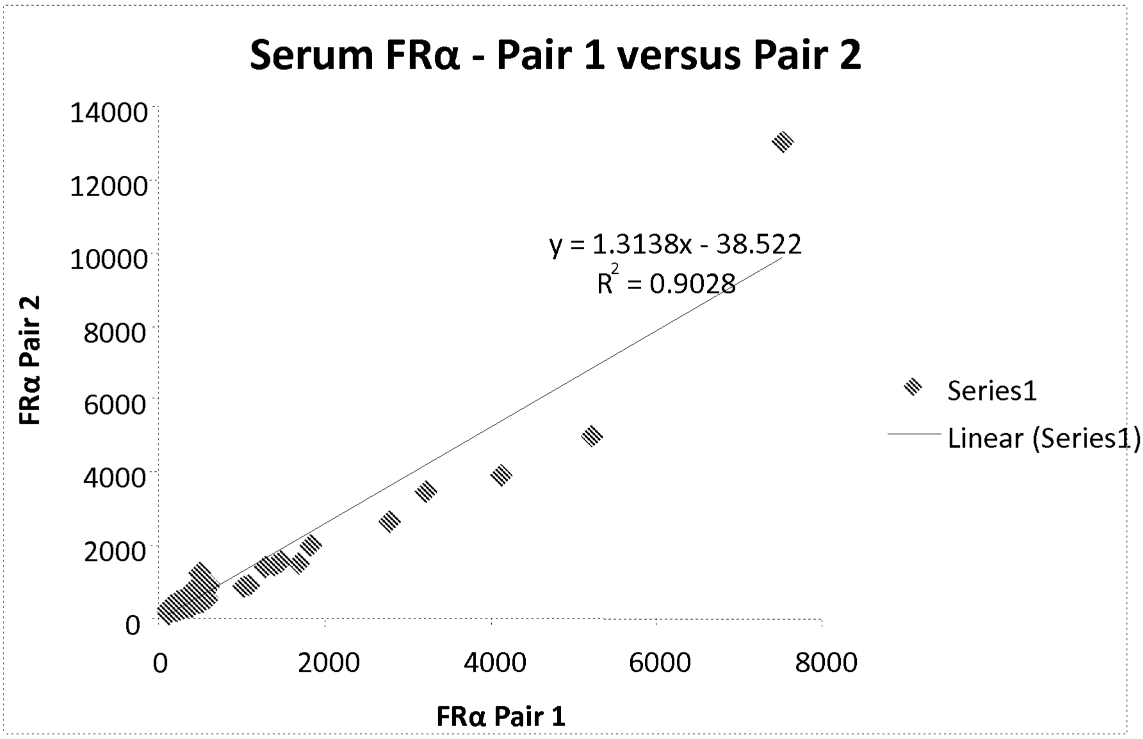
Serum FRα - Pair 1 versus Pair 2
14000
12000
10000
8000
6000
4000
2000
0
FRα Pair 2
y = 1.3138x - 38.522
R2 = 0.9028
Series1
Linear (Series1)
0
2000
4000
6000
8000
FRα Pair 1

Figure 19

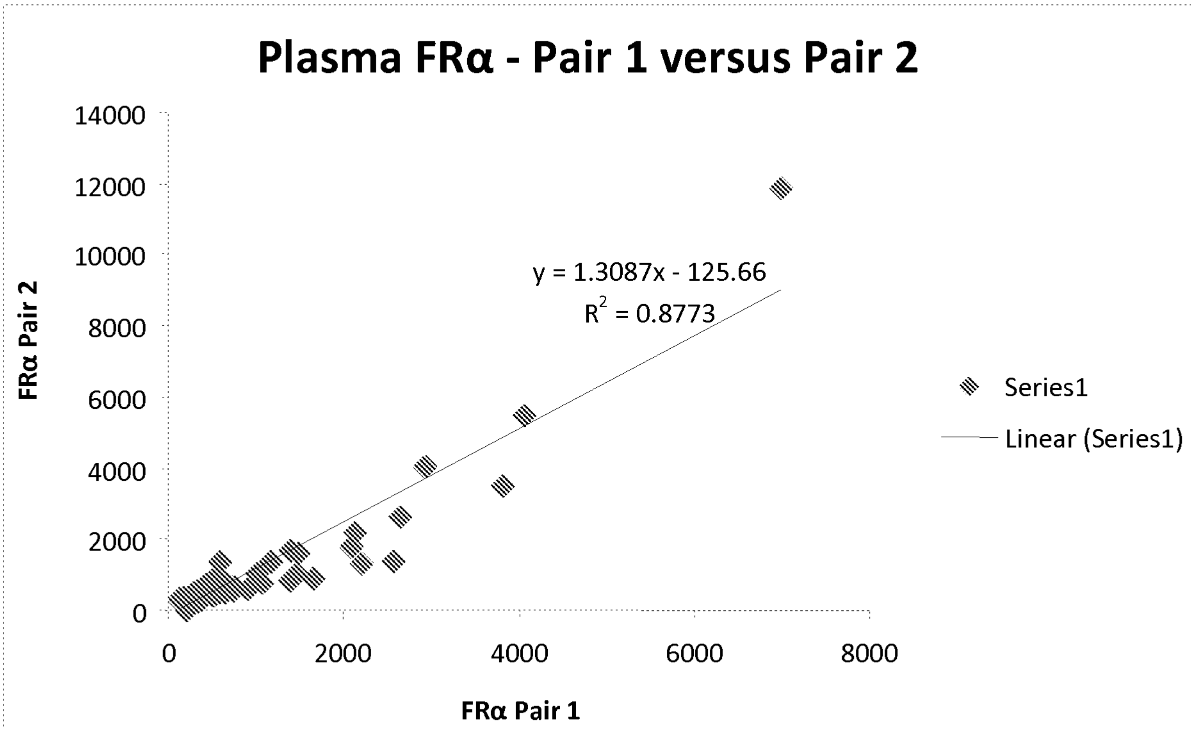
Plasma FRα - Pair 1 versus Pair 2
14000
12000
10000
8000
6000
4000
2000
0
0
2000
4000
6000
8000
FRα Pair 2
FRα Pair 1
y = 1.3087x - 125.66
R2 = 0.8773
Series1
Linear (Series1)

**Figure 20**

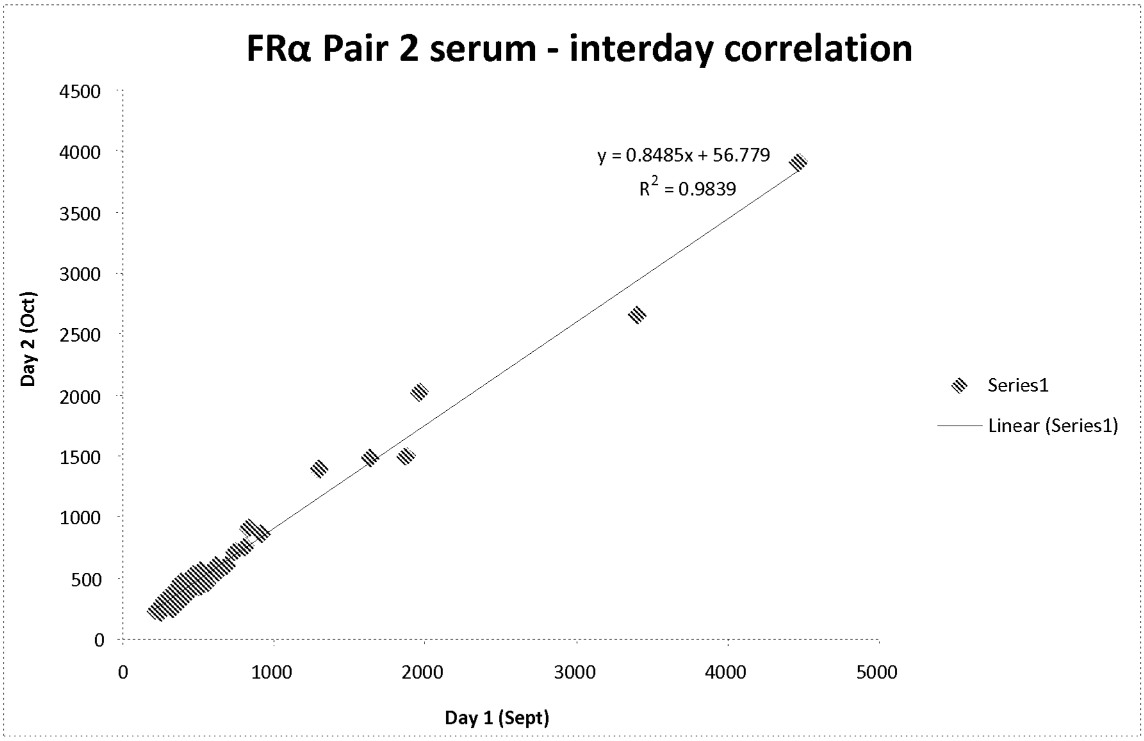
FRα Pair 2 serum - interday correlation
4500
4000
3500
3000
2500
2000
1500
1000
500
0
Day 2 (Oct)
0
1000
2000
3000
4000
5000
Day 1 (Sept)
y = 0.8485x + 56.779
R2 = 0.9839
Series1
Linear (Series1)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61410497 A
- US 61508444 A
- US 7754698 B
- US 20050232919 A
- WO 2009132081 A
- WO 9404678 A
- US 5759808 A
- US 7115396 B
- US 6818418 B
- US 6261804 B
- US 6258558 B
- WO 9831700 A, Szostak
- EP 86104170 A
- WO 2004113388 A
- US 5646253 A
- US 6124106 A
- US 5622824 A
- US 5605798 A
- US 5547835 A
- US 5223409 A
- US 2011059411 W
- US 61508444 B
- US 61410497 B


### Non-patent literature cited in the description


- **WEITMAN, SD et al.** *Cancer Res,* 1992, vol. 52, 3396-3401
- **WEITMAN SD et al.** *Cancer Res,* vol. 52, 6708-6711
- **BUENO R et al.** *J of Thoracic and Cardiovascular Surgery,* 2001, vol. 121 (2), 225-233
- **ELKANAT H ; RATNAM M.** *Frontiers in Bioscience,* 2006, vol. 11, 506-519
- **FISHER R.E.** *J Nucl Med,* 2008, vol. 49, 899-906
- **FRANKLIN, WA et al.** *Int J Cancer,* 1994, vol. 8, 89-95
- **HARTMAN L.C. et al.** *Int J Cancer,* 2007, vol. 121, 938-942
- **IWAKIRI S et al.** *Annals of Surgical Oncology,* vol. 15 (3), 889-899
- **PARKER N. et al.** *Analytical Biochemistry,* 2005, vol. 338, 284-293
- **SABA N.F. et al.** *Head Neck,* 2009, vol. 31 (4), 475-481
- **YANG R et al.** *Clin Cancer Res,* 2007, vol. 13, 2557-2567
- **RICHART et al.** *Am. J. Obstet. Gynecol.,* 1969, vol. 105, 386
- **SPIRA J ; ETTINGER, D.S.** Multidisciplinary management of lung cancer. *N Engl J Med,* 2004, vol. 350, 382
- AJCC Cancer Staging Manual. Springer-Verlag, 2002, 167-77
- International Union Against Cancer. TNM classification of malignant tumours. Wiley-Liss, 2002
- **R. SCOPES.** Protein Purification. Springer-Verlag, 1982
- Methods in Enzymology. **DEUTSCHER.** Guide to Protein Purification. Academic Press, Inc, 1990, vol. 182
- Principles And Practice Of Immunoassay. MacMillan, 1997
- Antibodies. A Laboratory Manual. Cold Spring Harbor Laboratory, 1988
- **WRIGHT, G.L., JR. et al.** *Expert Rev Mol Diagn,* 2002, vol. 2, 549
- **LI, J. et al.** *Clin Chem,* 2002, vol. 48, 1296
- **LARONGA, C. et al.** *Dis Markers,* 2003, vol. 19, 229
- **ADAM, B.L. et al.** *Cancer Res,* 2002, vol. 62, 3609
- **TOLSON, J. et al.** *Lab Invest,* 2004, vol. 84, 845
- **XIAO, Z. et al.** *Cancer Res,* 2001, vol. 61, 6029
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **STIJLEMANS et al.** *J. Biol. Chem.,* 2004, vol. 279, 1256-1261
- **DUMOULIN et al.** *Nature,* 2003, vol. 424, 783-788
- **PLESCHBERGER et al.** *Bioconjugate Chem.,* 2003, vol. 14, 440-448

- **CORTEZ-RETAMOZO et al.** *Int. J. Cancer,* 2002, vol. 89, 456-62
- **LAUWEREYS et al.** *EMBO J.,* 1998, vol. 17, 3512-3520
- **HOLLIGER et al.** *Proc. Natl. Acad. Sci.,* 1993, vol. 90, 6444-6448
- **POLJAK et al.** *Structure,* 1994, vol. 2, 1121-1123
- **HOLLIGER ; WINTER.** *Cancer Immunol. Immunother.,* 1997, vol. 45 (3-4), 128-30
- **WU et al.** *Immunotechnology,* 1996, vol. 2 (1), 21-36
- **HOLLIGER ; WINTER.** *Cancer Immunol. Immunother.,* 1997, vol. 45 (34), 128-30
- **PLUCKTHUN ; PACK.** *Immunotechnology,* 1997, vol. 3 (2), 83-105
- **RIDGWAY et al.** *Protein Eng.,* 1996, vol. 9 (7), 617-21
- **LU et al.** *J. Biol. Chem.,* 2004, vol. 279 (4), 2856-65
- **ROBERTS ; SZOSTAK.** *Proc. Natl. Acad. Sci,* 1997, vol. 94, 12297
- **MIOTTI, S. et al.** *Int J Cancer,* 1987, vol. 38, 297-303
- **CONEY et al.** *Cancer Res,* 1991, vol. 51, 6125-6132
- **BIBBO et al.** *Acta. Cytol.,* 2002, vol. 46, 25-29
- **SAQI et al.** *Diagn. Cytopathol.,* 2003, vol. 27, 365-370
- **BIBBO et al.** *Anal. Quant. Cytol. Histol.,* 2003, vol. 25, 8-11
- **HILL, T ; LEWICKI, P.** STATISTICS Methods and Applications. *StatSoft, Tulsa, OK,* 2006, www.evidence-based-medicine.co.uk
- **HARLOW ; LANE.** Antibodies: A Laboratory Manual. Cold Spring Harbor Laboratory, 1988
- **WEIR, D. M.** Handbook of Experimental Immunology. Blackwell Scientific, 1986
- **SCOUTEN, W. H.** *Methods in Enzymology,* 1987, vol. 135, 30-65
- **ZUCKERMANN et al.** *J. Med. Chem.,* 1994, vol. 37, 2678-85
- **LAM.** *Anticancer Drug Des.,* 1997, vol. 12, 145
- **DEWITT et al.** *Proc. Natl. Acad. Sci. U.S.A.,* 1993, vol. 90, 6909
- **ERB et al.** *Proc. Natl. Acad. Sci. USA,* 1994, vol. 91, 11422
- **ZUCKERMANN et al.** *J. Med. Chem.,* 1994, vol. 37, 2678
- **CHO et al.** *Science,* 1993, vol. 261, 1303
- **CARRELL et al.** *Angew. Chem. Int. Ed. Engl.,* 1994, vol. 33, 2059
- **CARELL et al.** *Angew. Chem. Int. Ed. Engl.,* vol. 33, 2061
- **GALLOP et al.** *J. Med. Chem.,* 1994, vol. 37, 1233
- **HOUGHTEN.** *Biotechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc Natl Acad Sci USA,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 87, 6378-6382
- **FELICI.** *J. Mol. Biol.,* 1991, vol. 222, 301-310
- **NAMBA et al.** *Analytical Science,* 1999, vol. 15, 1087-1093

SEQUENCE LISTING

<110> MORPHOTEK INC.

<120> FOLATE RECEPTOR ALPHA AS A DIAGNOSTIC AND PROGNOSTIC MARKER FOR FOLATE RECEPTOR ALPHA-EXPRESSING CANCERS

<130> 118557-01420

<140> PCT/US2011/059411
<141> 2011-11-04

<150> 61/508,444
<151> 2011-07-15

<150> 61/410,497
<151> 2010-11-05

<160> 90

<170> PatentIn version 3.5

<210> 1
<211> 10
<212> PRT
<213> Artificial Sequence

<220>
<221> source
<223> /note="Description of Artificial Sequence: Synthetic peptide"

<400> 1

```
Gly Phe Thr Phe Ser Gly Tyr Gly Leu Ser
1               5                   10
```

<210> 2
<211> 17
<212> PRT
<213> Artificial Sequence

<220>
<221> source
<223> /note="Description of Artificial Sequence: Synthetic peptide"

<400> 2

```
Met Ile Ser Ser Gly Gly Ser Tyr Thr Tyr Tyr Ala Asp Ser Val Lys
1               5                   10                  15

Gly
```

<210> 3
<211> 10
<212> PRT
<213> Artificial Sequence

<220>
<221> source
<223> /note="Description of Artificial Sequence: Synthetic peptide"

<400> 3

```
His Gly Asp Asp Pro Ala Trp Phe Ala Tyr
1               5                   10
```

<210> 4
<211> 12
<212> PRT
<213> Artificial Sequence

<220>
<221> source
<223> /note="Description of Artificial Sequence: Synthetic peptide"

<400> 4

```
Ser Val Ser Ser Ser Ile Ser Ser Asn Asn Leu His
1               5                   10
```

<210> 5
<211> 7
<212> PRT
<213> Artificial Sequence

<220>
<221> source
<223> /note="Description of Artificial Sequence: Synthetic peptide"

<400> 5

```
Gly Thr Ser Asn Leu Ala Ser
1               5
```

<210> 6
<211> 11
<212> PRT
<213> Artificial Sequence

<220>
<221> source
<223> /note="Description of Artificial Sequence: Synthetic peptide"

<400> 6

```
Gln Gln Trp Ser Ser Tyr Pro Tyr Met Tyr Thr
1               5                   10
```

<210> 7
<211> 449
<212> PRT
<213> Artificial Sequence

<220>
<221> source
<223> /note="Description of Artificial Sequence: Synthetic polypeptide"

<400> 7

```
Glu Val Gln Leu Val Glu Ser Gly Gly Gly Val Val Gln Pro Gly Arg
1               5                   10                  15
```

```
Ser Leu Arg Leu Ser Cys Ser Ala Ser Gly Phe Thr Phe Ser Gly Tyr
            20                  25                  30

Gly Leu Ser Trp Val Arg Gln Ala Pro Gly Lys Gly Leu Glu Trp Val
        35                  40                  45

Ala Met Ile Ser Ser Gly Gly Ser Tyr Thr Tyr Tyr Ala Asp Ser Val
    50                  55                  60

Lys Gly Arg Phe Ala Ile Ser Arg Asp Asn Ala Lys Asn Thr Leu Phe
65                  70                  75                  80

Leu Gln Met Asp Ser Leu Arg Pro Glu Asp Thr Gly Val Tyr Phe Cys
                85                  90                  95

Ala Arg His Gly Asp Asp Pro Ala Trp Phe Ala Tyr Trp Gly Gln Gly
            100                 105                 110

Thr Pro Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270
```

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 8
<211> 217
<212> PRT
<213> Artificial Sequence

<220>
<221> source
<223> /note="Description of Artificial Sequence: Synthetic polypeptide"

<400> 8

```
Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Pro Trp
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Tyr Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215
```

<210> 9
<211> 468
<212> PRT
<213> Artificial Sequence

<220>
<221> source

<223> /note="Description of Artificial Sequence: Synthetic polypeptide"

<400> 9

```
Met Gly Trp Ser Cys Ile Ile Leu Phe Leu Val Ala Thr Ala Thr Gly
1               5                   10                  15
```

```
Val His Ser Glu Val Gln Leu Val Glu Ser Gly Gly Gly Val Val Gln
            20                  25                  30

Pro Gly Arg Ser Leu Arg Leu Ser Cys Ser Ala Ser Gly Phe Thr Phe
        35                  40                  45

Ser Gly Tyr Gly Leu Ser Trp Val Arg Gln Ala Pro Gly Lys Gly Leu
    50                  55                  60

Glu Trp Val Ala Met Ile Ser Ser Gly Gly Ser Tyr Thr Tyr Tyr Ala
65                  70                  75                  80

Asp Ser Val Lys Gly Arg Phe Ala Ile Ser Arg Asp Asn Ala Lys Asn
                85                  90                  95

Thr Leu Phe Leu Gln Met Asp Ser Leu Arg Pro Glu Asp Thr Gly Val
            100                 105                 110

Tyr Phe Cys Ala Arg His Gly Asp Asp Pro Ala Trp Phe Ala Tyr Trp
        115                 120                 125

Gly Gln Gly Thr Pro Val Thr Val Ser Ser Ala Ser Thr Lys Gly Pro
    130                 135                 140

Ser Val Phe Pro Leu Ala Pro Ser Ser Lys Ser Thr Ser Gly Gly Thr
145                 150                 155                 160

Ala Ala Leu Gly Cys Leu Val Lys Asp Tyr Phe Pro Glu Pro Val Thr
                165                 170                 175

Val Ser Trp Asn Ser Gly Ala Leu Thr Ser Gly Val His Thr Phe Pro
            180                 185                 190

Ala Val Leu Gln Ser Ser Gly Leu Tyr Ser Leu Ser Ser Val Val Thr
        195                 200                 205

Val Pro Ser Ser Ser Leu Gly Thr Gln Thr Tyr Ile Cys Asn Val Asn
    210                 215                 220

His Lys Pro Ser Asn Thr Lys Val Asp Lys Lys Val Glu Pro Lys Ser
225                 230                 235                 240

Cys Asp Lys Thr His Thr Cys Pro Pro Cys Pro Ala Pro Glu Leu Leu
                245                 250                 255

Gly Gly Pro Ser Val Phe Leu Phe Pro Pro Lys Pro Lys Asp Thr Leu
            260                 265                 270

Met Ile Ser Arg Thr Pro Glu Val Thr Cys Val Val Val Asp Val Ser
```

275 280 285

His Glu Asp Pro Glu Val Lys Phe Asn Trp Tyr Val Asp Gly Val Glu
290 295 300

Val His Asn Ala Lys Thr Lys Pro Arg Glu Glu Gln Tyr Asn Ser Thr
305 310 315 320

Tyr Arg Val Val Ser Val Leu Thr Val Leu His Gln Asp Trp Leu Asn
325 330 335

Gly Lys Glu Tyr Lys Cys Lys Val Ser Asn Lys Ala Leu Pro Ala Pro
340 345 350

Ile Glu Lys Thr Ile Ser Lys Ala Lys Gly Gln Pro Arg Glu Pro Gln
355 360 365

Val Tyr Thr Leu Pro Pro Ser Arg Asp Glu Leu Thr Lys Asn Gln Val
370 375 380

Ser Leu Thr Cys Leu Val Lys Gly Phe Tyr Pro Ser Asp Ile Ala Val
385 390 395 400

Glu Trp Glu Ser Asn Gly Gln Pro Glu Asn Asn Tyr Lys Thr Thr Pro
405 410 415

Pro Val Leu Asp Ser Asp Gly Ser Phe Phe Leu Tyr Ser Lys Leu Thr
420 425 430

Val Asp Lys Ser Arg Trp Gln Gln Gly Asn Val Phe Ser Cys Ser Val
435 440 445

Met His Glu Ala Leu His Asn His Tyr Thr Gln Lys Ser Leu Ser Leu
450 455 460

Ser Pro Gly Lys
465

<210> 10
<211> 236
<212> PRT
<213> Artificial Sequence

<220>
<221> source
<223> /note="Description of Artificial Sequence: Synthetic polypeptide"

<400> 10

```
Met Gly Trp Ser Cys Ile Ile Leu Phe Leu Val Ala Thr Ala Thr Gly
1               5                   10                  15

Val His Ser Asp Ile Gln Leu Thr Gln Ser Pro Ser Ser Leu Ser Ala
            20                  25                  30

Ser Val Gly Asp Arg Val Thr Ile Thr Cys Ser Val Ser Ser Ser Ile
        35                  40                  45

Ser Ser Asn Asn Leu His Trp Tyr Gln Gln Lys Pro Gly Lys Ala Pro
    50                  55                  60

Lys Pro Trp Ile Tyr Gly Thr Ser Asn Leu Ala Ser Gly Val Pro Ser
65                  70                  75                  80

Arg Phe Ser Gly Ser Gly Ser Gly Thr Asp Tyr Thr Phe Thr Ile Ser
                85                  90                  95

Ser Leu Gln Pro Glu Asp Ile Ala Thr Tyr Tyr Cys Gln Gln Trp Ser
            100                 105                 110

Ser Tyr Pro Tyr Met Tyr Thr Phe Gly Gln Gly Thr Lys Val Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235
```

<210> 11
<211> 1407
<212> DNA

<213> Artificial Sequence

<220>
<221> source
<223> /note="Description of Artificial Sequence: Synthetic polynucleotide"

<400> 11

```
atgggatgga gctgtatcat cctcttcttg gtagcaacag ctacaggtgt ccactccgag      60
gtccaactgg tggagagcgg tggaggtgtt gtgcaacctg gccggtccct gcgcctgtcc     120
tgctccgcat ctggcttcac cttcagcggc tatgggttgt cttgggtgag acaggcacct     180
ggaaaaggtc ttgagtgggt tgcaatgatt agtagtggtg gtagttatac ctactatgca     240
gacagtgtga agggtagatt tgcaatatcg cgagacaacg ccaagaacac attgttcctg     300
caaatggaca gcctgagacc cgaagacacc ggggtctatt tttgtgcaag acatggggac     360
gatcccgcct ggttcgctta ttggggccaa gggaccccgg tcaccgtctc ctcagcctcc     420
accaagggcc catcggtctt ccccctggca ccctcctcca agagcacctc tgggggcaca     480
gcggccctgg gctgcctggt caaggactac ttccccgaac cggtgacggt gtcgtggaac     540
tcaggcgccc tgaccagcgg cgtgcacacc ttcccggctg tcctacagtc ctcaggactc     600
tactccctca gcagcgtggt gaccgtgccc tccagcagct tgggcaccca gacctacatc     660
tgcaacgtga atcacaagcc cagcaacacc aaggtggaca agaaagttga gcccaaatct     720
tgtgacaaaa ctcacacatg cccaccgtgc ccagcacctg aactcctggg gggaccgtca     780
gtcttcctct tccccccaaa acccaaggac accctcatga tctcccggac ccctgaggtc     840
acatgcgtgg tggtggacgt gagccacgaa gaccctgagg tcaagttcaa ctggtacgtg     900
gacggcgtgg aggtgcataa tgccaagaca aagccgcggg aggagcagta caacagcacg     960
taccgtgtgg tcagcgtcct caccgtcctg caccaggact ggctgaatgg caaggagtac    1020
aagtgcaagg tctccaacaa agccctccca gcccccatcg agaaaaccat ctccaaagcc    1080
aaagggcagc cccgagaacc acaggtgtac accctgcccc catcccggga tgagctgacc    1140
aagaaccagg tcagcctgac ctgcctggtc aaaggcttct atcccagcga catcgccgtg    1200
gagtgggaga gcaatgggca gccggagaac aactacaaga ccacgcctcc cgtgctggac    1260
tccgacggct ccttcttctt atattcaaag ctcaccgtgg acaagagcag gtggcagcag    1320
gggaacgtct tctcatgctc cgtgatgcat gaggctctgc acaaccacta cacgcagaag    1380
agcctctccc tgtctcccgg gaaatga                                        1407
```

<210> 12
<211> 711
<212> DNA
<213> Artificial Sequence

<220>
<221> source
<223> /note="Description of Artificial Sequence: Synthetic polynucleotide"

<400> 12

```
atgggatgga gctgtatcat cctcttcttg gtagcaacag ctacaggtgt ccactccgac        60

atccagctga cccagagccc aagcagcctg agcgccagcg tgggtgacag agtgaccatc       120

acctgtagtg tcagctcaag tataagttcc aacaacttgc actggtacca gcagaagcca       180

ggtaaggctc caaagccatg gatctacggc acatccaacc tggcttctgg tgtgccaagc       240

agattcagcg gtagcggtag cggtaccgac tacaccttca ccatcagcag cctccagcca       300

gaggacatcg ccacctacta ctgccaacag tggagtagtt acccgtacat gtacacgttc       360

ggccaaggga ccaaggtgga aatcaaacga actgtggctg caccatctgt cttcatcttc       420

ccgccatctg atgagcagtt gaaatctgga actgcctctg ttgtgtgcct gctgaataac       480

ttctatccca gagaggccaa agtacagtgg aaggtggata acgccctcca atcgggtaac       540

tcccaggaga gtgtcacaga gcaggacagc aaggacagca cctacagcct cagcagcacc       600

ctgacgctga gcaaagcaga ctacgagaaa cacaaagtct acgcctgcga agtcacccat       660

cagggcctga gctcgcccgt cacaaagagc ttcaacaggg gagagtgtta a                711
```

<210> 13
<211> 110
<212> PRT
<213> Artificial Sequence

<220>
<221> source
<223> /note="Description of Artificial Sequence: Synthetic polypeptide"

<400> 13

```
Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Leu Leu
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Phe Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys
            100                 105                 110
```

<210> 14
<211> 110
<212> PRT
<213> Artificial Sequence

<220>
<221> source
<223> /note="Description of Artificial Sequence: Synthetic polypeptide"

<400> 14

Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Leu Leu
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Tyr Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys
            100                 105                 110

<210> 15
<211> 110
<212> PRT
<213> Artificial Sequence

<220>
<221> source
<223> /note="Description of Artificial Sequence: Synthetic polypeptide"

<400> 15

Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Pro Trp
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Phe Thr Phe Thr Ile Ser Ser Leu Gln
65                  70                  75                  80

Pro Glu Asp Ile Ala Thr Tyr Tyr Cys Gln Gln Trp Ser Ser Tyr Pro
85 90 95

Tyr Met Tyr Thr Phe Gly Gln Gly Thr Lys Val Glu Ile Lys
100 105 110

<210> 16
<211> 110
<212> PRT
<213> Artificial Sequence

<220>
<221> source
<223> /note="Description of Artificial Sequence: Synthetic polypeptide"

<400> 16

Asp Ile Gln Leu Thr Gln Ser Pro Ser Ser Leu Ser Ala Ser Val Gly
1 5 10 15

Asp Arg Val Thr Ile Thr Cys Ser Val Ser Ser Ser Ile Ser Ser Asn
20 25 30

Asn Leu His Trp Tyr Gln Gln Lys Pro Gly Lys Ala Pro Lys Pro Trp
35 40 45

Ile Tyr Gly Thr Ser Asn Leu Ala Ser Gly Val Pro Ser Arg Phe Ser
50 55 60

Gly Ser Gly Ser Gly Thr Asp Tyr Thr Phe Thr Ile Ser Ser Leu Gln
65 70 75 80

Pro Glu Asp Ile Ala Thr Tyr Tyr Cys Gln Gln Trp Ser Ser Tyr Pro
85 90 95

Tyr Met Tyr Thr Phe Gly Gln Gly Thr Lys Val Glu Ile Lys
100 105 110

<210> 17
<211> 119
<212> PRT
<213> Artificial Sequence

<220>
<221> source
<223> /note="Description of Artificial Sequence: Synthetic polypeptide"

<400> 17

Gln Val Gln Leu Gln Glu Ser Gly Pro Gly Leu Val Arg Pro Ser Gln
1 5 10 15

Thr Leu Ser Leu Thr Cys Thr Ala Ser Gly Phe Thr Phe Ser Gly Tyr

20 25 30

Gly Leu Ser Trp Val Arg Gln Pro Pro Gly Arg Gly Leu Glu Trp Val
35 40 45

Ala Met Ile Ser Ser Gly Gly Ser Tyr Thr Tyr Tyr Ala Asp Ser Val
50 55 60

Lys Gly Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg His Gly Asp Asp Pro Ala Trp Phe Ala Tyr Trp Gly Gln Gly
100 105 110

Ser Leu Val Thr Val Ser Ser
115

<210> 18
<211> 119
<212> PRT
<213> Artificial Sequence

<220>
<221> source
<223> /note="Description of Artificial Sequence: Synthetic polypeptide"

<400> 18

Gln Val Gln Leu Gln Glu Ser Gly Pro Gly Leu Val Arg Pro Ser Gln
1 5 10 15

Thr Leu Ser Leu Thr Cys Thr Ala Ser Gly Phe Thr Phe Ser Gly Tyr
20 25 30

Gly Leu Ser Trp Val Arg Gln Pro Pro Gly Arg Gly Leu Glu Trp Val
35 40 45

Ala Met Ile Ser Ser Gly Gly Ser Tyr Thr Tyr Tyr Ala Asp Ser Val
50 55 60

Lys Gly Arg Phe Ala Ile Ser Arg Asp Asn Ser Lys Asn Gln Phe Ser
65 70 75 80

Leu Arg Leu Ser Ser Val Thr Ala Ala Asp Thr Ala Val Tyr Tyr Cys
85 90 95

Ala Arg His Gly Asp Asp Pro Ala Trp Phe Ala Tyr Trp Gly Gln Gly
100 105 110

Ser Leu Val Thr Val Ser Ser
115

<210> 19
<211> 119
<212> PRT
<213> Artificial Sequence

<220>
<221> source
<223> /note="Description of Artificial Sequence: Synthetic polypeptide"

<400> 19

Gln Val Gln Leu Gln Glu Ser Gly Pro Gly Leu Val Arg Pro Ser Gln
1 5 10 15

Thr Leu Ser Leu Thr Cys Thr Ala Ser Gly Phe Thr Phe Ser Gly Tyr
20 25 30

Gly Leu Ser Trp Val Arg Gln Pro Pro Gly Arg Gly Leu Glu Trp Val
35 40 45

Ala Met Ile Ser Ser Gly Gly Ser Tyr Thr Tyr Tyr Ala Asp Ser Val
50 55 60

Lys Gly Arg Val Thr Met Leu Arg Asp Thr Ser Lys Asn Ser Leu Phe
65 70 75 80

Leu Arg Leu Ser Ser Val Thr Ala Ala Asp Thr Ala Val Tyr Tyr Cys
85 90 95

Ala Arg His Gly Asp Asp Pro Ala Trp Phe Ala Tyr Trp Gly Gln Gly
100 105 110

Thr Thr Val Thr Val Ser Ser
115

<210> 20
<211> 119
<212> PRT
<213> Artificial Sequence

<220>
<221> source
<223> /note="Description of Artificial Sequence: Synthetic polypeptide"

<400> 20

Gln Val Gln Leu Gln Glu Ser Gly Pro Gly Leu Val Arg Pro Ser Gln
1 5 10 15

Thr Leu Ser Leu Thr Cys Thr Ala Ser Gly Phe Thr Phe Ser Gly Tyr
20 25 30

Gly Leu Ser Trp Val Arg Gln Pro Pro Gly Arg Gly Leu Glu Trp Val
35 40 45

Ala Met Ile Ser Ser Gly Gly Ser Tyr Thr Tyr Tyr Ala Asp Ser Val
50 55 60

Lys Gly Arg Val Thr Met Leu Arg Asp Thr Ser Lys Asn Gln Phe Ser
65 70 75 80

Leu Arg Leu Ser Ser Val Thr Ala Ala Asp Thr Ala Ile Tyr Ile Cys
85 90 95

Ala Arg His Gly Asp Asp Pro Ala Trp Phe Ala Tyr Trp Gly Gln Gly
100 105 110

Ser Leu Val Thr Val Ser Ser
115

<210> 21
<211> 119
<212> PRT
<213> Artificial Sequence

<220>
<221> source
<223> /note="Description of Artificial Sequence: Synthetic polypeptide"

<400> 21

Glu Val Gln Leu Val Glu Ser Gly Gly Gly Val Val Gln Pro Gly Arg
1 5 10 15

Ser Leu Arg Leu Ser Cys Ser Ala Ser Gly Phe Thr Phe Ser Gly Tyr
20 25 30

Gly Leu Ser Trp Val Arg Gln Ala Pro Gly Lys Gly Leu Glu Trp Val
35 40 45

Ala Met Ile Ser Ser Gly Gly Ser Tyr Thr Tyr Tyr Ala Asp Ser Val
50 55 60

Lys Gly Arg Phe Ala Ile Ser Arg Asp Asn Ala Lys Asn Thr Leu Phe
65 70 75 80

Leu Gln Met Asp Ser Leu Arg Pro Glu Asp Thr Gly Val Tyr Phe Cys
85 90 95

Ala Arg His Gly Asp Asp Pro Ala Trp Phe Ala Tyr Trp Gly Gln Gly
100 105 110

Thr Pro Val Thr Val Ser Ser
115

<210> 22
<211> 119
<212> PRT
<213> Mus sp.

<220>
<221> MOD_RES
<222> (3)..(3)
<223> Any amino acid

<220>
<221> MOD_RES
<222> (6) .. (6)
<223> Any amino acid

<400> 22

Gln Val Xaa Leu Gln Xaa Ser Gly Gly Asp Leu Val Lys Pro Gly Gly
1 5 10 15

Ser Leu Lys Leu Ser Cys Ala Ala Ser Gly Phe Thr Phe Ser Gly Tyr
20 25 30

Gly Leu Ser Trp Val Arg Gln Thr Pro Asp Lys Arg Leu Glu Trp Val
35 40 45

Ala Met Ile Ser Ser Gly Gly Ser Tyr Thr Tyr Tyr Ala Asp Ser Val
50 55 60

Lys Gly Arg Phe Ala Ile Ser Arg Asp Asn Ala Lys Asn Ser Leu Phe
65 70 75 80

Leu Gln Met Ser Ser Leu Lys Ser Asp Asp Thr Ala Ile Tyr Ile Cys
85 90 95

Ala Arg His Gly Asp Asp Pro Ala Trp Phe Ala Tyr Trp Gly Gln Gly
100 105 110

Thr Leu Val Thr Val Ser Ala
115

<210> 23
<211> 110
<212> PRT
<213> Mus sp.

<400> 23

```
Asp Ile Glu Leu Thr Gln Ser Pro Ala Leu Met Ala Ala Ser Pro Gly
1               5                   10                  15

Glu Lys Val Thr Ile Thr Cys Ser Val Ser Ser Ser Ile Ser Ser Asn
            20                  25                  30

Asn Leu His Trp Tyr Gln Gln Lys Ser Glu Thr Ser Pro Lys Pro Trp
        35                  40                  45

Ile Tyr Gly Thr Ser Asn Leu Ala Ser Gly Val Pro Leu Arg Phe Arg
    50                  55                  60

Gly Phe Gly Ser Gly Thr Ser Tyr Ser Leu Thr Ile Ser Ser Met Glu
65                  70                  75                  80

Ala Glu Asp Ala Ala Thr Tyr Tyr Cys Gln Gln Trp Ser Ser Tyr Pro
                85                  90                  95

Tyr Met Tyr Thr Phe Gly Gly Gly Thr Lys Leu Glu Ile Lys
            100                 105                 110
```

<210> 24
<211> 962
<212> DNA
<213> Homo sapiens

<400> 24

```
tcaaggttaa acgacaagga cagacatggc tcagcggatg acaacacagc tgctgctcct    60
tctagtgtgg gtggctgtag taggggaggc tcagacaagg attgcatggg ccaggactga   120
gcttctcaat gtctgcatga acgccaagca ccacaaggaa aagccaggcc ccgaggacaa   180
gttgcatgag cagtgtcgac cctggaggaa gaatgcctgc tgttctacca acaccagcca   240
ggaagcccat aaggatgttt cctacctata tagattcaac tggaaccact gtggagagat   300
ggcacctgcc tgcaaacggc atttcatcca ggacacctgc ctctacgagt gctcccccaa   360
cttggggccc tggatccagc aggtggatca gagctggcgc aaagagcggg tactgaacgt   420
gcccctgtgc aaagaggact gtgagcaatg gtgggaagat tgtcgcacct cctacacctg   480
caagagcaac tggcacaagg gctggaactg gacttcaggg tttaacaagt gcgcagtggg   540
agctgcctgc caacctttcc atttctactt ccccacaccc actgttctgt gcaatgaaat   600
ctggactcac tcctacaagg tcagcaacta cagccgaggg agtggccgct gcatccagat   660
gtggttcgac ccagcccagg gcaaccccaa tgaggaggtg gcgaggttct atgctgcagc   720
catgagtggg gctgggccct gggcagcctg gcctttcctg cttagcctgg ccctaatgct   780
gctgtggctg ctcagctgac ctccttttac cttctgatac ctggaaatcc ctgccctgtt   840
cagccccaca gctcccaact atttggttcc tgctccatgg tcgggcctct gacagccact   900
ttgaataaac cagacaccgc acatgtgtct tgagaattat ttggaaaaaa aaaaaaaaaa   960
aa                                                                  962
```

<210> 25
<211> 257
<212> PRT
<213> Homo sapiens

<400> 25

```
Met Ala Gln Arg Met Thr Thr Gln Leu Leu Leu Leu Leu Val Trp Val
1               5                   10                  15

Ala Val Val Gly Glu Ala Gln Thr Arg Ile Ala Trp Ala Arg Thr Glu
            20                  25                  30

Leu Leu Asn Val Cys Met Asn Ala Lys His His Lys Glu Lys Pro Gly
        35                  40                  45

Pro Glu Asp Lys Leu His Glu Gln Cys Arg Pro Trp Arg Lys Asn Ala
    50                  55                  60

Cys Cys Ser Thr Asn Thr Ser Gln Glu Ala His Lys Asp Val Ser Tyr
65                  70                  75                  80

Leu Tyr Arg Phe Asn Trp Asn His Cys Gly Glu Met Ala Pro Ala Cys
                85                  90                  95

Lys Arg His Phe Ile Gln Asp Thr Cys Leu Tyr Glu Cys Ser Pro Asn
            100                 105                 110

Leu Gly Pro Trp Ile Gln Gln Val Asp Gln Ser Trp Arg Lys Glu Arg
        115                 120                 125

Val Leu Asn Val Pro Leu Cys Lys Glu Asp Cys Glu Gln Trp Trp Glu
    130                 135                 140

Asp Cys Arg Thr Ser Tyr Thr Cys Lys Ser Asn Trp His Lys Gly Trp
145                 150                 155                 160

Asn Trp Thr Ser Gly Phe Asn Lys Cys Ala Val Gly Ala Ala Cys Gln
                165                 170                 175

Pro Phe His Phe Tyr Phe Pro Thr Pro Thr Val Leu Cys Asn Glu Ile
            180                 185                 190

Trp Thr His Ser Tyr Lys Val Ser Asn Tyr Ser Arg Gly Ser Gly Arg
        195                 200                 205

Cys Ile Gln Met Trp Phe Asp Pro Ala Gln Gly Asn Pro Asn Glu Glu
    210                 215                 220

Val Ala Arg Phe Tyr Ala Ala Ala Met Ser Gly Ala Gly Pro Trp Ala
225                 230                 235                 240

Ala Trp Pro Phe Leu Leu Ser Leu Ala Leu Met Leu Leu Trp Leu Leu
```

245 250 255

Ser

<210> 26
<211> 27
<212> PRT
<213> Artificial Sequence

<220>
<221> source
<223> /note="Description of Artificial Sequence: Synthetic peptide"

<220>
<221> MOD_RES
<222> (7)..(7)
<223> Any amino acid

<220>
<221> VARIANT
<222> (12)..(12)
<223> /replace="His"

<220>
<221> misc_feature
<222> (12)..(12)
<223> /note="Residue given in the sequence has no preference with respect to the annotation for said positions"

<220>
<221> MOD_RES
<222> (13)..(13)
<223> Any amino acid

<220>
<221> MOD_RES
<222> (18)..(18)
<223> Any amino acid

<220>
<221> MOD_RES
<222> (20)..(20)
<223> Any amino acid

<220>
<221> VARIANT
<222> (21) .. (21)
<223> /replace="Glu"

<220>
<221> misc_feature
<222> (21) .. (21)
<223> /note="Residue given in the sequence has no preference with respect to the annotation for said positions"

<220>
<221> MOD_RES
<222> (24) .. (25)
<223> Any amino acid

<220>
<221> MOD_RES
<222> (27) .. (27)
<223> Any amino acid

<400> 26

```
Thr Glu Leu Leu Asn Val Xaa Met Asn Ala Lys Trp Xaa Lys Glu Lys
1               5                   10                  15

Pro Xaa Pro Xaa Asp Lys Leu Xaa Xaa Gln Xaa
            20                  25
```

<210> 27
<211> 12
<212> PRT
<213> Artificial Sequence

<220>
<221> source
<223> /note="Description of Artificial Sequence: Synthetic peptide"

<400> 27

```
Arg Ala Ser Ser Thr Val Ser Tyr Ser Tyr Leu His
1               5                   10
```

<210> 28
<211> 7
<212> PRT
<213> Artificial Sequence

<220>
<221> source
<223> /note="Description of Artificial Sequence: Synthetic peptide"

<400> 28

```
Gly Thr Ser Asn Leu Ala Ser
1               5
```

<210> 29
<211> 9
<212> PRT
<213> Artificial Sequence

<220>
<221> source
<223> /note="Description of Artificial Sequence: Synthetic peptide"

<400> 29

```
Gln Gln Tyr Ser Gly Tyr Pro Leu Thr
1               5
```

<210> 30
<211> 107

<212> PRT
<213> Artificial Sequence

<220>
<221> source
<223> /note="Description of Artificial Sequence: Synthetic polypeptide"

<400> 30

```
Pro Ala Ile Met Ser Ala Ser Pro Gly Glu Lys Val Thr Met Thr Cys
1               5                   10                  15

Arg Ala Ser Ser Thr Val Ser Tyr Ser Tyr Leu His Trp Tyr Gln Gln
            20                  25                  30

Lys Ser Gly Ala Ser Pro Gln Leu Trp Ile Tyr Gly Thr Ser Asn Leu
        35                  40                  45

Ala Ser Gly Val Pro Ala Arg Phe Ser Gly Ser Gly Ser Gly Thr Ser
    50                  55                  60

Tyr Ser Leu Thr Ile Ser Ser Val Glu Ala Glu Asp Ala Ala Thr Tyr
65                  70                  75                  80

Tyr Cys Gln Gln Tyr Ser Gly Tyr Pro Leu Thr Phe Gly Ala Gly Thr
                85                  90                  95

Lys Leu Glu Leu Lys Arg Ala Asp Ala Ala Pro
            100                 105
```

<210> 31
<211> 6
<212> PRT
<213> Artificial Sequence

<220>
<221> source
<223> /note="Description of Artificial Sequence: Synthetic peptide"

<400> 31

```
Ser Gly Tyr Tyr Trp Asn
1               5
```

<210> 32
<211> 16
<212> PRT
<213> Artificial Sequence

<220>
<221> source
<223> /note="Description of Artificial Sequence: Synthetic peptide"

<400> 32

Tyr Ile Lys Ser Asp Gly Ser Asn Asn Tyr Asn Pro Ser Leu Lys Asn
1 5 10 15

<210> 33
<211> 7
<212> PRT
<213> Artificial Sequence

<220>
<221> source
<223> /note="Description of Artificial Sequence: Synthetic peptide"

<400> 33

Glu Trp Lys Ala Met Asp Tyr
1 5

<210> 34
<211> 129
<212> PRT
<213> Artificial Sequence

<220>
<221> source
<223> /note="Description of Artificial Sequence: Synthetic polypeptide"

<400> 34

Glu Ser Gly Pro Gly Leu Val Arg Pro Ser Gln Ser Leu Ser Leu Thr
1 5 10 15

Cys Ser Val Thr Gly Tyr Ser Ile Thr Ser Gly Tyr Tyr Trp Asn Trp
20 25 30

Ile Arg Gln Phe Pro Gly Ser Arg Leu Glu Trp Met Gly Tyr Ile Lys
35 40 45

Ser Asp Gly Ser Asn Asn Tyr Asn Pro Ser Leu Lys Asn Arg Ile Ser
50 55 60

Ile Thr Arg Asp Thr Ser Lys Asn Gln Phe Phe Leu Lys Leu Asn Ser
65 70 75 80

Val Thr Thr Glu Asp Thr Ala Thr Tyr Phe Cys Thr Arg Glu Trp Lys
85 90 95

Ala Met Asp Tyr Trp Gly Gln Gly Thr Ser Val Thr Val Ser Ser Ala
100 105 110

Lys Thr Thr Pro Pro Ser Val Tyr Pro Leu Ala Pro Gly Cys Gly Asp
115 120 125

Thr

<210> 35
<211> 15
<212> PRT
<213> Artificial Sequence

<220>
<221> source
<223> /note="Description of Artificial Sequence: Synthetic peptide"

<400> 35

Arg Ala Ser Glu Ser Val Asp Thr Tyr Gly Asn Asn Phe Ile His
1 5 10 15

<210> 36
<211> 7
<212> PRT
<213> Artificial Sequence

<220>
<221> source
<223> /note="Description of Artificial Sequence: Synthetic peptide"

<400> 36

Leu Ala Ser Asn Leu Glu Ser
1 5

<210> 37
<211> 9
<212> PRT
<213> Artificial Sequence

<220>
<221> source
<223> /note="Description of Artificial Sequence: Synthetic peptide"

<400> 37

Gln Gln Asn Asn Gly Asp Pro Trp Thr
1 5

<210> 38
<211> 110
<212> PRT
<213> Artificial Sequence

<220>
<221> source
<223> /note="Description of Artificial Sequence: Synthetic polypeptide"

<400> 38

Pro Ala Ser Leu Ala Val Ser Leu Gly Gln Arg Ala Thr Ile Ser Cys
1 5 10 15

Arg Ala Ser Glu Ser Val Asp Thr Tyr Gly Asn Asn Phe Ile His Trp
20 25 30

Tyr Gln Gln Lys Pro Gly Gln Pro Pro Lys Leu Leu Ile Tyr Leu Ala
35 40 45

Ser Asn Leu Glu Ser Gly Val Pro Ala Arg Phe Ser Gly Ser Gly Ser
50 55 60

Arg Thr Asp Phe Thr Leu Thr Ile Asp Pro Val Glu Ala Asp Asp Ala
65 70 75 80

Ala Thr Tyr Tyr Cys Gln Gln Asn Asn Gly Asp Pro Trp Thr Phe Gly
85 90 95

Gly Gly Thr Lys Leu Glu Ile Lys Arg Ala Asp Ala Ala Pro
100 105 110

<210> 39
<211> 5
<212> PRT
<213> Artificial Sequence

<220>
<221> source
<223> /note="Description of Artificial Sequence: Synthetic peptide"

<400> 39

His Pro Tyr Met His
1 5

<210> 40
<211> 17
<212> PRT
<213> Artificial Sequence

<220>
<221> source
<223> /note="Description of Artificial Sequence: Synthetic peptide"

<400> 40

Arg Ile Asp Pro Ala Asn Gly Asn Thr Lys Tyr Asp Pro Lys Phe Gln
1 5 10 15

Gly

<210> 41
<211> 10
<212> PRT
<213> Artificial Sequence

<220>
<221> source
<223> /note="Description of Artificial Sequence: Synthetic peptide"

<400> 41

Glu Glu Val Ala Asp Tyr Thr Met Asp Tyr
1 5 10

<210> 42
<211> 126
<212> PRT
<213> Artificial Sequence

<220>
<221> source
<223> /note="Description of Artificial Sequence: Synthetic polypeptide"

<400> 42

Gly Ala Glu Leu Val Lys Pro Gly Ala Ser Val Lys Leu Ser Cys Thr
1 5 10 15

Ala Ser Gly Phe Asn Ile Lys His Pro Tyr Met His Trp Val Lys Gln
20 25 30

Arg Pro Asp Gln Gly Leu Glu Trp Ile Gly Arg Ile Asp Pro Ala Asn
35 40 45

Gly Asn Thr Lys Tyr Asp Pro Lys Phe Gln Gly Lys Ala Thr Ile Thr
50 55 60

Ala Asp Thr Ser Ser Asn Thr Ala Tyr Leu Gln Leu Ser Ser Leu Thr
65 70 75 80

Ser Glu Asp Thr Ala Val Tyr Tyr Cys Gly Arg Glu Glu Val Ala Asp
85 90 95

Tyr Thr Met Asp Tyr Trp Gly Gln Gly Thr Ser Val Thr Val Ser Ser
100 105 110

Ala Lys Thr Thr Ala Pro Ser Val Tyr Pro Leu Ala Pro Val
115 120 125

<210> 43
<211> 11
<212> PRT
<213> Artificial Sequence

<220>
<221> source
<223> /note="Description of Artificial Sequence: Synthetic peptide"

<400> 43

Ser Ala Ser Gln Gly Ile Asn Asn Phe Leu Asn
1 5 10

<210> 44
<211> 7
<212> PRT
<213> Artificial Sequence

<220>
<221> source
<223> /note="Description of Artificial Sequence: Synthetic peptide"

<400> 44

Tyr Thr Ser Ser Leu His Ser
1 5

<210> 45
<211> 9
<212> PRT
<213> Artificial Sequence

<220>
<221> source
<223> /note="Description of Artificial Sequence: Synthetic peptide"

<400> 45

```
Gln His Phe Ser Lys Leu Pro Trp Thr
1               5
```

<210> 46
<211> 106
<212> PRT
<213> Artificial Sequence

<220>
<221> source
<223> /note="Description of Artificial Sequence: Synthetic polypeptide"

<400> 46

```
Thr Ser Ser Leu Ser Ala Ser Leu Gly Asp Arg Val Thr Ile Ser Cys
1               5                   10                  15

Ser Ala Ser Gln Gly Ile Asn Asn Phe Leu Asn Trp Tyr Gln Gln Lys
            20                  25                  30

Pro Asp Gly Thr Val Lys Leu Leu Ile Tyr Tyr Thr Ser Ser Leu His
        35                  40                  45

Ser Gly Val Pro Ser Arg Phe Ser Gly Ser Gly Ser Gly Thr Asp Tyr
    50                  55                  60

Ser Leu Thr Ile Ser Asn Leu Glu Pro Glu Asp Ile Ala Ile Tyr Tyr
65                  70                  75                  80

Cys Gln His Phe Ser Lys Leu Pro Trp Thr Phe Gly Gly Gly Thr Lys
                85                  90                  95

Leu Glu Ile Lys Arg Ala Asp Ala Ala Pro
            100                 105
```

<210> 47
<211> 5
<212> PRT
<213> Artificial Sequence

<220>
<221> source

<223> /note="Description of Artificial Sequence: Synthetic peptide"

<400> 47

```
Ser Tyr Ala Met Ser
1               5
```

<210> 48
<211> 17
<212> PRT
<213> Artificial Sequence

<220>
<221> source
<223> /note="Description of Artificial Sequence: Synthetic peptide"

<400> 48

```
Glu Ile Gly Ser Gly Gly Ser Tyr Thr Tyr Tyr Pro Asp Thr Val Thr
1               5                   10                  15

Gly
```

<210> 49
<211> 9
<212> PRT
<213> Artificial Sequence

<220>
<221> source
<223> /note="Description of Artificial Sequence: Synthetic peptide"

<400> 49

```
Glu Thr Thr Ala Gly Tyr Phe Asp Tyr
1               5
```

<210> 50
<211> 112
<212> PRT
<213> Artificial Sequence

<220>
<221> source
<223> /note="Description of Artificial Sequence: Synthetic polypeptide"

<400> 50

Ser Gly Gly Gly Leu Val Arg Pro Gly Gly Ser Leu Lys Leu Ser Cys
1 5 10 15

Ala Ala Ser Gly Phe Thr Phe Ser Ser Tyr Ala Met Ser Trp Val Arg
20 25 30

Gln Ser Pro Glu Lys Arg Leu Glu Trp Val Ala Glu Ile Gly Ser Gly
35 40 45

Gly Ser Tyr Thr Tyr Tyr Pro Asp Thr Val Thr Gly Arg Phe Thr Ile
50 55 60

Ser Arg Asp Asn Ala Lys Ser Thr Leu Tyr Leu Glu Met Ser Ser Leu
65 70 75 80

Arg Ser Glu Asp Thr Ala Ile Tyr Tyr Cys Ala Arg Glu Thr Thr Ala
85 90 95

Gly Tyr Phe Asp Tyr Trp Gly Gln Gly Thr Thr Leu Thr Val Ser Ser
100 105 110

<210> 51
<211> 11
<212> PRT
<213> Artificial Sequence

<220>
<221> source
<223> /note="Description of Artificial Sequence: Synthetic peptide"

<400> 51

Arg Thr Ser Glu Asn Ile Phe Ser Tyr Leu Ala
1 5 10

<210> 52
<211> 7
<212> PRT
<213> Artificial Sequence

<220>
<221> source
<223> /note="Description of Artificial Sequence: Synthetic peptide"

<400> 52

Asn Ala Lys Thr Leu Ala Glu
1 5

<210> 53
<211> 9
<212> PRT
<213> Artificial Sequence

<220>
<221> source
<223> /note="Description of Artificial Sequence: Synthetic peptide"

<400> 53

```
Gln His His Tyr Ala Phe Pro Trp Thr
1               5
```

<210> 54
<211> 106
<212> PRT
<213> Artificial Sequence

<220>
<221> source
<223> /note="Description of Artificial Sequence: Synthetic polypeptide"

<400> 54

```
Pro Ala Ser Leu Ser Ala Ser Val Gly Glu Thr Val Thr Ile Thr Cys
1               5                   10                  15

Arg Thr Ser Glu Asn Ile Phe Ser Tyr Leu Ala Trp Tyr Gln Gln Lys
            20                  25                  30

Gln Gly Ile Ser Pro Gln Leu Leu Val Tyr Asn Ala Lys Thr Leu Ala
        35                  40                  45

Glu Gly Val Pro Ser Arg Phe Ser Gly Ser Gly Ser Gly Thr Gln Phe
    50                  55                  60

Ser Leu Lys Ile Asn Ser Leu Gln Pro Glu Asp Phe Gly Ser Tyr Tyr
65                  70                  75                  80

Cys Gln His His Tyr Ala Phe Pro Trp Thr Phe Gly Gly Gly Ser Lys
                85                  90                  95

Leu Glu Ile Lys Arg Ala Asp Ala Ala Pro
            100                 105
```

<210> 55
<211> 5
<212> PRT
<213> Artificial Sequence

<220>
<221> source
<223> /note="Description of Artificial Sequence: Synthetic peptide"

<400> 55

Gly Tyr Phe Met Asn
1 5

<210> 56
<211> 17
<212> PRT
<213> Artificial Sequence

<220>
<221> source
<223> /note="Description of Artificial Sequence: Synthetic peptide"

<400> 56

Arg Ile Phe Pro Tyr Asn Gly Asp Thr Phe Tyr Asn Gln Lys Phe Lys
1 5 10 15

Gly

<210> 57
<211> 7
<212> PRT
<213> Artificial Sequence

<220>
<221> source
<223> /note="Description of Artificial Sequence: Synthetic peptide"

<400> 57

Gly Thr His Tyr Phe Asp Tyr
1 5

<210> 58
<211> 128
<212> PRT
<213> Artificial Sequence

<220>
<221> source
<223> /note="Description of Artificial Sequence: Synthetic polypeptide"

<400> 58

```
Gly Pro Glu Leu Val Lys Pro Gly Ala Ser Val Lys Ile Ser Cys Lys
1               5                   10                  15

Ala Ser Asp Tyr Ser Phe Thr Gly Tyr Phe Met Asn Trp Val Met Gln
            20                  25                  30

Ser His Gly Lys Ser Leu Glu Trp Ile Gly Arg Ile Phe Pro Tyr Asn
        35                  40                  45

Gly Asp Thr Phe Tyr Asn Gln Lys Phe Lys Gly Arg Ala Thr Leu Thr
    50                  55                  60

Val Asp Lys Ser Ser Ser Thr Ala His Met Glu Leu Arg Ser Leu Ala
65                  70                  75                  80

Ser Glu Asp Ser Ala Val Tyr Phe Cys Ala Arg Gly Thr His Tyr Phe
                85                  90                  95

Asp Tyr Trp Gly Gln Gly Thr Thr Leu Thr Val Ser Ser Ala Lys Thr
            100                 105                 110

Thr Pro Pro Ser Val Tyr Pro Leu Ala Pro Gly Ser Ala Ala Gln Thr
        115                 120                 125
```

<210> 59
<211> 36
<212> DNA
<213> Artificial Sequence

<220>
<221> source
<223> /note="Description of Artificial Sequence: Synthetic oligonucleotide"

<400> 59
agggccagct caactgtaag ttacagttac ttgcac 36

<210> 60
<211> 21
<212> DNA
<213> Artificial Sequence

<220>
<221> source
<223> /note="Description of Artificial Sequence: Synthetic oligonucleotide"

<400> 60
ggcacatcca acttggcttc t 21

<210> 61
<211> 27
<212> DNA
<213> Artificial Sequence

<220>
<221> source
<223> /note="Description of Artificial Sequence: Synthetic oligonucleotide"

<400> 61
cagcagtaca gtggttaccc actcacg 27

<210> 62
<211> 323
<212> DNA
<213> Artificial Sequence

<220>
<221> source
<223> /note="Description of Artificial Sequence: Synthetic polynucleotide"

<400> 62

```
ccagcaatca tgtctgcatc tccaggggaa aaggtcacca tgacctgcag ggccagctca    60
actgtaagtt acagttactt gcactggtac cagcagaagt caggtgcctc cccccaactc   120
tggatttatg gcacatccaa cttggcttct ggagtccctg ctcgcttcag tggcagtggg   180
tctgggacct cttactctct cacaatcagc agtgtggagg ctgaagatgc tgccacttat   240
tactgccagc agtacagtgg ttacccactc acgttcggtg ctgggaccaa gctggagctg   300
aaacgggctg atgctgcacc aac                                           323
```

<210> 63
<211> 18
<212> DNA
<213> Artificial Sequence

<220>
<221> source
<223> /note="Description of Artificial Sequence: Synthetic oligonucleotide"

<400> 63
agtggttatt actggaac 18

<210> 64
<211> 48
<212> DNA
<213> Artificial Sequence

<220>
<221> source
<223> /note="Description of Artificial Sequence: Synthetic oligonucleotide"

<400> 64
tacataaagt ccgacggtag caataattac aacccatctc tcaaaaat 48

<210> 65
<211> 21
<212> DNA
<213> Artificial Sequence

<220>
<221> source
<223> /note="Description of Artificial Sequence: Synthetic oligonucleotide"

<400> 65
gagtggaagg ctatggacta c 21

<210> 66
<211> 389
<212> DNA
<213> Artificial Sequence

<220>
<221> source
<223> /note="Description of Artificial Sequence: Synthetic polynucleotide"

<400> 66

**gagtcaggac ctggcctcgt gagaccttct cagtctctgt ctctcacctg ctctgtcact 60**

**ggctactcca tcaccagtgg ttattactgg aactggatcc ggcagtttcc aggaagcaga 120**

**ctggaatgga tgggctacat aaagtccgac ggtagcaata attacaaccc atctctcaaa 180**

**aatcgaatct ccatcactcg tgacacatct aagaaccagt ttttcctgaa gttgaattct 240**

**gtgactactg aggacacagc tacatatttc tgtacaaggg agtggaaggc tatggactac 300**

**tggggtcagg gaacctcagt caccgtctcc tcagccaaaa caacaccccc atcagtctat 360**

**ccactggccc ctgggtgtgg agatacaac 389**

<210> 67
<211> 45
<212> DNA
<213> Artificial Sequence

<220>
<221> source
<223> /note="Description of Artificial Sequence: Synthetic oligonucleotide"

<400> 67
agagccagtg aaagtgttga tacttatggc aataatttta tacac 45

<210> 68
<211> 21
<212> DNA
<213> Artificial Sequence

<220>
<221> source
<223> /note="Description of Artificial Sequence: Synthetic oligonucleotide"

<400> 68
cttgcatcca acctagaatc t 21

<210> 69

<211> 27
<212> DNA
<213> Artificial Sequence

<220>
<221> source
<223> /note="Description of Artificial Sequence: Synthetic oligonucleotide"

<400> 69
cagcaaaata atggggatcc gtggacg 27

<210> 70
<211> 331
<212> DNA
<213> Artificial Sequence

<220>
<221> source
<223> /note="Description of Artificial Sequence: Synthetic polynucleotide"

<400> 70

**ccagcttctt tggctgtgtc tctagggcag agggccacca tatcctgcag agccagtgaa 60**

**agtgttgata cttatggcaa taattttata cactggtacc agcagaaacc aggacagcca 120**

**cccaaactcc tcatttatct tgcatccaac ctagaatctg ggtccctgc caggttcagt 180**

**ggcagtgggt ctaggacaga cttcaccctc accattgatc ctgtggaggc tgatgatgct 240**

**gcaacctatt actgtcagca aaataatggg gatccgtgga cgttcggtgg aggcaccaag 300**

**ctggagatca aacgggctga tgctgcacca a 331**

<210> 71
<211> 15
<212> DNA
<213> Artificial Sequence

<220>
<221> source
<223> /note="Description of Artificial Sequence: Synthetic oligonucleotide"

<400> 71
cacccctata tgcac 15

<210> 72
<211> 51
<212> DNA
<213> Artificial Sequence

<220>
<221> source
<223> /note="Description of Artificial Sequence: Synthetic oligonucleotide"

<400> 72
aggattgatc ctgcgaatgg taatactaaa tatgacccga agttccaggg c 51

<210> 73
<211> 30
<212> DNA
<213> Artificial Sequence

<220>
<221> source
<223> /note="Description of Artificial Sequence: Synthetic oligonucleotide"

<400> 73
gaggaggtgg cggactatac tatggactac 30

<210> 74
<211> 380
<212> DNA
<213> Artificial Sequence

<220>
<221> source
<223> /note="Description of Artificial Sequence: Synthetic polynucleotide"

<400> 74

ggggcagagc ttgtgaagcc aggggcctca gtcaagttgt cctgcacagc ttctggcttc 60

aacattaaac acccctatat gcactgggtg aagcagaggc ctgaccaggg cctggagtgg 120

attggaagga ttgatcctgc gaatggtaat actaaatatg acccgaagtt ccagggcaag 180

gccactataa cagcagacac atcctccaac acagcctacc tacagctcag cagcctgaca 240

tctgaggaca ctgccgtcta ttactgtggt agagaggagg tggcggacta tactatggac 300

tactggggtc aaggaacctc agtcaccgtc tcctcagcca aaacaacagc cccatcggtc 360

tatccactgg cccctgtgtg 380

<210> 75
<211> 33
<212> DNA
<213> Artificial Sequence

<220>
<221> source
<223> /note="Description of Artificial Sequence: Synthetic oligonucleotide"

<400> 75
agtgcaagtc agggcattaa caatttttta aac 33

<210> 76
<211> 21
<212> DNA
<213> Artificial Sequence

<220>
<221> source
<223> /note="Description of Artificial Sequence: Synthetic oligonucleotide"

<400> 76
tacacatcaa gtttacactc a 21

<210> 77
<211> 27
<212> DNA
<213> Artificial Sequence

<220>
<221> source
<223> /note="Description of Artificial Sequence: Synthetic oligonucleotide"

<400> 77
cagcacttta gtaagcttcc gtggacg 27

<210> 78
<211> 320
<212> DNA
<213> Artificial Sequence

<220>
<221> source
<223> /note="Description of Artificial Sequence: Synthetic polynucleotide"

<400> 78

acatcctccc tgtctgcctc tctgggagac agagtcacca tcagttgcag tgcaagtcag 60

ggcattaaca attttttaaa ctggtatcag cagaaaccag atggcactgt taaactcctg 120

atctattaca catcaagttt acactcagga gtcccatcaa ggttcagtgg cagtgggtct 180

gggacagatt attctctcac catcagcaac ctggaacctg aagatattgc catatactat 240

tgtcagcact ttagtaagct tccgtggacg ttcggtggag gcaccaagct ggaaatcaaa 300

cgggctgatg ctgcaccaac 320

<210> 79
<211> 15
<212> DNA
<213> Artificial Sequence

<220>
<221> source
<223> /note="Description of Artificial Sequence: Synthetic oligonucleotide"

<400> 79
agctatgcca tgtct 15

<210> 80
<211> 51
<212> DNA
<213> Artificial Sequence

<220>
<221> source
<223> /note="Description of Artificial Sequence: Synthetic oligonucleotide"

<400> 80
gaaattggta gtggtggtag ttacacctac tatccagaca ctgtgacggg c 51

<210> 81
<211> 27
<212> DNA
<213> Artificial Sequence

<220>
<221> source
<223> /note="Description of Artificial Sequence: Synthetic oligonucleotide"

<400> 81
gaaactacgg cgggctactt tgactac 27

<210> 82
<211> 336
<212> DNA
<213> Artificial Sequence

<220>
<221> source
<223> /note="Description of Artificial Sequence: Synthetic polynucleotide"

<400> 82

```
tctgggggag gcttagtgag gcctggaggg tccctgaaac tctcctgtgc agcctctgga      60

ttcactttca gtagctatgc catgtcttgg gttcgccagt ctccagagaa gaggctggag     120

tgggtcgcag aaattggtag tggtggtagt tacacctact atccagacac tgtgacgggc     180

cgattcacca tctccagaga caatgccaag agcaccctgt acctggaaat gagcagtctg     240

aggtctgagg acacggccat ctattactgt gcaagggaaa ctacggcggg ctactttgac     300

tactggggcc aaggcaccac tctcacagtc tcctca                               336
```

<210> 83
<211> 33
<212> DNA
<213> Artificial Sequence

<220>
<221> source
<223> /note="Description of Artificial Sequence: Synthetic oligonucleotide"

<400> 83
cgaacaagtg agaatatttt cagttattta gca 33

<210> 84
<211> 21
<212> DNA
<213> Artificial Sequence

<220>
<221> source

<223> /note="Description of Artificial Sequence: Synthetic oligonucleotide"

<400> 84
aatgcaaaaa ccttagcaga g 21

<210> 85
<211> 27
<212> DNA
<213> Artificial Sequence

<220>
<221> source
<223> /note="Description of Artificial Sequence: Synthetic oligonucleotide"

<400> 85
caacatcatt atgcttttcc gtggacg 27

<210> 86
<211> 320
<212> DNA
<213> Artificial Sequence

<220>
<221> source
<223> /note="Description of Artificial Sequence: Synthetic polynucleotide"

<400> 86

```
ccagcctccc tatctgcatc tgtgggagaa actgtcacca tcacatgtcg aacaagtgag      60
aatattttca gttatttagc atggtatcag cagaaacagg gaatatctcc tcagctcctg     120
gtctataatg caaaaacctt agcagagggt gtgccatcaa ggttcagtgg cagtggatca     180
ggcacacagt tttctctgaa gatcaacagc ctgcagcctg aagattttgg gagttattac     240
tgtcaacatc attatgcttt tccgtggacg ttcggtggag gctccaagct ggaaatcaaa     300
cgggctgatg ctgcaccaac                                                 320
```

<210> 87
<211> 15
<212> DNA
<213> Artificial Sequence

<220>
<221> source
<223> /note="Description of Artificial Sequence: Synthetic oligonucleotide"

<400> 87
ggctacttta tgaac 15

<210> 88
<211> 51
<212> DNA
<213> Artificial Sequence

<220>
<221> source
<223> /note="Description of Artificial Sequence: Synthetic oligonucleotide"

<400> 88
cgtatttttc cttacaatgg tgatactttc tacaaccaga agttcaaggg c 51

<210> 89
<211> 21
<212> DNA
<213> Artificial Sequence

<220>
<221> source
<223> /note="Description of Artificial Sequence: Synthetic oligonucleotide"

<400> 89
gggactcatt actttgacta c 21

<210> 90
<211> 386
<212> DNA
<213> Artificial Sequence

<220>
<221> source
<223> /note="Description of Artificial Sequence: Synthetic polynucleotide"

<400> 90

```
ggacctgagc tggtgaagcc tggggcttca gtgaagatat cctgcaaggc ttctgattac    60
tcttttactg gctactttat gaactgggtg atgcagagcc atggaaagag ccttgagtgg   120
attggacgta tttttcctta caatggtgat actttctaca accagaagtt caagggcagg   180
gccacattga ctgtagacaa atcctctagc acagcccaca tggagctccg gagcctggca   240
tctgaggact ctgcagtcta tttttgtgca agagggactc attactttga ctactggggc   300
caaggcacca ctctcactgt ctcctcagcc aaaacgacac ccccatctgt ctatccactg   360
gcccctggat ctgctgccca aactaa                                        386
```